(19) Europäisches Patentamt
European Patent Office
Office européen des brevets

(11) EP 4 755 380 A1

(12) EUROPEAN PATENT APPLICATION

(43) Date of publication:
**10.06.2026 Bulletin 2026/24**

(21) Application number: **25221998.5**

(22) Date of filing: **09.12.2025**

(51) International Patent Classification (IPC):
***A61K 31/08*** (2006.01) ***A61P 1/16*** (2006.01)
***A61P 3/04*** (2006.01) ***A61P 3/06*** (2006.01)
***A61P 3/08*** (2006.01) ***A61P 5/50*** (2006.01)
***A61P 9/12*** (2006.01) ***A61P 19/02*** (2006.01)

(52) Cooperative Patent Classification (CPC):
**A61K 31/08; A61P 1/16; A61P 3/04; A61P 3/06; A61P 3/08; A61P 5/50; A61P 9/12; A61P 19/02**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **09.12.2024 KR 20240181636**
**20.12.2024 KR 20240193342**
**27.03.2025 KR 20250039326**
**05.09.2025 KR 20250126258**

(71) Applicant: **Lux Anima Co., Ltd.**
**Jeollanam-do 58141 (KR)**

(72) Inventors:
- **LEE, Seung-Rock**
  **Gwangju (KR)**
- **NGUYEN, Huu Thang**
  **Jeollanam-do (KR)**
- **LEE, Geunhaeng**
  **Gwangju (KR)**
- **CHOI, Jin Myung**
  **Jeollanam-do (KR)**
- **YOON, Hyun Joong**
  **Gwangju (KR)**
- **TRINH, Hoang Vu**
  **Jeollanam-do (KR)**
- **SAH, Dhiraj Kumar**
  **Jeollanam-do (KR)**

(74) Representative: **Vossius & Partner**
**Patentanwälte Rechtsanwälte mbB**
**Siebertstraße 3**
**81675 München (DE)**

(54) **AMPK ACTIVATOR CONTAINING 1,1-DIETHOXYETHANE AS AN ACTIVE INGREDIENT AND ITS USE**

(57) **[Summary]**

The present invention relates to an AMPK activator comprising 1,1-diethoxyethane (1,1-DEE) as an active ingredient and the uses thereof. More specifically, the present invention relates to an AMPK activator comprising 1,1-diethoxyethane as an active ingredient and the uses thereof, for example, compositions for preventing, treating, or improving diseases requiring AMPK activation, anti-aging, extending lifespan, or enhancing physical vitality, including pharmaceutical compositions, cosmetic compositions, food compositions, or feed compositions.

The present invention also relates to a composition for improving insulin resistance comprising 1,1-diethoxyethane (1,1-DEE) as an active ingredient and the uses thereof. More specifically, the present invention relates to a composition comprising 1,1-diethoxyethane as an active ingredient as an oxidative PTEN inhibitor for increasing insulin sensitivity or improving insulin resistance, and the uses thereof, for example, compositions for preventing, treating, or improving diseases associated with insulin resistance, including pharmaceutical compositions, cosmetic compositions, food compositions, or feed compositions.

The present invention further relates to a composition for preventing or treating obesity or related metabolic disorders comprising 1,1-diethoxyethane (1,1-DEE) as an active ingredient. More specifically, the present invention relates to pharmaceutical compositions, cosmetic compositions, food compositions, or feed compositions comprising 1,1-diethoxyethane as an active ingredient for preventing or treating obesity.

EP 4 755 380 A1

**Figure 1A**

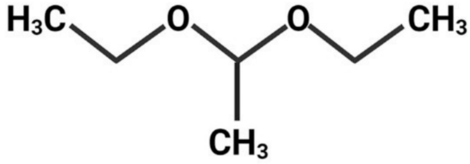

1,1-diethoxyethane

**Figure 1B**

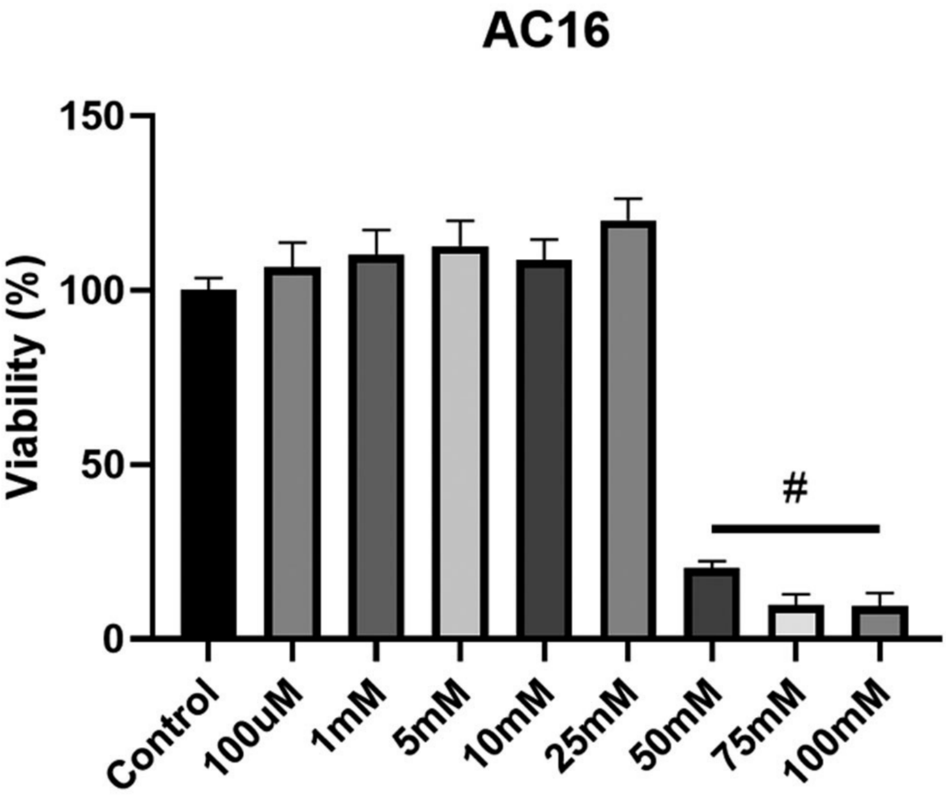




(Cont. next page)

Figure 1C

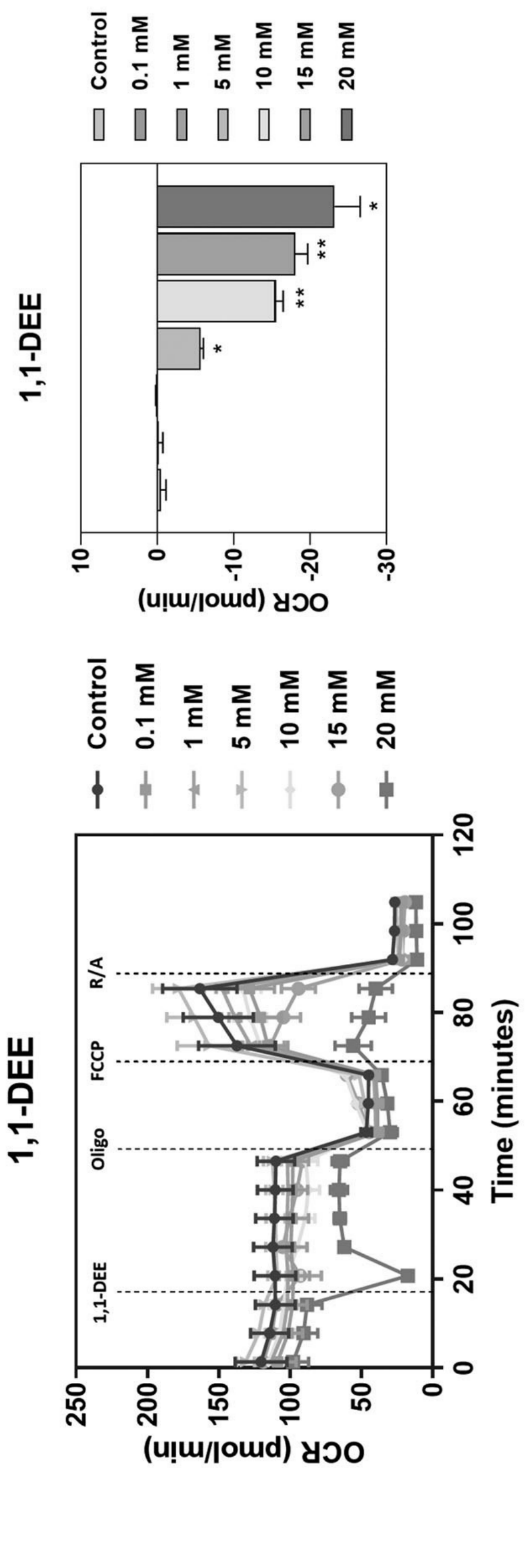




(Cont. next page)

**Figure 1C continued**

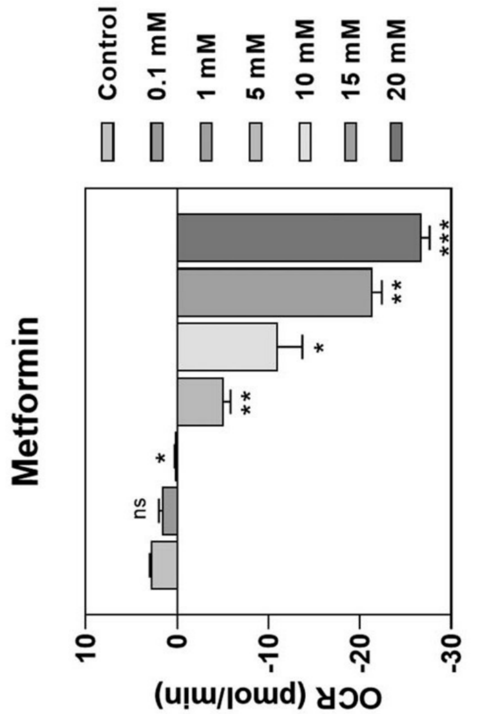


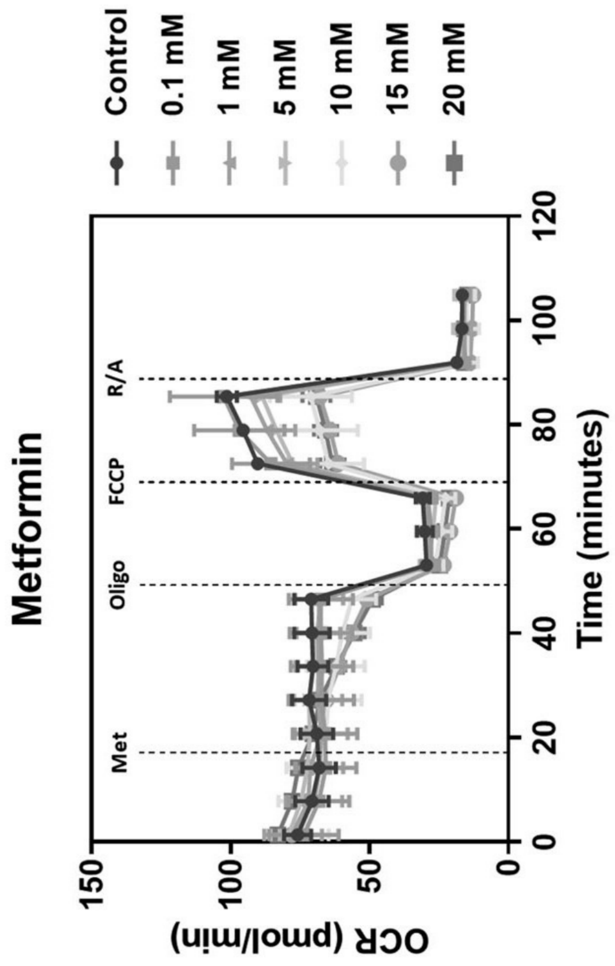




(Cont. next page)

**Figure 1C continued**

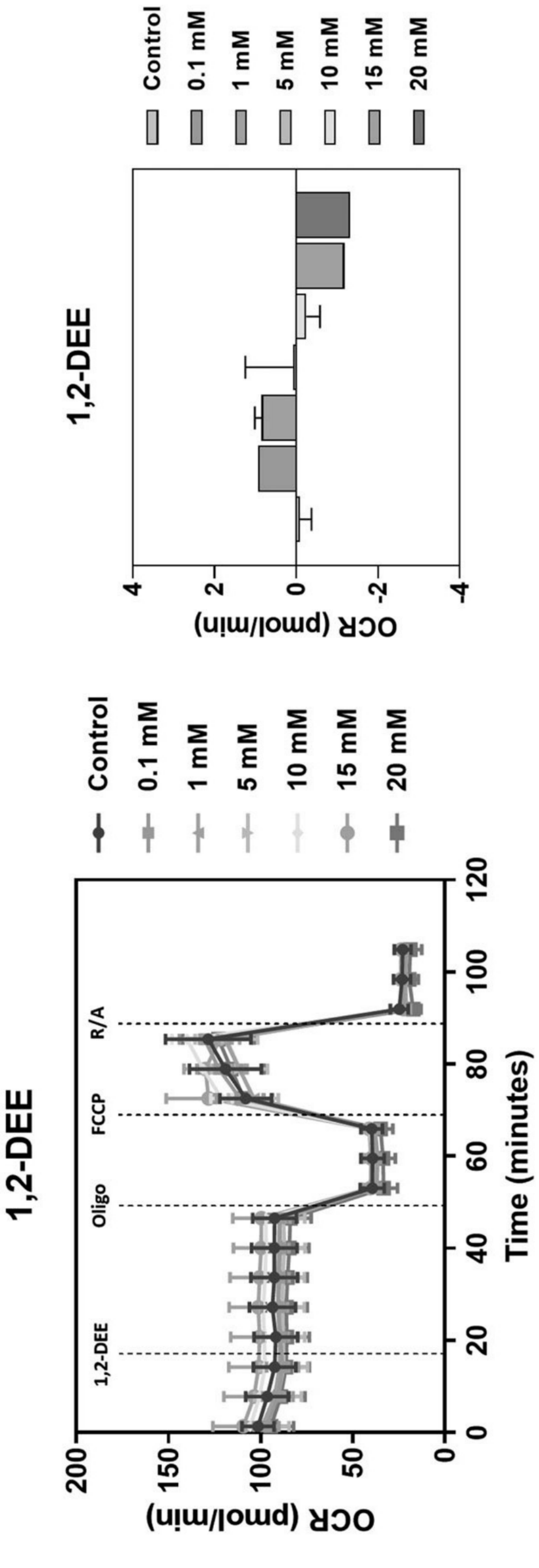




(Cont. next page)

**Figure 1D**

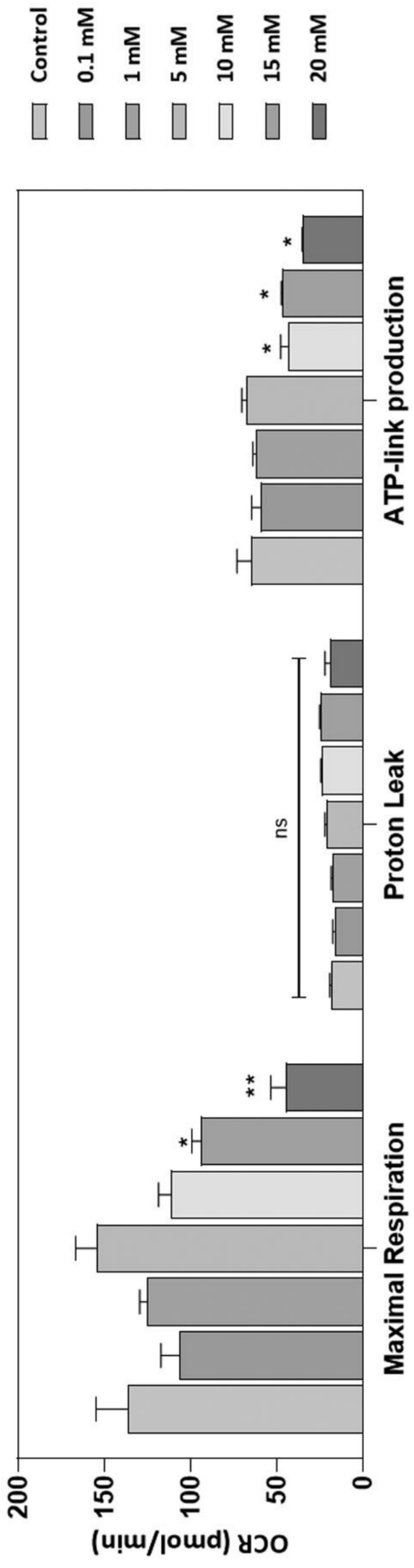




(Cont. next page)

Figure 1E

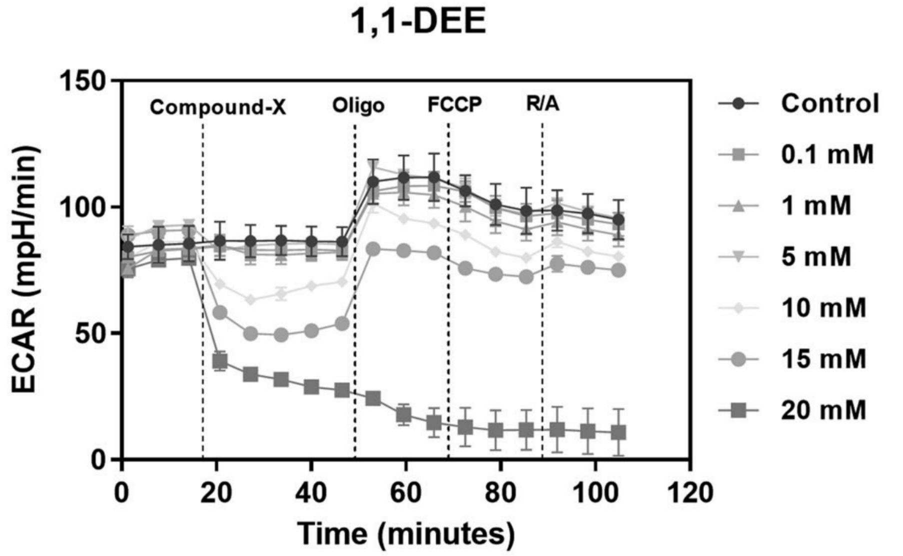
1,1-DEE
Compound-X
Oligo
FCCP
R/A
ECAR (mpH/min)
Time (minutes)
0
20
40
60
80
100
120
50
150
Control
0.1 mM
1 mM
5 mM
10 mM
15 mM
20 mM

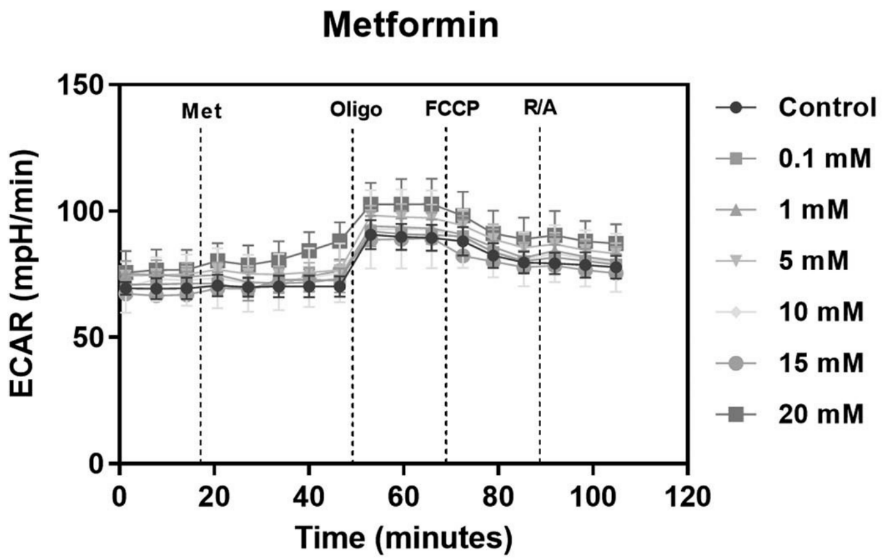
Metformin
Met
Oligo
FCCP
R/A
ECAR (mpH/min)
Time (minutes)
0
20
40
60
80
100
120
50
150
Control
0.1 mM
1 mM
5 mM
10 mM
15 mM
20 mM

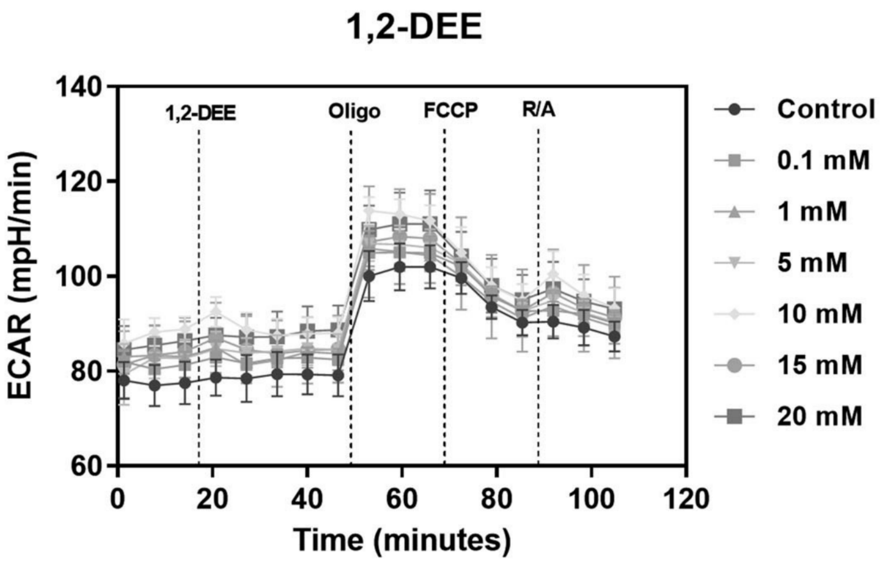
1,2-DEE
1,2-DEE
Oligo
FCCP
R/A
ECAR (mpH/min)
Time (minutes)
0
20
40
60
80
100
120
140
Control
0.1 mM
1 mM
5 mM
10 mM
15 mM
20 mM

## Description

[Technical Field]

The present invention relates to an AMPK activator comprising 1,1-diethoxyethane (1,1-DEE) as an active ingredient and the uses thereof. More specifically, the present invention relates to an AMPK activator comprising 1,1-diethoxyethane as an active ingredient and the uses thereof, for example, compositions for preventing, treating, or improving diseases requiring AMPK activation, anti-aging, extending lifespan, or enhancing physical vitality, including pharmaceutical compositions, cosmetic compositions, food compositions, or feed compositions.

The present invention also relates to a composition for improving insulin resistance comprising 1,1-diethoxyethane (1,1-DEE) as an active ingredient and the uses thereof. More specifically, the present invention relates to a composition comprising 1,1-diethoxyethane as an active ingredient as an oxidative PTEN inhibitor for increasing insulin sensitivity or improving insulin resistance, and the uses thereof, for example, compositions for preventing, treating, or improving diseases associated with insulin resistance, including pharmaceutical compositions, cosmetic compositions, food compositions, or feed compositions.

The present invention further relates to a composition for preventing or treating obesity or related metabolic disorders comprising 1,1-diethoxyethane (1,1-DEE) as an active ingredient. More specifically, the present invention relates to pharmaceutical compositions, cosmetic compositions, food compositions, or feed compositions comprising 1,1-diethoxyethane as an active ingredient for preventing or treating obesity.

[Background Art]

<u>AMPK Activation</u>

5'-Adenosine monophosphate (AMP)-activated protein kinase (AMPK) is an evolutionarily conserved serine (Ser)/threonine (Thr) kinase enzyme responsible for maintaining cellular energy homeostasis, and this enzyme plays an important role in regulating energy balance [1,2]. AMPK is composed of a catalytic $\alpha$-subunit, a regulatory $\beta$-subunit, and a non-catalytic $\gamma$-subunit, each of which regulates various physiological functions [3]. The $\alpha$-subunit activates AMPK through phosphorylation of the Thr172 residue, the $\beta$-subunit promotes the binding of AMPK to glycogen, and the $\gamma$-subunit binds AMP and ADP to stimulate AMPK phosphorylation [4]. Activation of AMPK promotes catabolic metabolism and suppresses anabolic metabolism, thereby generating ATP and improving energy balance. Upstream kinases such as LKB1 (liver kinase B1), CAMKK2 (Ca2+/calmodulin-dependent protein kinase kinase-2), and TAK (transforming growth factor-beta-activated kinase) play important roles in AMPK activation [5-7]. Drugs such as metformin, AICAR (5-aminoimidazole-4-carboxamide ribonucleotide), and resveratrol activate AMPK by regulating upstream kinases [8-10]. Energy states such as exercise and nutrient deprivation can also activate AMPK. Activation of AMPK may have potential effects on obesity, cardiovascular diseases (CVD), and cancer prevention [11], and plays particularly important preventive roles in cardiovascular diseases [12-17].

AMPK contributes critically to energy balance by regulating downstream effectors of catabolic processes such as fatty acid synthesis and glycolysis. Acetyl-CoA carboxylase (ACC) plays an important role in fatty acid biosynthesis, and phosphorylation of Ser79 (ACC1) or Ser212 (ACC2) by AMPK inhibits ACC enzymatic activity and increases fatty acid oxidation [1]. In the heart, fatty acid oxidation constitutes a major part of energy production. The AMPK-ACC signaling pathway regulates various physiological processes, including platelet phospholipids and thrombogenesis, cardiac hypertrophy and contractility, and ischemic regulation [19-21]. In addition, glycolysis is also regulated by PFKFB2 (6-phosphofructo-2-kinase/fructose-2,6-bisphosphatase-2), which is involved in the formation and degradation of fructose-2,6-bisphosphate (Fru-2,6-P2) and thereby modulates the enzymatic activity of PFK1 (phosphofructokinase-1). Phosphorylation of Ser466 and Ser483 of PFKFB2 promotes glycolysis and is regulated by Akt (protein kinase B) and AMPK. Activation of PFKFB2 plays an important role in cardiac remodeling, suppression of ferroptosis during I/R injury, and improvement of cardiac function under hypoxic conditions [22-24].

Mitochondrial biosynthesis is an important process for adapting to cellular energy demands, and AMPK can promote this process. AMPK deficiency can lead to mitochondrial dysfunction, and PGC-1$\alpha$ (peroxisome proliferator-activated receptor gamma coactivator 1-alpha), a key downstream regulator of AMPK, plays an important role in mitochondrial biosynthesis [2]. AMPK activates PGC-1$\alpha$ through activation of transcription factor EB and direct binding, and promotes mitochondrial biosynthesis by phosphorylating Thr177 and Ser538 of PGC-1$\alpha$ [27,28]. This process makes a significant contribution to cardioprotection [29,30].

<u>Insulin Resistance</u>

The PI3K/Akt (phosphoinositide-3-kinase/protein kinase B) pathway is a critical signaling system that regulates

various cellular processes such as proliferation, survival, and differentiation. When ligands, such as growth factors, bind to receptor tyrosine kinases (RTKs), PI3K is activated, and PIP2 (phosphatidylinositol (4,5)-bisphosphate) is phosphorylated to PIP3 (phosphatidylinositol (3,4,5)-trisphosphate). When PIP3 accumulates at the cell membrane, Akt is activated, which regulates various downstream effectors.

PTEN (phosphatase and tensin homolog deleted on chromosome 10) functions as a lipid phosphatase that dephosphorylates PIP3 to PIP2, playing an important role as a negative regulator of the PI3K/Akt signaling pathway. PTEN is regulated by various post-translational modifications, and changes such as oxidation, acetylation, or S-nitrosylation can inhibit its lipid phosphatase activity. For example, the catalytic nucleophilic residue Cys124 of PTEN is readily oxidized by reactive oxygen species (ROS), leading to the formation of a disulfide bond between Cys124 and Cys71 residues, thereby suppressing PTEN function.

Inhibition of PTEN increases the concentration of PIP3 and can activate the PI3K/Akt pathway through Akt activation. This, in turn, enhances insulin signaling and is expected to improve insulin sensitivity. Such a mechanism presents new possibilities for the treatment of metabolic diseases, and related research is actively ongoing.

The PI3K/Akt pathway plays a particularly important role in the regulation of insulin sensitivity. When insulin binds to the transmembrane RTK, the insulin receptor (IR), the pathway is activated through autophosphorylation. This activation of Akt promotes glycolysis and glycogen synthesis, thereby increasing glucose uptake and intracellular metabolism.

Obesity

In modern society, while rapid economic development and abundant nutritional intake have increased, physical activity has significantly decreased, leading to a rise in the prevalence of metabolic syndrome, in which two or more conditions such as obesity, diabetes, hypertension, hypertriglyceridemia, hypercholesterolemia, and atherosclerosis occur concurrently. Cardiovascular diseases and strokes resulting from these conditions have increased to the extent that they rank as the second and third leading causes of death among Koreans. These disorders arise from imbalanced metabolism, causing the accumulation of metabolic wastes and toxins in the body, which manifest as symptoms resulting in loss of bodily functions. Consequently, metabolic syndrome can progress to insulin resistance syndrome and act as a contributing factor to cardiovascular and brain diseases.

Metabolic syndrome can cause damage to the coronary arteries, leading to heart disease or serving as a cause of stroke. It can reduce the kidney's ability to excrete salt, resulting in hypertension, and increase the proportion of triglycerides, which are a risk factor for cardiovascular disease. Furthermore, it can elevate the risk of blood coagulation and, through type 2 diabetes-induced insulin resistance, cause damage to the eyes, kidneys, and nerves, thereby giving rise to multiple health complications.

Drugs for the effective treatment of metabolic syndrome have not yet been developed, and medications used to treat diabetes, dyslipidemia, and hypertension are currently employed for managing metabolic syndrome. Drugs that may be used for the treatment of metabolic syndrome include the antidiabetic drug metformin, thiazolidinediones (TZDs), glucosidase inhibitors, and dipeptidyl peptidase-IV (DPP-IV) inhibitors, along with antihypertensive and lipid-lowering agents. However, these medications have limitations in fundamentally improving metabolic syndrome.

Factors related to the causes and treatment of metabolic syndrome are known to directly or indirectly influence the condition, including physical activity, dietary habits, body weight, blood glucose, triglycerides, cholesterol, insulin resistance, adiponectin, leptin, AMP-activated protein kinase (AMPK) activity, sex hormones such as estrogen, genetic factors, and in vivo concentrations of malonyl-CoA.

Accordingly, for the effective management or treatment of metabolic syndrome, it is ideal to develop agents that can maintain normal blood glucose levels while simultaneously addressing obesity, the fundamental cause of metabolic syndrome. However, research and development for such therapeutic agents remain insufficient to date.

Prior Art References

[Non-Patent Document 1] Steinberg, G.R. and D.G. Hardie, New insights into activation and function of the AMPK. Nat Rev Mol Cell Biol, 2023. 24(4): p. 255-272.

[Non-Patent Document 2] Herzig, S. and R.J. Shaw, AMPK: guardian of metabolism and mitochondrial homeostasis. Nat Rev Mol Cell Biol, 2018. 19(2): p. 121-135.

[Non-Patent Document 3] Davies, S.P., et al., Purification of the AMP-activated protein kinase on ATP-gamma-sepharose and analysis of its subunit structure. Eur J Biochem, 1994. 223(2): p. 351-7.

[Non-Patent Document 4] Hardie, D.G., F.A. Ross, and S.A. Hawley, AMPK: a nutrient and energy sensor that maintains energy homeostasis. Nat Rev Mol Cell Biol, 2012. 13(4): p. 251-62.

[Non-Patent Document 5] Woods, A., et al., LKB1 is the upstream kinase in the AMP-activated protein kinase cascade. Curr Biol, 2003. 13(22): p. 2004-8.
[Non-Patent Document 6] Woods, A., et al., Ca2+/calmodulin-dependent protein kinase kinase-beta acts upstream of AMP-activated protein kinase in mammalian cells. Cell Metab, 2005. 2(1): p. 21-33.
[Non-Patent Document 7] Xie, M., et al., A pivotal role for endogenous TGF-beta-activated kinase-1 in the LKB1/AMP-activated protein kinase energy-sensor pathway. Proc Natl Acad Sci U S A, 2006. 103(46): p. 17378-83.
[Non-Patent Document 8] Shaw, R.J., et al., The kinase LKB1 mediates glucose homeostasis in liver and therapeutic effects of metformin. Science, 2005. 310(5754): p. 1642-6.
[Non-Patent Document 9] Chan, A.Y., et al., Resveratrol inhibits cardiac hypertrophy via AMP-activated protein kinase and Akt. J Biol Chem, 2008. 283(35): p. 24194-201.
[Non-Patent Document 10] Corton, J.M., et al., 5-aminoimidazole-4-carboxamide ribonucleoside. A specific method for activating AMP-activated protein kinase in intact cells? Eur J Biochem, 1995. 229(2): p. 558-65.
[Non-Patent Document 11] Jeon, S.M., Regulation and function of AMPK in physiology and diseases. Exp Mol Med, 2016. 48(7): p. e245.
[Non-Patent Document 12] Li, Y., et al., AMPK phosphorylates and inhibits SREBP activity to attenuate hepatic steatosis and atherosclerosis in diet-induced insulin-resistant mice. Cell Metab, 2011. 13(4): p. 376-388.
[Non-Patent Document 13] Pulipaka, S., et al., Therapeutic efficacies of mitochondria-targeted esculetin and metformin in the improvement of age-associated atherosclerosis via regulating AMPK activation. Geroscience, 2024. 46(2): p. 2391-2408.
[Non-Patent Document 14] Wang, Z., et al., Dexmedetomidine attenuates myocardial ischemia/reperfusion-induced ferroptosis via AMPK/GSK-3β/Nrf2 axis. Biomed Pharmacother, 2022. 154: p. 113572.
[Non-Patent Document 15] Zhang, Y., et al., Melatonin attenuates myocardial ischemia-reperfusion injury via improving mitochondrial fusion/mitophagy and activating the AMPK-OPA1 signaling pathways. J Pineal Res, 2019. 66(2): p. e12542.
[Non-Patent Document 16] Gélinas, R., et al., AMPK activation counteracts cardiac hypertrophy by reducing O-GlcNAcylation. Nat Commun, 2018. 9(1): p. 374.
[Non-Patent Document 17] Guo, R. and J. Ren, Deficiency in AMPK attenuates ethanol-induced cardiac contractile dysfunction through inhibition of autophagosome formation. Cardiovasc Res, 2012. 94 (3): p. 480-91.
[Non-Patent Document 18] Tong, L., Acetyl-coenzyme A carboxylase: crucial metabolic enzyme and attractive target for drug discovery. Cell Mol Life Sci, 2005. 62(16): p. 1784-803.
[Non-Patent Document 19] Lepropre, S., et al., AMPK-ACC signaling modulates platelet phospholipids and potentiates thrombus formation. Blood, 2018. 132(11): p. 1180-1192.
[Non-Patent Document 20] Turdi, S., et al., Deficiency in AMP-activated protein kinase exaggerates high fat diet-induced cardiac hypertrophy and contractile dysfunction. J Mol Cell Cardiol, 2011. 50(4): p. 712-22.
[Non-Patent Document 21] Hopkins, T.A., J.R. Dyck, and G.D. Lopaschuk, AMP-activated protein kinase regulation of fatty acid oxidation in the ischaemic heart. Biochem Soc Trans, 2003. 31(Pt 1): p. 207-12.
[Non-Patent Document 22] Harold, K.M., et al., Loss of Cardiac PFKFB2 Drives Metabolic, Functional, and Electrophysiological Remodeling in the Heart. J Am Heart Assoc, 2024. 13(7): p. e033676.
[Non-Patent Document 23] Fu, C., et al., PFKFB2 Inhibits Ferroptosis in Myocardial Ischemia/Reperfusion Injury Through Adenosine Monophosphate-Activated Protein Kinase Activation. J Cardiovasc Pharmacol, 2023. 82(2): p. 128-137.
[Non-Patent Document 24] Gao, J., et al., HIF-1/AKT Signaling-Activated PFKFB2 Alleviates Cardiac Dysfunction and Cardiomyocyte Apoptosis in Response to Hypoxia. Int Heart J, 2021. 62(2): p. 350-358.
[Non-Patent Document 25] Bergeron, R., et al., Chronic activation of AMP kinase results in NRF-1 activation and mitochondrial biogenesis. Am J Physiol Endocrinol Metab, 2001. 281(6): p. E1340-6.
[Non-Patent Document 26] Lantier, L., et al., AMPK controls exercise endurance, mitochondrial oxidative capacity, and skeletal muscle integrity. Faseb j, 2014. 28(7): p. 3211-24.
[Non-Patent Document 27] Settembre, C., et al., TFEB controls cellular lipid metabolism through a starvation-induced autoregulatory loop. Nat Cell Biol, 2013. 15(6): p. 647-58.
[Non-Patent Document 28] Jäger, S., et al., AMP-activated protein kinase(AMPK) action in skeletal muscle via direct phosphorylation of PGC-1alpha. Proc Natl Acad Sci U S A, 2007. 104 (29): p. 12017-22.
[Non-Patent Document 29] Chen, L., et al., PGC-1α-Mediated Mitochondrial Quality Control: Molecular Mechanisms and Implications for Heart Failure. Front Cell Dev Biol, 2022. 10: p. 871357.
[Non-Patent Document 30] Tian, L., et al., Pretreatment with Tilianin improves mitochondrial energy metabolism and oxidative stress in rats with myocardial ischemia/reperfusion injury via AMPK/SIRT1/PGC-1 alpha signaling pathway. J Pharmacol Sci, 2019. 139(4): p. 352-360.

[Detailed Description of the Invention]

[Technical Problem]

As a result of diligent efforts to investigate the physiological functions of 1,1-diethoxyethane (1,1-diethoxyethane, 1,1-DEE), the inventors confirmed that 1,1-DEE phosphorylates the Ser172 residue of AMPK (AMP-activated protein kinase), reversibly activating AMPK within 5 to 10 minutes, and that its efficacy can be further enhanced through a partially ROS (reactive oxygen species)-dependent mechanism. Furthermore, long-term exposure of cells to 1,1-DEE resulted in sustained AMPK activation and increased expression of PGC-1$\alpha$, a gene associated with mitochondrial metabolism. These findings demonstrate that 1,1-DEE can provide both acute and long-term activation effects of AMPK.

Accordingly, the present invention aims to provide an AMPK (AMP-activated protein kinase) activator comprising 1,1-diethoxyethane (1,1-DEE) as an active ingredient, as well as pharmaceutical compositions, cosmetic compositions, food compositions, or feed compositions for preventing, improving, or treating diseases requiring AMPK activation, anti-aging, extending lifespan, or enhancing physical vitality.

Furthermore, as a result of diligent efforts to investigate the physiological functions of 1,1-diethoxyethane (1,1-diethoxyethane, 1,1-DEE), the inventors confirmed that 1,1-DEE induces oxidative inactivation of PTEN (phosphatase and tensin homolog deleted on chromosome 10) through the formation of a disulfide bond between the Cys124 and Cys71 residues. This oxidative inactivation of PTEN leads to increased phosphorylation of Akt at Ser473 and Thr308, thereby activating Akt. Akt activation, in turn, increases phosphorylation of PFKFB2 (6-phosphofructo-2-kinase/fructose-2,6-biphosphatase 2) at the Ser483 residue, which can promote the rate of glycolysis. Furthermore, co-treatment with 1,1-DEE and insulin was shown to enhance Akt activation, thereby improving insulin sensitivity, and 1,1-DEE was also found to alleviate palmitate-induced insulin resistance.

Accordingly, the present invention aims to provide a composition for increasing insulin sensitivity or improving insulin resistance comprising 1,1-diethoxyethane (1,1-DEE) as an active ingredient, as well as pharmaceutical compositions, cosmetic compositions, food compositions, or feed compositions for preventing, improving, or treating diseases associated with insulin resistance comprising 1,1-diethoxyethane (1,1-DEE) as an active ingredient.

Furthermore, the inventors investigated the effects of 1,1-diethoxyethane (1,1-diethoxyethane, 1,1-DEE) on body weight gain, blood glucose regulation, insulin resistance, liver damage, and lipogenesis in obesity and metabolic disease models induced by a high-fat diet (HFD). The results confirmed that 1,1-DEE can suppress body weight gain in HFD mice, improve blood glucose levels and insulin resistance, alleviate liver damage and lipogenesis, and reduce the amount of LDL cholesterol in the blood.

Accordingly, the present invention aims to provide pharmaceutical compositions, cosmetic compositions, food compositions, or feed compositions for preventing, improving, or treating obesity or related metabolic diseases, comprising 1,1-diethoxyethane (1,1-DEE) as an active ingredient.

However, the problems to be solved by the present invention are not limited to the aforementioned ones, and other unmentioned problems can be clearly understood by those skilled in the art from the following description.

[Technical Solution]

The present invention discloses a composition comprising 1,1-diethoxyethane (1,1-DEE) as an active ingredient, as a pharmaceutical composition, a cosmetic composition, a food composition, or a feed composition.

In the present invention, the 1,1-diethoxyethane (1,1-DEE) has the molecular formula $C_6H_{14}O_2$ and is represented by the following Structural Formula 1. It is also known as acetaldehyde diethyl acetal or ethylidene diethyl ether.

[Structural Formula 1]

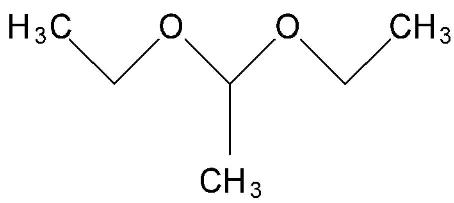

1. AMPK Activator

According to one embodiment, an AMPK (AMP-activated protein kinase) activator comprising 1,1-diethoxyethane (1,1-DEE) as an active ingredient is disclosed.

In the present invention, the 1,1-diethoxyethane may induce phosphorylation of the Ser172 residue of AMPK, thereby promoting fatty acid oxidation and regulating glycolysis.

In the present invention, the 1,1-diethoxyethane may transiently inhibit oxidative phosphorylation (OXPHOS) and activate AMPK through a mechanism mediated by reactive oxygen species (ROS).

In the present invention, the 1,1-diethoxyethane may increase the expression of PGC-1$\alpha$ (peroxisome proliferator-activated receptor gamma coactivator-1 alpha), thereby promoting mitochondrial biosynthesis.

According to another embodiment, a pharmaceutical composition for preventing or treating diseases requiring AMPK (AMP-activated protein kinase) activation, for anti-aging, lifespan extension, or enhancement of physical vitality, comprising 1,1-diethoxyethane (1,1-DEE) as an active ingredient is disclosed.

In the present invention, the diseases requiring AMPK activation may include one or more selected from metabolic disorders such as diabetes, obesity, nonalcoholic fatty liver disease (NAFLD), nonalcoholic steatohepatitis (NASH), liver fibrosis, and dyslipidemia; cardiovascular diseases such as atherosclerosis, hypertension, heart failure, and ischemic heart disease; neurodegenerative diseases such as Alzheimer's disease and Parkinson's disease; inflammatory diseases such as inflammatory bowel disease and rheumatoid arthritis; leukemia; and cancer.

In the present invention, the enhancement of physical vitality may include one or more selected from improved immunity, improved physical strength, enhanced endurance, increased energy levels, increased vitality, enhanced physical resilience, or support for sustained physical activity.

In the present invention, the pharmaceutical composition may be administered by one or more administration routes selected from the group consisting of oral administration, intravenous injection, subcutaneous injection, intramuscular injection, intraperitoneal injection, epithelial administration, topical administration, intravaginal administration, pulmonary administration, rectal administration, sublingual administration, buccal administration, transdermal administration, ocular administration, inhalation, intracavernous injection, intrathecal injection, epidural injection, and rectal administration.

According to another embodiment, a cosmetic composition for preventing or improving diseases requiring AMPK (AMP-activated protein kinase) activation, for anti-aging, lifespan extension, or enhancement of physical vitality, comprising 1,1-diethoxyethane (1,1-DEE) as an active ingredient is disclosed.

In the present invention, the cosmetic composition may be formulated into one or more dosage forms selected from the group consisting of solution, topical ointment, cream, foam, nourishing toner, softening toner, perfume, pack, softening lotion, emulsion, makeup base, essence, soap, liquid cleanser, bath agent, sunscreen cream, sun oil, suspension, emulsion, paste, gel, lotion, powder, soap, surfactant-containing cleansing, oil, powder foundation, emulsion foundation, wax foundation, patch, and spray.

According to another embodiment, a food composition for preventing or improving diseases requiring AMPK (AMP-activated protein kinase) activation, for anti-aging, lifespan extension, or enhancement of physical vitality, comprising 1,1-diethoxyethane (1,1-DEE) as an active ingredient is disclosed.

In the present invention, the food may include meat, sausage, bread, chocolate, candy, snacks, cookies, pizza, ramen, other noodles, chewing gum, ice cream and dairy products, various soups, beverages, tea, coffee beverages, stamina drinks, alcoholic beverages, or vitamin complexes.

According to another embodiment, a feed composition for preventing or improving diseases requiring AMPK (AMP-activated protein kinase) activation, for anti-aging, lifespan extension, or enhancement of physical vitality, comprising 1,1-diethoxyethane (1,1-DEE) as an active ingredient is disclosed.

In the present invention, the feed may include powdered feed, solid feed, moist pellet feed, dry pellet feed, extruder pellet (EP) feed, or raw feed.

2. Composition for Increasing Insulin Sensitivity or Improving Insulin Resistance

According to one embodiment, a composition for increasing insulin sensitivity or improving insulin resistance comprising 1,1-diethoxyethane (1,1-DEE) as an active ingredient is disclosed.

In the present invention, the 1,1-diethoxyethane may induce oxidative inactivation of PTEN through disulfide bond formation between Cys124 and Cys71 residues of PTEN.

In the present invention, the 1,1-diethoxyethane may activate Akt by increasing phosphorylation at Ser473 and Thr308 of Akt.

In the present invention, the 1,1-diethoxyethane may promote glycolysis by increasing phosphorylation at the Ser483 residue of PFKFB2 (6-phosphofructo-2-kinase/fructose-2,6-biphosphatase 2).

According to another embodiment, a pharmaceutical composition for preventing or treating diseases related to insulin resistance comprising 1,1-diethoxyethane (1,1-DEE) as an active ingredient is disclosed.

In the present invention, the diseases related to insulin resistance may include one or more selected from metabolic disorders such as Type 1 Diabetes, Type 2 Diabetes, Impaired Glucose Tolerance (IGT), Impaired Fasting Glucose (IFG), Hyperglycemia, Postprandial Hyperglycemia, Polycystic Ovary Syndrome (PCOS), Hyperlipidemia, Hypertension, Overweight, Obesity, and Metabolic Syndrome; glucose regulation disorders including reduction of Fasting Plasma Glucose (FPG), Postprandial Plasma Glucose (PPG), and/or glycated hemoglobin (HbA1c) and improvement of

blood glucose control; diabetes progression-related disorders including prevention, slowing, delay, or reversal of progression from IGT, IFG, insulin resistance, or Metabolic Syndrome to Type 2 Diabetes; diabetic complications including Cataracts, Microvascular and Macrovascular Diseases, Nephropathy, Retinopathy, Neuropathy, Tissue Ischemia, Diabetic Foot, Atherosclerosis, Myocardial Infarction, Acute Coronary Syndrome, Unstable Angina, Stable Angina, Stroke, Peripheral Artery Occlusive Disease, Cardiomyopathy, Heart Failure, Cardiac Arrhythmia, and Vascular Restenosis; weight management disorders including Weight Loss, Prevention of Weight Gain, or Promotion of Weight Loss; beta cell-related disorders including prevention, slowing, delay, or treatment of Beta Cell Degeneration and/or Beta Cell Dysfunction, improvement and/or recovery of pancreatic beta cell function, and/or recovery of pancreatic insulin secretion; liver disorders including prevention, slowing, delay, or treatment of diseases or conditions caused by Abnormal Accumulation of Liver Fat; and insulin-related disorders including maintenance and/or improvement of Insulin Sensitivity, prevention or treatment of Hyperinsulinemia and/or Insulin Resistance.

In the present invention, the pharmaceutical composition may be administered by one or more administration routes selected from the group consisting of oral administration, intravenous injection, subcutaneous injection, intramuscular injection, intraperitoneal injection, epithelial administration, topical administration, intravaginal administration, pulmonary administration, rectal administration, sublingual administration, buccal administration, transdermal administration, ocular administration, inhalation, intracavernous injection, intrathecal injection, epidural injection, and rectal administration.

According to another embodiment, a cosmetic composition for preventing or improving diseases related to insulin resistance, comprising 1,1-diethoxyethane (1,1-DEE) as an active ingredient is disclosed.

In the present invention, the cosmetic composition may be formulated into one or more dosage forms selected from the group consisting of solution, topical ointment, cream, foam, nourishing toner, softening toner, perfume, pack, softening lotion, emulsion, makeup base, essence, soap, liquid cleanser, bath agent, sunscreen cream, sun oil, suspension, emulsion, paste, gel, lotion, powder, soap, surfactant-containing cleansing, oil, powder foundation, emulsion foundation, wax foundation, patch, and spray.

According to another embodiment, a food composition for preventing or improving diseases related to insulin resistance, comprising 1,1-diethoxyethane (1,1-DEE) as an active ingredient is disclosed.

In the present invention, the food may include meat, sausage, bread, chocolate, candy, snacks, cookies, pizza, ramen, other noodles, chewing gum, ice cream and dairy products, various soups, beverages, tea, coffee beverages, stamina drinks, alcoholic beverages, or vitamin complexes.

According to another embodiment, a feed composition for preventing or improving diseases related to insulin resistance, comprising 1,1-diethoxyethane (1,1-DEE) as an active ingredient, is disclosed.

In the present invention, the feed may include powdered feed, solid feed, moist pellet feed, dry pellet feed, extruder pellet (EP) feed, or raw feed.

3. A composition for Preventing, Improving, or Treating Obesity or Related Metabolic Diseases.

According to one embodiment, a pharmaceutical composition for preventing or treating obesity or related metabolic diseases, comprising 1,1-diethoxyethane (1,1-DEE) as an active ingredient, is disclosed.

In the present invention, the obesity-related metabolic diseases may include type 2 diabetes, fatty liver, hyperlipidemia, hypertension, insulin resistance, atherosclerosis, stroke, polycystic ovary syndrome (PCOS), metabolic syndrome, inflammatory bowel disease (IBD), and sleep apnea.

In the present invention, the pharmaceutical composition may be administered by one or more administration routes selected from the group consisting of oral administration, intravenous injection, subcutaneous injection, intramuscular injection, intraperitoneal injection, epithelial administration, topical administration, intravaginal administration, pulmonary administration, rectal administration, sublingual administration, buccal administration, transdermal administration, ocular administration, inhalation, intracavernous injection, intrathecal injection, epidural injection, oral mucosa administration, intrabronchial administration, intralymphatic administration, head and neck administration, intracardiac administration, and rectal administration.

In the present invention, the pharmaceutical composition may be supported on a delivery vehicle, and the delivery vehicle may include one or more selected from virus particles, vesicles, nanoparticles, microparticles, liposomes, transposons, micelles, antibodies, and exosomes.

According to another embodiment, a cosmetic composition for preventing or improving cellulite comprising 1,1-diethoxyethane (1,1-DEE) as an active ingredient is disclosed.

In the present invention, the cosmetic composition may be formulated into one or more dosage forms selected from the group consisting of solution, topical ointment, cream, foam, nourishing toner, softening toner, perfume, pack, softening lotion, emulsion, makeup base, essence, soap, liquid cleanser, bath agent, sunscreen cream, sun oil, suspension, emulsion, paste, gel, lotion, powder, soap, surfactant-containing cleansing, oil, powder foundation, emulsion foundation, wax foundation, patch, spray, body lotion, hand cream, massage cream, hair tonic, foot cream, body scrub,

facial mask, serum, hair mask, toner, and mist.

According to another embodiment, a food composition for preventing or improving obesity or related metabolic diseases comprising 1,1-diethoxyethane (1,1-DEE) as an active ingredient is disclosed.

In the present invention, the obesity-related metabolic diseases may include type 2 diabetes, fatty liver, hyperlipidemia, hypertension, insulin resistance, atherosclerosis, stroke, polycystic ovary syndrome (PCOS), metabolic syndrome, inflammatory bowel disease (IBD), and sleep apnea.

In the present invention, the food may include meat, sausage, bread, chocolate, candy, snacks, cookies, pizza, ramen, other noodles, chewing gum, ice cream and dairy products, various soups, beverages, tea, coffee beverages, stamina drinks, alcoholic beverages, vitamin complexes, fruit juices, desserts, dietary supplements, instant foods, seasonings and sauces, and processed meat products.

According to another embodiment, a feed composition for preventing or improving obesity or related metabolic diseases comprising 1,1-diethoxyethane (1,1-DEE) as an active ingredient is disclosed.

In the present invention, the obesity-related metabolic diseases may include type 2 diabetes, fatty liver, hyperlipidemia, hypertension, insulin resistance, atherosclerosis, stroke, polycystic ovary syndrome (PCOS), metabolic syndrome, inflammatory bowel disease (IBD), and sleep apnea.

In the present invention, the feed may include powdered feed, solid feed, moist pellet feed, dry pellet feed, extruder pellet (EP) feed, wet feed, snacks, special feed, functional feed, dry feed, rice and grain feed, natural feed, or raw feed.

[Effects of the Invention]

The 1,1-diethoxyethane (1,1-DEE) according to the present invention may provide the following effects.

(1) 1,1-DEE can rapidly and reversibly activate AMPK through ATP depletion and ROS generation, and the activated AMPK can inhibit fatty acid synthesis and promote β-oxidation through ACC phosphorylation, and regulate glycolysis through PFKFB2 phosphorylation. 1,1-DEE can increase the expression of PGC-1α by activating AMPK, thereby enhancing mitochondrial biogenesis and energy metabolism. Therefore, compositions comprising 1,1-DEE according to the present invention may be useful not only for the prevention, treatment, or improvement of diseases requiring AMPK activation, but also as pharmaceutical compositions, cosmetic compositions, food compositions, or feed compositions for anti-aging, lifespan extension, and enhancement of physical vitality.

(2) 1,1-DEE can induce oxidative inactivation of PTEN, thereby increasing Akt activation and PFKFB2 Ser483 phosphorylation to promote glycolysis. 1,1-DEE can improve insulin sensitivity and alleviate palmitate-induced insulin resistance. Therefore, compositions comprising 1,1-DEE according to the present invention may be useful as pharmaceutical compositions, cosmetic compositions, food compositions, or feed compositions for the prevention, treatment, or improvement of insulin resistance-related diseases.

(3) 1,1-DEE can reduce body weight gain and improve fat accumulation in eWAT and iWAT in high-fat diet (HFD)-induced obese mice. 1,1-DEE can also improve glucose tolerance and enhance insulin sensitivity. Moreover, 1,1-DEE can decrease biomarkers of HFD-induced liver injury such as ALT, AST, and LDH, and improve TG and LDL-C levels, thereby reducing fat accumulation and the risk of atherosclerosis. Therefore, compositions comprising 1,1-DEE according to the present invention may be useful as pharmaceutical compositions, food compositions, cosmetic compositions, or feed compositions for the prevention, treatment, or improvement of obesity, diabetes, fatty liver, and related metabolic diseases.

However, the scope of the present invention is not limited by the above-described effects.

[Brief Description of Drawings]

FIG. 1 illustrates the reversible inhibitory effect on oxygen consumption rate and glycolysis in AC16 cells upon acute exposure to 1,1-DEE: (A) 2D structure of 1,1-diethoxyethane (1,1-DEE). (B) Cell viability of AC16 cells cultured for 24 hours with the indicated concentrations of 1,1-DEE, as determined using the EZ-Cytox assay. (C) Mitochondrial respiration was assessed using the Agilent Seahorse XF96 Cell Mito Stress Test Kit after acute exposure to 1,1-DEE, metformin, or 1,2-DEE for 30 minutes. Oxygen consumption rate (OCR) was measured following sequential addition of various concentrations of 1,1-DEE, 1.5 μM oligomycin (Oligo), 1.5 μM FCCP, and 0.5 μM rotenone/antimycin A (R/A). (D) Proton leak, ATP production, maximal respiration, and spare respiratory capacity were measured using the Seahorse XF96 Cell Mito Stress Test Kit.

(E) Extracellular acidification rate (ECAR) was measured under the conditions described in (C).

FIG. 2 illustrates the concentration- and time-dependent activation of AMPK upon acute exposure to 1,1-DEE. Western blot analysis of AC16 cells was performed to assess the phosphorylation of AMPK under the following conditions: (A) Cells cultured for 10 minutes with various concentrations of 1,1-DEE; (B) Cells cultured for 30 minutes with 15 mM 1,1-DEE; (C) Cells cultured for 30 minutes with 15 mM 1,2-DEE; and (D) Cells cultured for 60 minutes with 15 mM metformin.

FIG. 3 illustrates the effects of AMPK activation on fatty acid oxidation and glycolysis upon acute exposure to 1,1-DEE: (A) AC16 cells were exposed to 15 mM 1,1-DEE for 30 minutes. Phosphorylation of AMPK, ACC, and PFKFB2 was assessed by Western blot analysis. (B) AC16 cells were exposed to 15 mM 1,1-DEE for 8 hours. mRNA expression levels of SREBP-1c and FASN were evaluated using semi-quantitative PCR and qRT-PCR. (C) Wild-type (WT) and AMPK double knockout (DKO) MEF cells were exposed to 15 mM 1,1-DEE for 30 minutes. (D) AC16 cells were pretreated with 10 $\mu$M compound C for 1 hour, followed by incubation with 15 mM 1,1-DEE for 120 minutes. Western blot analysis was performed to assess the phosphorylation of AMPK effectors, including ACC and PFKFB2.

FIG. 4 illustrates that AMPK activation may be partially mediated by 1,1-DEE-induced ROS generation. AC16 cells were pretreated with 10 mM NAC for 2 hours or 10 $\mu$M ebselen for 1 hour, followed by exposure to 15 mM 1,1-DEE for 30 minutes. ROS generation was observed by immunofluorescence staining using DCFH-DA, and AMPK activation was assessed by Western blot analysis through phosphorylation at the Thr172 residue.

Figure 5 illustrates that long-term exposure to 1,1-DEE may increase the expression of PGC-1$\alpha$ through AMPK activation. (A-C) AC16 cells were cultured with 15 mM 1,1-DEE for up to 8 hours: (A) AMPK phosphorylation was evaluated by Western blot analysis. (B) The mRNA expression level of PPARGC1A was analyzed by RT-PCR. (C) The protein expression level of PGC-1$\alpha$ was confirmed by Western blot analysis. (D) MEF cells were cultured with 15 mM 1,1-DEE for up to 12 hours, after which AMPK phosphorylation and PGC-1$\alpha$ expression were evaluated by Western blot analysis.

Figure 6 illustrates that PGC-1$\alpha$-mediated AMPK activation may promote mitochondrial biogenesis. (A-B) AC16 cells were cultured with 15 mM 1,1-DEE for up to 8 hours: (A) The mRNA expression levels of Nrfl, Nrf2, and Tfam were evaluated by real-time PCR. (B) The protein expression levels of Nrfl, Nrf2, and Tfam were analyzed by Western blot. (C) WT and AMPK DKO MEF cells were cultured with 15 mM 1,1-DEE for up to 8 hours, after which the protein expression levels of Nrfl, Nrf2, and Tfam were confirmed by Western blot analysis. (D) AC16 cells were pretreated with compound C (10 $\mu$M) for 4 hours and then exposed to 15 mM 1,1-DEE for 24 hours, and the protein expression level of Tfam was evaluated by Western blot analysis. (E) AC16 cells were pretreated with compound C (10 $\mu$M) for 4 hours and then exposed to 15 mM 1,1-DEE for 8 hours, followed by staining with MitoTracker Red CMXRos (200 nM) for 30 minutes. Mitochondrial biogenesis was evaluated at 40× magnification using confocal microscopy. (F) Flow cytometric analysis of AC16 cells was performed under the same conditions as in (E).

Figure 7 illustrates 1,1-DEE detected in various ethanol batches: (A) HepG2 cells were treated with 100 mM E1, E2, or E3 for 10 minutes. (B) The lyophilization process flow of 100 mM E1, E2, and E4. (C) Cells were treated with 100 mM E1, E2, or E4, or the corresponding lyophilized samples described in (B) for 10 minutes. After treatment, cell extracts were alkylated with 10 mM NEM, followed by non-reducing or reducing electrophoresis and staining with PTEN and $\beta$-actin antibodies. (D) The presence and chemical structure of 1,1-DEE confirmed by GC-MS analysis of E1.

Figure 8 illustrates that 1,1-DEE can induce PTEN oxidation in a concentration-dependent manner: (A) C2C12, MEF, AC16, and Ea.hy926 cells were treated with various concentrations of 1,1-DEE (0-50 mM) for 10 minutes. (B) C2C12 cells were treated with the same concentrations of 1,2-DEE as in (A). Cell lysates were alkylated with 10 mM NEM, and Western blot analysis was performed using PTEN and GAPDH antibodies to assess the level of PTEN oxidation. (C) HepG2 cells were transfected with HA-tagged pCGN PTEN WT, C71S, C124S, or C71S/C124S. After transfection, cells were treated with 10 mM 1,1-DEE for 10 minutes. Following treatment, cells were lysed, alkylated with 10 mM NEM, and immunoblotting was performed using an antibody against the HA tag.

Figure 9 illustrates that 1,1-DEE can induce PTEN oxidation in a time-dependent manner: (A) C2C12, MEF, AC16, and EA.hy926 cells were treated with 10 mM 1,1-DEE for various time intervals up to 120 minutes. (B) C2C12 cells were treated under the same conditions as in (A) with 10 mM 1,2-DEE. Cell lysates were alkylated with 10 mM NEM, followed by reducing or non-reducing electrophoresis, and immunoblot analysis was performed using a PTEN antibody.

Figure 10 illustrates that PTEN oxidation induced by 1,1-DEE can activate the Akt signaling pathway: (A) C2C12 cells were treated with various concentrations of 1,1-DEE for 10 minutes. (B) C2C12 cells were treated with 10 mM 1,1-DEE for various time intervals up to 120 minutes. Cell lysates were used for immunoblot analysis with antibodies against phosphorylated Akt at Ser473 and Thr308, as well as total Akt.

Figure 11 illustrates that PTEN oxidation induced by 1,1-DEE is mediated by ROS generation: (A) HepG2 cells were treated with 10 mM 1,1-DEE or 0.5 mM H2O2 for 10 minutes. Cells were then stained with 10 $\mu$M DCFH-DA for 30 minutes, and representative images of stained cells were acquired using a fluorescence microscope at 10× magnification. (B) HepG2 cells were treated with 0.5 mM H2O2 for 10 minutes or with 10 mM 1,1-DEE for various time intervals up to 30 minutes. Cells were subsequently stained with 10 $\mu$M DCFH-DA, and flow cytometric analysis

was performed with 10,000 events per sample using the FITC channel. (C) HepG2 cells were pretreated with 10 mM NAC for 120 minutes and then treated with 10 mM 1,1-DEE for various time intervals up to 120 minutes. Cell lysates were alkylated with 10 mM NEM, followed by non-reducing or reducing immunoblotting using antibodies against PTEN, phosphorylated Akt, Akt, and GAPDH.

Figure 12 illustrates that 1,1-DEE can promote its effects through Akt activation: (A) AC16 cells were pretreated with 10 μM MK-2206 for 24 hours and then treated with 10 mM 1,1-DEE for 30 minutes. (B) AC16 cells were pretreated with 10 μM Ebselen for 60 minutes and then treated with 10 mM 1,1-DEE for various time intervals up to 30 minutes. Cell lysates were alkylated with 10 mM NEM, followed by Western blot analysis using antibodies against PTEN, phosphorylated Akt, Akt, phosphorylated PFKFB2, PFKFB2, and GAPDH.

Figure 13 illustrates that 1,1-DEE can enhance insulin sensitivity and alleviate palmitate-induced insulin resistance: (A) C2C12 cells were treated with various concentrations of 1,1-DEE (0-10 mM) for 10 minutes in the presence or absence of 20 nM insulin. (B) C2C12 cells were pretreated with 500 μM palmitate for 24 hours and then treated with 10 mM 1,1-DEE, 20 nM insulin, or both for 10 minutes. Cell lysates were alkylated with 10 mM NEM, followed by Western blot analysis using antibodies against PTEN, phosphorylated Akt, Akt, and GAPDH.

Figure 14 illustrates the protocol for establishing a HFD-induced obesity model and the administration scheme of 1,1-DEE.

Figure 15 illustrates the effects of 1,1-DEE on body weight changes and fat accumulation in an HFD-induced obesity model: (A) Representative images of mice in each treatment group, (B) Changes in body weight, (C) Changes in food intake, (D) Changes in eWAT fat accumulation, (E) Changes in iWAT fat accumulation, (F) Changes in heart weight, (G) Changes in liver weight, and (H) Changes in colon length.

Figure 16 illustrates the effect of 1,1-DEE on suppressing body weight gain in HFD mice. Eight-week-old male mice were divided into six groups and administered different diets. The groups were as follows: ND (normal diet), ND+1,1-DEE (treated with 1,1-DEE 100 mg/kg), HFD (high-fat diet), HFD+1,1-DEE (treated with 1,1-DEE 100 mg/kg), HFD+Met (treated with metformin 100 mg/kg), and HFD+1,2-DEE (treated with 1,2-DEE 100 mg/kg) ($n = 7$ per group). After 4 weeks of treatment, the following parameters were measured: (A) 4-week body weight changes in each treatment group, (B) body weight comparison at week 12, and (C) food intake. Data are presented as mean ± SEM, and statistical significance was evaluated using an unpaired two-tailed Student's t-test (nsp > 0.05, $p \leq 0.01$)

Figure 17 illustrates the effects of 1,1-DEE on blood glucose and insulin sensitivity in HFD-induced obese mice: (A) Data on random blood glucose changes, (B) Changes in fasting blood glucose, (C) Glucose tolerance test, (D) Changes in serum insulin levels, and (E) Insulin tolerance test results.

Figure 18 illustrates the effects of 1,1-DEE on improving liver injury and lipid production in HFD-induced obese mice: (A) Changes in ALT levels, (B) Changes in AST levels, (C) Changes in LHD levels, (D) Changes in ALP levels, (E) Changes in T-Bil levels, (F) Changes in Alb levels, (G) Changes in TG levels, (H) Changes in T-chol levels, (I) Changes in HDL-C levels, and (K) Changes in LDL-C levels.

[DETAILED DESCRIPTION OF THE EXEMPLARY EMBODIMENTS]

Hereinafter, detailed descriptions are provided for an AMPK activator comprising 1,1-diethoxyethane as an active ingredient, a composition for increasing insulin sensitivity or improving insulin resistance comprising 1,1-diethoxyethane as an active ingredient, and a composition for preventing or treating obesity or related metabolic disorders comprising 1,1-diethoxyethane as an active ingredient, as well as the uses thereof, according to specific embodiments of the invention. However, this is presented merely as one example of the invention, and the scope of the invention is not limited thereby. It will be apparent to those skilled in the art that various modifications of the embodiments are possible within the scope of the claims of the invention. Unless otherwise specifically stated in the present specification, the terms "comprising" or "containing" refer to the inclusion of certain components (or constituents) without particular limitation, and should not be construed as excluding the presence of other components (or constituents).

As used herein, the term "treatment" refers to any form of therapy or prevention administered to a subject who has a disease or is at risk of developing a disease, and provides effects such as improvement of the subject's condition, delay in disease progression, delay in symptom onset, or slowing of symptom progression. Accordingly, the term "treatment" encompasses prophylactic treatment to prevent the occurrence of symptoms. In addition, the terms "treatment" and "prevention" are not intended to imply the complete cure or elimination of symptoms.

As used herein, the term "improvement" may refer to any action that alleviates a condition or reduces at least the severity of symptoms or parameters related to treatment.

As used herein, the term "subject" refers to an animal, including, but not limited to, cattle, monkeys, horses, sheep, pigs, chickens, turkeys, quails, cats, dogs, mice, rats, rabbits, or guinea pigs. For example, the subject may be a mammal, and particularly, a human.

## 1. AMPK Activator

The present invention provides an AMPK (AMP-activated protein kinase) activator comprising 1,1-diethoxyethane (1,1-DEE) as an active ingredient.

In the AMPK activator according to the present invention, the 1,1-diethoxyethane may induce phosphorylation of the Ser172 residue of AMPK, thereby promoting fatty acid oxidation and regulating glycolysis.

In the AMPK activator according to the present invention, the 1,1-diethoxyethane may inhibit oxidative phosphorylation (OXPHOS) and activate AMPK via the generation of reactive oxygen species (ROS).

In the AMPK activator according to the present invention, the 1,1-diethoxyethane may promote mitochondrial biosynthesis by increasing the expression of PGC-1α (peroxisome proliferator-activated receptor gamma coactivator-1 alpha).

In the AMPK activator according to the present invention, the 1,1-diethoxyethane may reduce malonyl-CoA production by inducing phosphorylation of ACC (acetyl-CoA carboxylase) in mitochondria.

In the AMPK activator according to the present invention, the AMPK activator may be used for preventing, treating, or improving one or more selected from metabolic disorders including Diabetes, Obesity, Nonalcoholic Fatty Liver Disease (NAFLD), Nonalcoholic Steatohepatitis (NASH), Liver Fibrosis, and Dyslipidemia; cardiovascular diseases including Atherosclerosis, Hypertension, Heart Failure, and Ischemic Heart Disease; neurodegenerative diseases including Alzheimer's Disease and Parkinson's Disease; inflammatory diseases including Inflammatory Bowel Disease and Rheumatoid Arthritis; Leukemia; and Cancer, but is not limited thereto. According to one exemplary embodiment, the AMPK activator may also be used as a blood glucose-lowering agent, cholesterol-lowering agent, or fatty acid-lowering agent.

In the AMPK activator according to the present invention, the AMPK activator may be used in the manufacture of a composition for anti-aging or lifespan extension.

In the AMPK activator according to the present invention, the AMPK activator may be used in the manufacture of a composition for enhancing immunity, improving physical strength, increasing physical endurance, increasing energy levels, enhancing vitality, improving physical recovery, or supporting sustained physical activity.

In the AMPK activator according to the present invention, the AMPK activator may also be used as a (stem) cell differentiation and maturation promoter.

In the AMPK activator according to the present invention, the concentration of the 1,1-diethoxyethane may be 1 mM to 25 mM.

## 2. Composition for Increasing Insulin Sensitivity or Improving Insulin Resistance

The present invention provides a composition for increasing insulin sensitivity or improving insulin resistance, comprising 1,1-diethoxyethane (1,1-DEE) as an active ingredient.

In the composition for increasing insulin sensitivity or improving insulin resistance according to the present invention, the 1,1-diethoxyethane may induce oxidative inactivation of PTEN by promoting the formation of a disulfide bond between the Cys124 and Cys71 residues of PTEN.

In the composition for increasing insulin sensitivity or improving insulin resistance according to the present invention, the 1,1-diethoxyethane may activate Akt by increasing phosphorylation at Ser473 and Thr308 of Akt.

In the composition for increasing insulin sensitivity or improving insulin resistance according to the present invention, the 1,1-diethoxyethane may promote glycolysis by increasing phosphorylation at the Ser483 residue of PFKFB2 (6-phosphofructo-2-kinase/fructose-2,6-biphosphatase 2).

In the composition for increasing insulin sensitivity or improving insulin resistance according to the present invention, the 1,1-diethoxyethane may be used for preventing, treating, or improving diseases associated with insulin resistance.

The composition for increasing insulin sensitivity or improving insulin resistance may include one or more selected from metabolic disorders, blood glucose regulation disorders, prevention or reversal of diabetes progression, diabetic complications, weight management disorders, protection of and improvement in pancreatic beta cell function, liver diseases, and insulin-related disorders.

In one embodiment, the metabolic disorders may include, but are not limited to, Type 1 Diabetes, Type 2 Diabetes, Impaired Glucose Tolerance (IGT), Impaired Fasting Glucose (IFG), Hyperglycemia, Postprandial Hyperglycemia, Polycystic Ovary Syndrome (PCOS), Hyperlipidemia, Hypertension, Overweight, Obesity, and Metabolic Syndrome.

In one embodiment, the blood glucose regulation disorders may include, but are not limited to, reduction of Fasting Plasma Glucose (FPG), Postprandial Plasma Glucose (PPG), and/or glycated hemoglobin (HbA1c), and improvement in blood glucose control.

In one embodiment, the disorders related to diabetes progression may include, but are not limited to, prevention,

retardation, delay, or reversal of progression from Impaired Glucose Tolerance (IGT), Impaired Fasting Glucose (IFG), Insulin Resistance, or Metabolic Syndrome to Type 2 Diabetes.

In one embodiment, the diabetic complications may include, but are not limited to, Cataracts, Microvascular and Macrovascular Diseases, Nephropathy, Retinopathy, Neuropathy, Tissue Ischemia, Diabetic Foot, Atherosclerosis, Myocardial Infarction, Acute Coronary Syndrome, Unstable Angina, Stable Angina, Stroke, Peripheral Artery Occlusive Disease, Cardiomyopathy, Heart Failure, Cardiac Arrhythmia, and Vascular Restenosis.

In one embodiment, the weight management disorders may include, but are not limited to, Weight Loss, Prevention of Weight Gain, or Promotion of Weight Loss.

In one embodiment, the pancreatic beta cell-related disorders may include, but are not limited to, prevention, retardation, delay, or treatment of Beta Cell Degeneration and/or Beta Cell Dysfunction; improvement and/or recovery of pancreatic beta cell function; and/or restoration of pancreatic insulin secretion function.

In one embodiment, the liver-related diseases may include, but are not limited to, prevention, retardation, delay, or treatment of diseases or conditions caused by abnormal accumulation of liver fat.

In one embodiment, the insulin-related disorders may include, but are not limited to, maintenance and/or improvement of Insulin Sensitivity, and prevention or treatment of Hyperinsulinemia and/or Insulin Resistance.

### 3. Composition for Preventing, Improving, or Treating Obesity or Related Metabolic Diseases

The present invention provides a composition for preventing, improving, or treating obesity or related metabolic diseases, comprising 1,1-diethoxyethane (1,1-DEE) as an active ingredient.

In the composition for preventing, improving, or treating obesity or related metabolic diseases according to the present invention, the obesity-related metabolic diseases may include, but are not limited to, Type 2 Diabetes, Fatty Liver, Hyperlipidemia, Hypertension, Insulin Resistance, Atherosclerosis, Stroke, Polycystic Ovary Syndrome (PCOS), Metabolic Syndrome, Inflammatory Bowel Disease (IBD), and Sleep Apnea.

### 4. Use

The present invention provides a composition comprising 1,1-diethoxyethane (1,1-DEE) as an active ingredient, the composition being a pharmaceutical composition, a cosmetic composition, a food composition, or a feed composition.

(1) Pharmaceutical Composition

In one embodiment,

the present invention provides a pharmaceutical composition for preventing or treating diseases requiring AMPK (AMP-activated protein kinase) activation, for anti-aging, for lifespan extension, or for enhancing physical vitality, comprising 1,1-diethoxyethane (1,1-DEE) as an active ingredient.

In the pharmaceutical composition according to the present invention, the diseases requiring AMPK activation may include, but are not limited to, metabolic disorders including Diabetes, Obesity, Nonalcoholic Fatty Liver Disease (NAFLD), Nonalcoholic Steatohepatitis (NASH), Liver Fibrosis, and Dyslipidemia; cardiovascular diseases including Atherosclerosis, Hypertension, Heart Failure, and Ischemic Heart Disease; neurodegenerative diseases including Alzheimer’s Disease and Parkinson’s Disease; inflammatory diseases including Inflammatory Bowel Disease and Rheumatoid Arthritis; and Leukemia and Cancer.

In the pharmaceutical composition according to the present invention, the enhancement of physical vitality may include, but is not limited to, one or more selected from enhancement of immune function, improvement of physical strength, increase in physical endurance, increase in energy levels, enhancement of vitality, improvement of physical recovery ability, or support for sustained physical activity.

In another embodiment,

the present invention provides a pharmaceutical composition for preventing or treating diseases associated with insulin resistance, comprising 1,1-diethoxyethane (1,1-DEE) as an active ingredient.

In the pharmaceutical composition according to the present invention, the diseases associated with insulin resistance may include, but are not limited to, metabolic disorders including Type 1 Diabetes, Type 2 Diabetes, Impaired Glucose Tolerance (IGT), Impaired Fasting Glucose (IFG), Hyperglycemia, Postprandial Hyperglycemia, Polycystic Ovary Syndrome (PCOS), Hyperlipidemia, Hypertension, Overweight, Obesity, and Metabolic Syndrome; glycemic control disorders including reduction of Fasting Plasma Glucose (FPG), Postprandial Plasma Glucose (PPG) and/or HbAlc, and improvement of glycemic control; diabetes progression-related disorders including prevention, slowing, delay, or reversal of progression from Impaired Glucose Tolerance (IGT), Impaired Fasting Glucose (IFG), Insulin Resistance, or Metabolic Syndrome to Type 2 Diabetes; diabetic complications including Cataracts, Microvascular and Macrovascular Diseases, Nephropathy, Retinopathy, Neuropathy, Tissue Ischemia, Diabetic Foot, Atherosclerosis, Myocardial Infarction, Acute Coronary Syndrome, Unstable Angina, Stable Angina, Stroke, Peripheral Artery Occlusive Disease, Cardiomyopathy, Heart Failure, Cardiac Arrhythmia, and Vascular Restenosis; weight management disorders including Weight Loss, Prevention of Weight Gain, or Promotion of Weight Loss; beta cell-related disorders including prevention, slowing, delay, or treatment of Beta Cell Degeneration and/or Beta Cell Dysfunction, improvement and/or recovery of beta cell function, and/or restoration of pancreatic insulin secretion function; liver diseases including prevention, slowing, delay, or treatment of diseases or conditions caused by Abnormal Accumulation of Liver Fat; and insulin-related disorders including maintenance and/or improvement of Insulin Sensitivity, and prevention or treatment of Hyperinsulinemia and/or Insulin Resistance.

In another embodiment,
the present invention provides a pharmaceutical composition for preventing or treating obesity or related metabolic diseases, comprising 1,1-diethoxyethane (1,1-DEE) as an active ingredient.

In the pharmaceutical composition according to the present invention, the obesity-related metabolic diseases may include, but are not limited to, Type 2 Diabetes, Fatty Liver, Hyperlipidemia, Hypertension, Insulin Resistance, Atherosclerosis, Stroke, Polycystic Ovary Syndrome (PCOS), Metabolic Syndrome, Inflammatory Bowel Disease (IBD), and Sleep Apnea.

The composition may be administered via oral administration, intravenous injection, subcutaneous injection, intramuscular injection, intraperitoneal injection, epithelial administration, topical administration, vaginal administration, pulmonary administration, rectal administration, sublingual administration, buccal administration, transdermal administration, ocular administration, inhalation, intracavernosal injection, intrathecal injection, epidural injection, or rectal administration, among others. For oral administration, for example, the pharmaceutical composition may be formulated as a tablet or may be coated or formulated to protect the active ingredient from degradation in the stomach. In addition, the composition may be administered by any device that allows the active substance to be delivered to target cells. The route of administration may vary depending on the general condition and age of the subject, the nature of the condition to be treated, and the choice of active ingredient.the pharmaceutical composition may be carried on a delivery vehicle. The delivery vehicle may include, but is not limited to, one or more selected from the group consisting of viral particles, vesicles, nanoparticles, microparticles, liposomes, transposons, micelles, antibodies, and exosomes.

The suitable dosage of the pharmaceutical composition may vary depending on factors such as the method of formulation, mode of administration, age, body weight, sex, pathological condition, food intake, time of administration, route of administration, rate of excretion, and responsiveness of the patient. A person skilled in the art can readily determine and prescribe an effective dose for the desired therapeutic or preventive effect. For example, the pharmaceutical composition may be administered as a single dose or in multiple doses, and may be administered one to four times per day. In one example, the pharmaceutical composition may be administered in an amount of 0.01 mg/kg to 100 mg/kg, preferably 0.02 mg/kg to 90 mg/kg, and more preferably 0.03 mg/kg to 80 mg/kg, based on an adult human subject.

The pharmaceutical composition may be prepared in a unit dosage form or contained within a multi-dose container by formulation using pharmaceutically acceptable carriers and/or excipients according to methods easily implementable by those skilled in the art. The formulation may be in the form of a solution, suspension, or emulsion in an oil or aqueous medium, or in the form of an extract, powder, granule, tablet, or capsule, and may further include a dispersing agent or stabilizer. In addition, the pharmaceutical composition may be administered in the form of a suppository, spray, ointment, cream, gel, inhalant, or transdermal patch. Furthermore, the pharmaceutical composition may be formulated for administration to mammals, and more preferably, for administration to humans. the pharmaceutically acceptable carrier may be solid or liquid, and may include one or more selected from the group consisting of excipients, antioxidants, buffers, bacteriostatic agents, dispersants, adsorbents, surfactants, binders, preservatives, disintegrants, sweeteners, flavoring agents, glidants, release controllers, humectants, stabilizers, suspending agents, and lubricants. Additionally, the pharmaceutically acceptable carrier may be selected from saline, sterile water, Ringer's solution, buffered saline, dextrose solution, maltodextrin solution, glycerol, ethanol, or mixtures thereof.

In one example, a suitable filler may include sugars (e.g., dextrose, sucrose, maltose, and lactose), starches (e.g., corn starch), sugar alcohols (e.g., mannitol, sorbitol, maltitol, erythritol, and xylitol), starch hydrolysates (e.g., dextrin and maltodextrin), cellulose or cellulose derivatives (e.g., microcrystalline cellulose), or mixtures thereof, but is not limited thereto.

In one example, a suitable binder may include povidone, copovidone, methylcellulose, hydroxyethylcellulose, hydroxypropylmethylcellulose, hydroxypropylcellulose, gelatin, gums, sucrose, starch, or mixtures thereof, but is not limited thereto.

In one example, a suitable preservative may include benzoic acid, sodium benzoate, benzyl alcohol, butylated hydroxyanisole, butylated hydroxytoluene, chlorobutanol, gallates, hydroxybenzoates, EDTA, or mixtures thereof, but is not limited thereto.

In one example, a suitable disintegrant may include sodium starch glycolate, cross-linked polyvinylpyrrolidone, cross-linked carboxymethylcellulose, starch, microcrystalline cellulose, or mixtures thereof, but is not limited thereto.

In one example, a suitable sweetener may include sucralose, saccharin, sodium, potassium, or calcium

saccharin, acesulfame potassium, sodium cyclamate, mannitol, fructose, sucrose, maltose, or mixtures thereof, but is not limited thereto.

In one example, a suitable glidant may include silica, colloidal silicon dioxide, or talc, but is not limited thereto.

In one example, a suitable lubricant may include long-chain fatty acids and their salts, such as magnesium stearate and stearic acid, talc, glyceryl wax, or mixtures thereof, but is not limited thereto.

(2) Cosmetic Composition

In one embodiment,
the present invention provides a cosmetic composition for preventing or improving diseases requiring AMPK (AMP-activated protein kinase) activation, for anti-aging, for lifespan extension, or for enhancing physical vitality, comprising 1,1-diethoxyethane (1,1-DEE) as an active ingredient.

In the cosmetic composition according to the present invention, the diseases requiring AMPK activation may include, but are not limited to, metabolic disorders including Diabetes, Obesity, Nonalcoholic Fatty Liver Disease (NAFLD), Nonalcoholic Steatohepatitis (NASH), Liver Fibrosis, and Dyslipidemia; cardiovascular diseases including Atherosclerosis, Hypertension, Heart Failure, and Ischemic Heart Disease; neurodegenerative diseases including Alzheimer's Disease and Parkinson's Disease; inflammatory diseases including Inflammatory Bowel Disease and Rheumatoid Arthritis; and Leukemia and Cancer.

In the cosmetic composition according to the present invention, the enhancement of physical vitality may include, but is not limited to, one or more selected from enhancement of immune function, improvement of physical strength, increase in physical endurance, increase in energy levels, enhancement of vitality, improvement of physical recovery ability, or support for sustained physical activity.

In the cosmetic composition according to the present invention, the cosmetic composition may be used for skin regeneration, wrinkle improvement, or skin moisturization.

In another embodiment,
the present invention provides a cosmetic composition for preventing or improving diseases associated with insulin resistance, comprising 1,1-diethoxyethane (1,1-DEE) as an active ingredient.

In the pharmaceutical composition according to the present invention, the diseases associated with insulin resistance may include, but are not limited to, metabolic disorders including Type 1 Diabetes, Type 2 Diabetes, Impaired Glucose Tolerance (IGT), Impaired Fasting Glucose (IFG), Hyperglycemia, Postprandial Hyperglycemia, Polycystic Ovary Syndrome (PCOS), Hyperlipidemia, Hypertension, Overweight, Obesity, and Metabolic Syndrome; glycemic control disorders including reduction of Fasting Plasma Glucose (FPG), Postprandial Plasma Glucose (PPG) and/or HbAlc, and improvement of glycemic control; diabetes progression-related disorders including prevention, slowing, delay, or reversal of progression from Impaired Glucose Tolerance (IGT), Impaired Fasting Glucose (IFG), Insulin Resistance, or Metabolic Syndrome to Type 2 Diabetes; diabetic complications including Cataracts, Microvascular and Macrovascular Diseases, Nephropathy, Retinopathy, Neuropathy, Tissue Ischemia, Diabetic Foot, Atherosclerosis, Myocardial Infarction, Acute Coronary Syndrome, Unstable Angina, Stable Angina, Stroke, Peripheral Artery Occlusive Disease, Cardiomyopathy, Heart Failure, Cardiac Arrhythmia, and Vascular Restenosis; weight management disorders including Weight Loss, Prevention of Weight Gain, or Promotion of Weight Loss; beta cell-related disorders including prevention, slowing, delay, or treatment of Beta Cell Degeneration and/or Beta Cell Dysfunction, improvement and/or recovery of beta cell function, and/or restoration of pancreatic insulin secretion function; liver diseases including prevention, slowing, delay, or treatment of diseases or conditions caused by Abnormal Accumulation of Liver Fat; and insulin-related disorders including maintenance and/or improvement of Insulin Sensitivity, and prevention or treatment of Hyperinsulinemia and/or Insulin Resistance.

In another embodiment,
the present invention provides a cosmetic composition for preventing or improving cellulite, comprising 1,1-diethoxyethane (1,1-DEE) as an active ingredient. As used herein, the term "cellulite" refers to the appearance of localized fat protrusions, particularly in the thigh or buttock regions, caused by circulatory disturbances resulting from excessive accumulation of fat and waste products. The fundamental cause of cellulite is an increase in or hypertrophy of adipocytes. Therefore, the term "prevention or improvement of cellulite" may refer to a reduction in the volume of cellulite, a decrease in the height of cellulite, removal of cellulite, or smoothing of cellulite and its surrounding area. The 1,1-diethoxyethane (1,1-DEE) according to the present invention has an effect of inhibiting fat accumulation, thereby preventing or improving cellulite. Furthermore, due to this effect, the composition exhibits a body slimming effect. The term "body slimming" may refer to promoting an even redistribution of fat in a body line where fat is unevenly distributed subcutaneously and appears bumpy, reducing the volume of the body or a part thereof, reducing swelling to thereby decrease the volume of the body or a part thereof, improving localized obesity, breaking down and eliminating fat within adipocytes, and reducing triglyceride and fluid accumulation within adipocytes.

The cosmetic composition may further comprise a dermatologically acceptable carrier. The dermatologically

acceptable carrier may include purified water, oil, wax, fatty acid, fatty alcohol, fatty acid ester, surfactant, humectant, thickener, antioxidant, viscosity stabilizer, chelating agent, buffer, preservative, and lower alcohol, but is not limited thereto. The type and concentration thereof may vary and can be modified by those skilled in the art within the scope of the present invention.

The cosmetic composition may further comprise, in addition to the active ingredient of the present invention, functional substances such as whitening agents, moisturizers, anti-inflammatory agents, antibacterial agents, antifungal agents, vitamins, ultraviolet (UV) blockers, antibiotics, anti-acne agents, perfumes, or dyes, which can be included in the cosmetic composition of the present invention in amounts conventionally used in the cosmetic field. To enhance its functional effects, the cosmetic composition of the present invention may further contain one or more moisturizing active ingredients exhibiting the same or similar function as the composition of the present invention.

The cosmetic composition may be prepared in the form of a conventional emulsion formulation or solubilized formulation. Examples of cosmetics in the form of an emulsion formulation include nourishing lotions, creams, and essences, and an example of a cosmetic in the form of a solubilized formulation includes softening lotion. The cosmetic composition may be prepared in the form of a topically or systemically applicable adjunct conventionally used in the art by further comprising a dermatologically acceptable carrier or vehicle in addition to the active ingredient of the present invention. Suitable cosmetic formulations include, for example, a solution, gel, solid or paste anhydrous product, an emulsion obtained by dispersing an oil phase in an aqueous phase, suspension, microemulsion, microcapsule, microparticulate form, ionic (liposomal) or non-ionic vesicular dispersions, cream, skin lotion, powder, ointment, spray, or conceal stick. In addition, the cosmetic composition may also be prepared in the form of a foam or an aerosol composition further comprising a compressed propellant.

The cosmetic composition may be formulated in one or more forms selected from the group consisting of a solution, topical ointment, cream, foam, nourishing lotion, softening lotion, perfume, pack, softening water, emulsion, makeup base, essence, soap, liquid cleanser, bath additive, sunscreen cream, sun oil, suspension, emulsion, paste, gel, lotion, powder, soap, surfactant-containing cleanser, oil, powder foundation, emulsion foundation, wax foundation, patch, and spray.

(3) Food Composition

As used herein, the term "food" refers to a natural product or processed product containing one or more nutrients, and preferably refers to a form that has undergone a certain degree of processing and is in a state suitable for direct consumption. In its conventional meaning, the term "food" may encompass foods, food additives, functional foods, and beverages.

As used herein, the terms "functional food" or "health-functional food" refer to a group of foods or food compositions to which added value has been imparted by physical, biochemical, biotechnological, or other methods, so that the functions of the respective food act or are expressed for a specific purpose. Such foods are designed and processed to sufficiently exhibit in vivo regulatory functions related to, for example, biological defense rhythm regulation, disease prevention, and recovery. Specifically, the functional food may be a health-functional food. The functional food may further comprise food-acceptable auxiliary additives and may additionally include suitable carriers, excipients, and diluents conventionally used in the production of functional foods. The types of the health supplements are not limited thereto, but may include, for example, powder, granule, tablet, capsule, or beverage forms.

In one embodiment,

the present invention provides a food composition for preventing or improving diseases requiring AMPK (AMP-activated protein kinase) activation, for anti-aging, for lifespan extension, or for enhancing physical vitality, comprising 1,1-diethoxyethane (1,1-DEE) as an active ingredient.

In the food composition according to the present invention, the diseases requiring AMPK activation may include, but are not limited to, metabolic disorders including Diabetes, Obesity, Nonalcoholic Fatty Liver Disease (NAFLD), Nonalcoholic Steatohepatitis (NASH), Liver Fibrosis, and Dyslipidemia; cardiovascular diseases including Atherosclerosis, Hypertension, Heart Failure, and Ischemic Heart Disease; neurodegenerative diseases including Alzheimer's Disease and Parkinson's Disease; inflammatory diseases including Inflammatory Bowel Disease and Rheumatoid Arthritis; and Leukemia and Cancer.

In the food composition according to the present invention, the enhancement of physical vitality may include, but is not limited to, one or more selected from enhancement of immune function, improvement of physical strength, increase in physical endurance, increase in energy levels, enhancement of vitality, improvement of physical recovery ability, or support for sustained physical activity.

In another embodiment,

the present invention provides a food composition for preventing or improving diseases associated with insulin resistance, comprising 1,1-diethoxyethane (1,1-DEE) as an active ingredient.

In the food composition according to the present invention, the diseases associated with insulin resistance may

include, but are not limited to, metabolic disorders including Type 1 Diabetes, Type 2 Diabetes, Impaired Glucose Tolerance (IGT), Impaired Fasting Glucose (IFG), Hyperglycemia, Postprandial Hyperglycemia, Polycystic Ovary Syndrome (PCOS), Hyperlipidemia, Hypertension, Overweight, Obesity, and Metabolic Syndrome; glycemic control disorders including reduction of Fasting Plasma Glucose (FPG), Postprandial Plasma Glucose (PPG) and/or HbAlc, and improvement of glycemic control; diabetes progression-related disorders including prevention, slowing, delay, or reversal of progression from Impaired Glucose Tolerance (IGT), Impaired Fasting Glucose (IFG), Insulin Resistance, or Metabolic Syndrome to Type 2 Diabetes; diabetic complications including Cataracts, Microvascular and Macrovascular Diseases, Nephropathy, Retinopathy, Neuropathy, Tissue Ischemia, Diabetic Foot, Atherosclerosis, Myocardial Infarction, Acute Coronary Syndrome, Unstable Angina, Stable Angina, Stroke, Peripheral Artery Occlusive Disease, Cardiomyopathy, Heart Failure, Cardiac Arrhythmia, and Vascular Restenosis; weight management disorders including Weight Loss, Prevention of Weight Gain, or Promotion of Weight Loss; beta cell-related disorders including prevention, slowing, delay, or treatment of Beta Cell Degeneration and/or Beta Cell Dysfunction, improvement and/or recovery of beta cell function, and/or restoration of pancreatic insulin secretion function; liver diseases including prevention, slowing, delay, or treatment of diseases or conditions caused by Abnormal Accumulation of Liver Fat; and insulin-related disorders including maintenance and/or improvement of Insulin Sensitivity, and prevention or treatment of Hyperinsulinemia and/or Insulin Resistance.

In another embodiment,
the present invention provides a food composition for preventing or improving obesity or related metabolic diseases, comprising 1,1-diethoxyethane (1,1-DEE) as an active ingredient.

In the food composition according to the present invention, the obesity-related metabolic diseases may include, but are not limited to, Type 2 Diabetes, Fatty Liver, Hyperlipidemia, Hypertension, Insulin Resistance, Atherosclerosis, Stroke, Polycystic Ovary Syndrome (PCOS), Metabolic Syndrome, Inflammatory Bowel Disease (IBD), and Sleep Apnea.

The food may be selected from the group consisting of meat, sausage, bread, chocolate, candy, snacks, confectioneries, pizza, ramen, other noodles, gum, dairy products including ice cream, various soups, beverages, tea, coffee drinks, stamina drinks, alcoholic beverages, or vitamin complexes.

The food composition may further comprise various nutritional supplements, vitamins, electrolytes, flavoring agents, coloring agents, pectic acid and salts thereof, alginic acid and salts thereof, organic acids, protective colloid thickeners, pH adjusters, stabilizers, preservatives, glycerin, alcohol, carbonating agents used in carbonated beverages, and the like. In addition, the composition of the present invention may further comprise pulp for preparing natural fruit juices, fruit juice beverages, or vegetable beverages. These ingredients may be used independently or in combination.

The term "functional food" or "health-functional food" refers to a group of foods or food compositions to which added value has been imparted by physical, biochemical, biotechnological, or other methods, such that the functions of the respective food act or are expressed for a specific purpose. Such foods are designed and processed to sufficiently exhibit in vivo regulatory functions related to biological defense rhythm regulation, disease prevention, and recovery. Specifically, the functional food may be a health-functional food. The functional food may further comprise food-acceptable auxiliary additives and may additionally include suitable carriers, excipients, and diluents conventionally used in the production of functional foods.

(4) Feed Composition

In one embodiment,
the present invention provides a Feed composition for preventing or improving diseases requiring AMPK (AMP-activated protein kinase) activation, for anti-aging, for lifespan extension, or for enhancing physical vitality, comprising 1,1-diethoxyethane (1,1-DEE) as an active ingredient.

In the Feed composition according to the present invention, the diseases requiring AMPK activation may include, but are not limited to, metabolic disorders including Diabetes, Obesity, Nonalcoholic Fatty Liver Disease (NAFLD), Nonalcoholic Steatohepatitis (NASH), Liver Fibrosis, and Dyslipidemia; cardiovascular diseases including Atherosclerosis, Hypertension, Heart Failure, and Ischemic Heart Disease; neurodegenerative diseases including Alzheimer's Disease and Parkinson's Disease; inflammatory diseases including Inflammatory Bowel Disease and Rheumatoid Arthritis; and Leukemia and Cancer.

In the feed composition according to the present invention, the enhancement of physical vitality may include, but is not limited to, one or more selected from enhancement of immune function, improvement of physical strength, increase in physical endurance, increase in energy levels, enhancement of vitality, improvement of physical recovery ability, or support for sustained physical activity.

In another embodiment,
the present invention provides a feed composition for preventing or improving diseases associated with insulin resistance, comprising 1,1-diethoxyethane (1,1-DEE) as an active ingredient.

In the feed composition according to the present invention, the diseases associated with insulin resistance may

include, but are not limited to, metabolic disorders including Type 1 Diabetes, Type 2 Diabetes, Impaired Glucose Tolerance (IGT), Impaired Fasting Glucose (IFG), Hyperglycemia, Postprandial Hyperglycemia, Polycystic Ovary Syndrome (PCOS), Hyperlipidemia, Hypertension, Overweight, Obesity, and Metabolic Syndrome; glycemic control disorders including reduction of Fasting Plasma Glucose (FPG), Postprandial Plasma Glucose (PPG) and/or HbAlc, and improvement of glycemic control; diabetes progression-related disorders including prevention, slowing, delay, or reversal of progression from Impaired Glucose Tolerance (IGT), Impaired Fasting Glucose (IFG), Insulin Resistance, or Metabolic Syndrome to Type 2 Diabetes; diabetic complications including Cataracts, Microvascular and Macrovascular Diseases, Nephropathy, Retinopathy, Neuropathy, Tissue Ischemia, Diabetic Foot, Atherosclerosis, Myocardial Infarction, Acute Coronary Syndrome, Unstable Angina, Stable Angina, Stroke, Peripheral Artery Occlusive Disease, Cardiomyopathy, Heart Failure, Cardiac Arrhythmia, and Vascular Restenosis; weight management disorders including Weight Loss, Prevention of Weight Gain, or Promotion of Weight Loss; beta cell-related disorders including prevention, slowing, delay, or treatment of Beta Cell Degeneration and/or Beta Cell Dysfunction, improvement and/or recovery of beta cell function, and/or restoration of pancreatic insulin secretion function; liver diseases including prevention, slowing, delay, or treatment of diseases or conditions caused by Abnormal Accumulation of Liver Fat; and insulin-related disorders including maintenance and/or improvement of Insulin Sensitivity, and prevention or treatment of Hyperinsulinemia and/or Insulin Resistance.

In another embodiment,
the present invention provides a feed composition for preventing or improving obesity or related metabolic diseases, comprising 1,1-diethoxyethane (1,1-DEE) as an active ingredient.

In the feed composition according to the present invention, the obesity-related metabolic diseases may include, but are not limited to, Type 2 Diabetes, Fatty Liver, Hyperlipidemia, Hypertension, Insulin Resistance, Atherosclerosis, Stroke, Polycystic Ovary Syndrome (PCOS), Metabolic Syndrome, Inflammatory Bowel Disease (IBD), and Sleep Apnea.

The feed comprises nutrients required by animals, such as energy, protein, lipids, vitamins, and minerals, and may be plant-based feed including grains, root crops, food processing by-products, algae, fibers, oils, starches, seed meals, and grain by-products, or animal-based feed including proteins, minerals, oils, mineral substances, and single-cell proteins, but is not limited thereto.

The feed may be in the form of powdered feed, solid feed, moist pellet feed, dry pellet feed, extruder pellet (EP) feed, or raw feed, but is not limited thereto.

The feed composition may further comprise binders, emulsifiers, preservatives, or the like, which are added to prevent quality deterioration, and may further comprise feed additives. To enhance efficacy, the feed may additionally comprise amino acids, vitamins, enzymes, flavoring agents, non-protein nitrogen compounds, silicates, buffers, extracts, oligosaccharides, and the like.

The feed composition may further comprise feed premixes or other additives, but is not limited thereto.

## 5. Therapeutic Methods

In one embodiment,
the present invention provides a method for preventing or treating diseases requiring AMPK activation, comprising administering to a subject an AMPK activator according to item 1 or a pharmaceutical composition according to item 2(1) comprising the AMPK activator as an active ingredient.

In another embodiment,
the present invention provides a method for preventing or treating diseases associated with insulin resistance, comprising administering to a subject a composition for increasing insulin sensitivity or improving insulin resistance according to item 2 or a pharmaceutical composition according to item 2(1) comprising 1,1-diethoxyethane (1,1-DEE) as an active ingredient.

In another embodiment,
the present invention provides a method for preventing or treating obesity or related metabolic diseases, comprising administering to a subject a composition for preventing, improving, or treating obesity or related metabolic diseases according to item 3 or a pharmaceutical composition according to item 2(1) comprising 1,1-diethoxyethane (1,1-DEE) as an active ingredient.

In the method for preventing or treating obesity or related metabolic diseases according to the present invention, the obesity-related metabolic diseases may include, but are not limited to, Type 2 Diabetes, Fatty Liver, Hyperlipidemia, Hypertension, Insulin Resistance, Atherosclerosis, Stroke, Polycystic Ovary Syndrome (PCOS), Metabolic Syndrome, Inflammatory Bowel Disease (IBD), and Sleep Apnea.

The subject may be a human, cow, monkey, horse, sheep, pig, chicken, turkey, quail, cat, dog, mouse, rat, rabbit, or guinea pig, but is not limited thereto.

The route, dosage, and frequency of administration of the pharmaceutical composition may be varied according to the condition of the patient and the presence or absence of side effects, and the optimal route, dosage, and frequency of administration can be selected by a person of ordinary skill in the art within an appropriate range. In the present invention,

the preferred dosage of the pharmaceutical composition may be in the range of 0.001 mg/kg to 100 mg/kg per day for an adult, depending on the condition, body weight, sex, age, severity of the patient, and route of administration. The administration may be performed once per day or divided into multiple administrations. Such dosage ranges should not be construed as limiting the scope of the present invention in any respect.

**6. Medical Uses**

The present invention relates, *inter alia,* to the provision of a composition comprising an AMPK activator which comprises/is 1,1-diethoxyethane (1,1-DEE). Accordingly, the present invention relates to the provision of 1,1-DEE as a means to activate AMPK signaling, specifically AMPK signaling in a cell expression AMPK.

According to the present invention and as shown in the appended non-limiting Examples, 1,1-DEE induces AMPK signaling, in particular by:

i) phosphorylation of the Ser172 residue of AMPK to promote fatty acid oxidation or inhibit fatty acid synthesis, thereby regulating glucose catabolism or suppresses gluconeogenesis; and/or

ii) inhibition of oxidative phosphorylation (OXPHOS) and activation of AMPK via reactive oxygen species (ROS) generation.

Further, 1,1-DEE as an AMPK activator can

i) promote ATP biosynthesis; and/or

ii) increase PGC-1$\alpha$ (peroxisome proliferator-activated receptor gamma coactivator-1 alpha) expression to promote mitochondrial biogenesis,

as also shown in the appended non-limiting examples.

The AMPK activator, i.e. 1,1-DEE, according to the present invention is of particular use for medical interventions directed at treatment of diseases that may be treated, ameliorated and/or prevented by AMPK activation.

Accordingly, the present invention relates in particular to the following pharmaceutical / medical use items, whereby an AMPK activator (i.e. 1,1-DEE) or a composition comprising an AMPK activator is to be used:

1. A pharmaceutical composition comprising 1,1-diethoxyethane (1,1,-DEE)for use in the treatment of a disease that requires AMPK activation.
As shown in the appended, non-limiting examples and as disclosed hereinabove, 1,1-DEE induces AMPK activation, in particular AMPK activation in a cell. Accordingly, it has been demonstrated that 1,1-DEE is of particular use in diseases that require AMPK activation, such as the diseases disclosed herein.

2. A pharmaceutical composition comprising 1,1-diethoxyethane (1,1,-DEE) for use in the treatment of diseases selected from the group consisting of a metabolic disorder, cardiovascular disease, neurodegenerative disease, inflammatory disease, leukemia and cancer,
particularly a metabolic disorder or a cardiovascular disease.

3. The pharmaceutical composition for use according to item 1, wherein the disease is selected from the group consisting of a metabolic disorder, cardiovascular disease, neurodegenerative disease, inflammatory disease, leukemia and cancer,
particularly a metabolic disorder or a cardiovascular disease.

4. The pharmaceutical composition for use according to item 2 or 3, wherein the metabolic disease is selected from the group consisting of Type 1 Diabetes, Type 2 Diabetes, impaired glucose tolerance (IGT), impaired fasting glucose (IFG), hyperglycemia, postprandial hyperglycemia, polycystic ovary syndrome (PCOS), hyperlipidemia, fasting plasma glucose (FPG), postprandial plasma glucose (PPG), HbA1c reduction, progression from IGT, IFG, obesity, nonalcoholic fatty liver disease (NAFLD), nonalcoholic steatohepatitis (NASH), liver fibrosis, dyslipidemia. As shown in the appended Examples, the pharmaceutical composition comprising 1,1,-DEE according to the present invention may increase insulin sensitivity and improve insulin resistance. As shown in the appended examples, the AMPK activator according to the present invention, i.e. 1,1-DEE, and/or the pharmaceutical composition comprising the AMPK activator according to the present invention:

i) induces oxidative inactivation of PTEN via disulfide bond formation between Cys124 and Cys71 residues;

ii) activates AKT signaling by increasing phosphorylation of Akt at Ser473 and Thr308; and/or

iii) promotes glycolysis by increasing phosphorylation of PFKFB2 (6-phosphofructo-2-kinase/fructose-2,6-biphosphatase 2) at Ser483.

The skilled artisan is aware of means and methods to measure/determine features such as oxidative inactivation of PTEN, disulfide bond formation between Cys124 and Cys71 residues of PTEN, activation of AKT signaling, phosphorylation of Akt at Ser473 and Thr308, glycolysis, promotion of glycolysis, phosphorylation of PFKFB2 (6-phosphofructo-2-kinase/fructose-2,6-biphosphatase 2) at Ser483, and increased phosphorylation of PFKFB2 (6-phosphofructo-2-kinase/fructose-2,6-biphosphatase 2) at Ser483. Suitable techniques to assess these parameters are abundantly known in the art. Experimental guidance is, *inter alia* provided in the appended Examples.

Accordingly, the pharmaceutical composition according to the present invention is useful in the treatment of metabolic diseases caused by or related to insulin resistance. The metabolic disease to be treated is, for example, Type 2 diabetes. Accordingly, the pharmaceutical composition according to the present invention may be used in the treatment of Type 2 diabetes.

As shown in the appended non-limiting examples, 1,1-DEE positively influences serum biomarkers which are indicative of liver injury, such as ALT, AST and LDH. Such liver injury is typically associated with diseases such as obesity, nonalcoholic fatty liver disease (NAFLD), nonalcoholic steatohepatitis (NASH) or liver fibrosis.

Accordingly, the pharmaceutical composition according to the present invention comprising the AMPK activator (i.e. 1,1,-DEE) is also for use in the treatment of obesity, nonalcoholic fatty liver disease (NAFLD), nonalcoholic steatohepatitis (NASH) or liver fibrosis, and particularly in the treatment of nonalcoholic fatty liver disease (NAFLD) or nonalcoholic steatohepatitis (NASH).

5. The pharmaceutical composition for use according to item 2 or 3, wherein cardiovascular disease is selected from the group consisting of atherosclerosis, hypertension, heart failure, and ischemic heart disease.

As shown in the appended non-limiting examples, the present inventors have found that treatment with 1,1-DEE improves serum biomarkers which are indicative of cardiovascular diseases such as triglycerides (TG), total cholesterol (T-chol), HDL-C, and LDL-C. Accordingly, the present invention relates to the use of the AMPK activator according to the present invention (i.e. 1,1-DEE) or the pharmaceutical composition comprising the AMPK activator according to the present invention in the treatment of cardiovascular diseases, such as atherosclerosis, hypertension, heart failure, and ischemic heart disease.

6. The pharmaceutical composition for use according to item 2 or 3, wherein the neurodegenerative diseases is Alzheimer's disease or Parkinson's disease.

7. The pharmaceutical composition for use according to item 2 or 3, wherein the inflammatory diseases is inflammatory bowel disease or rheumatoid arthritis.

8. The pharmaceutical composition for use according to any one of items 1 to 7, wherein said composition is to be administered at a dose of 0.01 mg/kg to 100 mg/kg, preferably 0.02 mg/kg to 90 mg/kg, and more preferably 0.03 mg/kg to 80 mg/kg to a subject in need thereof, based on an adult human subject.

The subject in need of treatment according to the present invention may be a human subject in need of treatment, particularly a human patient in need of treatment. It is understood that need of treatment relates to need of treatment with the AMPK activator according to the invention, in particular the pharmaceutical comprising 1,1-DEE according to the present invention.

9. The pharmaceutical composition for use according to any one of items 1 to 8, wherein the composition promotes ATP biosynthesis and/or increase PGC-1$\alpha$ (peroxisome proliferator-activated receptor gamma coactivator-1 alpha) expression to promote mitochondrial biogenesis by:

i. phosphorylation of the Ser172 residue of AMPK to promote fatty acid oxidation or inhibit fatty acid synthesis, thereby regulating glucose catabolism or suppresses gluconeogenesis; and/or

ii. inhibition of oxidative phosphorylation (OXPHOS) and activation of AMPK via reactive oxygen species (ROS) generation.

The pharmaceutical position for use according to item 9, wherein the pharmaceutical composition comprises 1,1-DEE.

The skilled artisan is aware of means and methods to measure/determine features such as phosphorylation of Ser172 residue of AMPK, fatty acid oxidation, fatty acid synthesis, glucose catabolism, gluconeogenesis, oxidative phosphorylation, inhibition of oxidative phosphorylation, activation of AMPK and generation of reactive oxygen species. Such techniques are abundantly known in the art. Experimental guidance is, *inter alia* provided in the appended Examples.

As further items, the present invention also provides for food, feed and cosmetic compositions comprising the AMPK activator (i.e. 1,1-DEE) according to the present invention. Such compositions may be generated by adding the AMPK activators according to the present invention (i.e. 1,1-DEE) to the respective composition. Such compositions are of particular use for improving non-medical conditions associated with a lack of AMPK signaling. For example, compositions

comprising 1,1-DEE may be useful in the context of anti-aging, lifespan extension, or enhancement of physical vitality.

Hereinafter, various examples are provided to facilitate understanding of the invention. The following examples are provided solely to aid in the understanding of the invention and are not intended to limit the scope of the invention to these examples.

## I. AMPK Activator

### <Materials and Methods>

1. **Materials**

1,1-DEE (A902), 2',7'-dichlorofluorescein diacetate (DCFH-DA, #35845), N-acetyl-L-cysteine (A9165), and hydrogen peroxide (H2O2, #88579) were purchased from Sigma-Aldrich (St. Louis, MO, USA). 1,2-DEE was purchased from Tokyo Chemical Industry (Tokyo, Japan). EZ-Cytox assay kit (EZ-3000) was purchased from DoGenBio (Geumcheon, Seoul, Republic of Korea). MitoTracker® Red CMXRos (#9082) and protease/phosphatase inhibitor cocktail (100×, #5872) were purchased from Cell Signaling Technology (CST, Danvers, MA, USA) .

The following antibodies were used: AMPK (#2532, 1:1000), phospho-AMPK (Thr172) (#2535, 1:1000), ACC (#3676, 1:1000), phospho-ACC (Ser79) (#11818, 1:1000), PFKFB2 (#13029, 1:1000), phospho-PFKFB2 (Ser483) (#13064, 1:1000), PGC-1α (#2178, 1:1000), Nrfl (nuclear respiratory factor 1) (#46743, 1:1000), Nrf2 (#12721, 1:1000), and β-actin (#4970, 1:1000) antibodies (CST). In addition, PGC-1α (#PA5-72948, 1:1000) and mitochondrial transcription factor A (Tfam) (#MA5-16148, 1:1000) antibodies were purchased from Invitrogen. GAPDH (LF-PA0212, 1:1000) and anti-rabbit IgG horseradish peroxidase-conjugated (LF-SA8002, 1:5000) antibodies were purchased from AbFrontier (Daejeon, Korea).

2. Cell Culture and Treatment

AC16 cells were maintained in Dulbecco's Modified Eagle Medium/Nutrient Mixture F-12 (DMEM/F12, LM002-05) supplemented with fetal bovine serum (FBS, S001-01) and 1× penicillin/streptomycin (P/S, LS 202-02) (Welgene, Gyeongsan, Korea). Cells were passaged at 70% confluency by trypsinization using 1× trypsin-EDTA (#25300-062). For specific experiments, cells were seeded in 6-well plates and allowed to attach overnight. 1,1-DEE was diluted in serum-free medium prior to incubation with the cells.

Wild-type (WT) and AMPKα double knockout (DKO) mouse embryonic fibroblast (MEF) cell lines were provided by Gachon University College of Medicine and were cultured in DMEM (LM001-05) supplemented with 10% (v/v) FBS and 1× P/S (Welgene).

3. Cell Viability Assay

The cytotoxicity of 1,1-DEE was evaluated using the EZ-Cytox assay according to the manufacturer's protocol. Cells were seeded in 96-well plates (17,000 cells/10 μL/well) and allowed to attach overnight in an incubator (37°C, 5% CO2). The next day, cells were cultured with 1,1-DEE for 24 hours. After 24 hours of incubation, cells were treated with EZ-Cytox reagent (1:10 v/v) and incubated for 30 minutes. The signal was detected at 450 nm using a microplate reader. Cell viability was calculated using the following Equation 1, and the results were analyzed using GraphPad Prism 8.0.1 (San Diego, CA, USA).

[Equation 1]

$$\text{Viability}\quad(\%) = \frac{\text{Exp} - \text{Blank}}{\text{Control} - \text{Blank}} \times 100$$

In Equation 1, "blank" refers to the absorbance of wells containing cell-free medium and EZ-Cytox, "control" refers to the absorbance of wells containing cells and EZ-Cytox but without 1,1-DEE treatment, and "exp" refers to the absorbance of wells containing cells, 1,1-DEE, and EZ-Cytox.

4. valuation of Mitochondrial Oxygen Consumption Rate (OCR) and Extracellular Acidification Rate (ECAR)

Mitochondrial OCR (oxygen consumption rate) and ECAR (extracellular acidification rate) were evaluated using the Agilent Seahorse Mito Stress Test Kit (#103015-100, Seahorse Bioscience, Houston, TX, USA) according to the

manufacturer's protocol. Prior to the assay, AC16 cells were seeded in a 96-well Seahorse cell culture microplate at 1.8 × 10^4 cells/well and allowed to attach overnight. The sensor cartridge was hydrated by placing it in 200 μL Seahorse XF calibrant (#100840-000) and incubated overnight at 37°C in a non-CO2 incubator.

On the day of the assay, cells were washed and replaced with 180 μL/well of Seahorse XF DMEM (#103575-100) according to a modified protocol. OCR and ECAR were measured using the Seahorse XFe96 analyzer (Agilent, California, USA) by sequential injection of 1,1-DEE, oligomycin (1.5 μM), carbonyl cyanide-4-(trifluoromethoxy)phenylhydrazone (FCCP, 1.5 μM), and rotenone/antimycin (0.5 μM) into the injection ports. The results were analyzed using GraphPad Prism 8.0.1.

5. Immunofluorescence Staining and Flow Cytometry Analysis

Cells were seeded in 6-well plates and allowed to attach overnight at 37°C under 5% CO2. The cells were then exposed to 1,1-DEE. For immunofluorescence staining, after specific treatments, cells were washed with PBS and incubated with 10 μM DCFH-DA for 30 minutes at 37°C under 5% CO2 for ROS detection, or with 200 nM MitoTracker® Red CMXRos for mitochondrial labeling. After incubation, cells were washed twice with PBS, and images were captured using a fluorescence microscope.

For flow cytometry analysis, cells were incubated with DCFH-DA or MitoTracker, collected by trypsinization, and fixed with 70% ethanol at 4°C for 30 minutes. The DCFH-DA signal was detected using a FACS Canto II at excitation and emission wavelengths of 485 nm and 535 nm, respectively, and the MitoTracker Red signal was detected at excitation and emission wavelengths of 579 nm and 599 nm, respectively.

6. Western Blot Analysis

Cells were washed twice with Dulbecco's phosphate-buffered saline (DPBS, LM001-01, Welgene) and lysed using a lysis buffer containing 20 mM Tris-HCl (pH 7.5), 150 mM NaCl, 5% glycerol, 0.1% NP-40, and 1× protease/phosphatase inhibitor cocktail. The cell lysates were kept on ice and gently shaken every 5 minutes for 30 minutes. Cell debris was removed by centrifugation at 13,000 rpm for 10 minutes. The supernatant was collected, and protein concentration was measured using a BCA protein assay kit (Pierce, USA).

The lysates were mixed with sample buffer containing 60 mM Tris (pH 6.8), 25% glycerol, 2% SDS, 5% 2-mercaptoethanol, 0.5% bromophenol, and 2-mercaptoethanol, and then heated at 95°C for 5 minutes. Samples were subjected to SDS-PAGE and transferred to Immobilon PVDF membranes. The membranes were blocked at room temperature for 1 hour in TBST containing 3% BSA, followed by overnight incubation at 4°C with primary antibodies. The next day, membranes were washed three times with TBST and incubated with appropriate secondary antibodies for 1 hour. Protein expression was detected using Immobilon chemiluminescent HRP substrate (#P90720, Merck Millipore) and Fusion Solo Vilber Lourmat (Vilber GmbH, Eberhardzell, Germany). Protein quantification was performed using ImageJ software (ImageJ 1.5i, National Institutes of Health, Bethesda, MD, USA).

7. RNA Extraction, Semi-Quantitative PCR, and Quantitative Real-Time PCR (qRT-PCR)

Total RNA was extracted using Trizol reagent (#155960626, Ambion, Carlsbad, California, USA). RNA concentration was measured using a NanoDrop spectrophotometer, and purity was assessed by an A260/A280 ratio of 1.8-2.0. Complementary DNA (cDNA) was synthesized using Improm-II™ reverse transcriptase (A3802, Promega, Madison, USA) according to the manufacturer's protocol. The resulting cDNA was then applied to semi-quantitative PCR using AccuPower® PCR Premix (Bioneer, Daejeon, Korea). In addition, qRT-PCR was performed using TOPreal™ SYBR Green qPCR Premix (#RT500S, Enzynomics, Daejeon, Korea). Primers were purchased from Bioneer and are listed in Table 1.

[Table 1]

| Gene | Forward Primer | Reverse Primer |
|---|---|---|
| *PPARGC1A* | AGCCCCATGGATGAAGGGTACT | CAGCCTTGGGGAGGTCTCAT |
| *Nrf1* | AGTCTCCACGGCGCAGG | TCGGGAGAAGAAGGCGAGTC |
| *Nrf2* | ACATCCAGTCAGAAACCAGTG | CCGGGAATATCAGGAACAAGTG |
| *Tfam* | TGATTCACCGCAGGAAAAGC | GTGCGACGTAGAAGATCCTTTC |
| *GAPDH* | GAAGGTGAAGGTCGGAGTC | GAAGATGGTGATGGGATTTC |

8. Statistical Analysis

Statistical analysis was performed using GraphPad Prism 8.0.1. Data are presented as mean ± standard error of the mean (SEM). Statistical significance was analyzed using two-way analysis of variance (ANOVA), and a p-value $\leq 0.05$ was considered statistically significant.

**<Results>**

1. Evaluation of Whether 1,1-DEE Temporarily Inhibits OCR and ECAR in AC16 Cells

To evaluate the effect of 1,1-DEE (Fig. 1A) on cell viability and to determine the concentration to be used in experiments, an EZ-Cytox assay was performed. AC16 cells were treated with various concentrations of 1,1-DEE for 24 hours, followed by the addition of EZ-Cytox reagent to assess cell viability. The results showed that concentrations up to 25 mM did not induce cytotoxicity (Fig. 1B). Metformin treatment at the same concentrations was also found to be safe; therefore, 25 mM was selected as the maximum treatment concentration for subsequent experiments.

Mitochondrial respiration was evaluated using the Agilent Seahorse XF96 Cell Mito Stress Test Kit. Cells were acutely injected with various concentrations of 1,1-DEE for 30 minutes, and OCR and ECAR were monitored with sequential injections of mitochondrial modulators, including oligomycin, FCCP, and rotenone/antimycin. The results showed that basal OCR sharply decreased immediately after 1,1-DEE injection, but a rapid recovery was observed, indicating a reversible response. The decrease in OCR occurred at 10-20 mM concentrations, but within this range, OCR did not fully recover even after 30 minutes. In contrast, injection of 1,2-DEE did not cause significant changes in OCR, and treatment with metformin at 5-20 mM showed a gradual decrease in OCR after 30 minutes (Fig. 1C). Additionally, mitochondrial ATP-linked respiration, maximal respiration, and spare respiratory capacity were evaluated using oligomycin, FCCP, and rotenone/antimycin injections. Acute injection of 1,1-DEE at 10-20 mM reduced ATP-linked respiration, maximal respiration, and spare respiratory capacity, but proton leak was not significantly affected (Fig. 1D).

ECAR, measured as the rate of lactate efflux converted from pyruvate, reflects cellular glycolytic activity. Similar to basal OCR, acute injection of 1,1-DEE reversibly reduced ECAR (Fig. 1E), whereas treatment with 1,2-DEE or metformin did not result in significant changes in ECAR (Fig. 1E) .

Taken together, these results suggest that 1,1-DEE rapidly modulates mitochondrial and glycolytic functions, leading to a reversible decrease in mitochondrial oxidative phosphorylation (OXPHOS) and cellular glycolysis.

2. Whether 1,1-DEE Activates AMPK

Eukaryotic cells possess a highly conserved homeostatic system to respond to decreased ATP levels when oxidative phosphorylation (OXPHOS) and glycolysis are impaired by mitochondrial toxins. A key component of this system is AMP-activated protein kinase (AMPK), a major regulator of cellular energy. When OXPHOS is inhibited, AMPK is fully activated within minutes to enhance catabolic processes and suppress anabolic processes.

To determine whether 1,1-DEE affects AMPK activation by phosphorylating the Ser172 residue of AMPK through OXPHOS inhibition, AC16 cells were treated with various concentrations of 1,1-DEE for 10 minutes, and the expression of phosphorylated AMPK was analyzed. The results showed that expression of phosphorylated AMPK increased within 10 minutes after 1,1-DEE treatment (Fig. 2A).

Furthermore, to examine whether 1,1-DEE transiently induces AMPK activation, cells were exposed to 1,1-DEE at defined time intervals. Expression of phosphorylated AMPK increased temporarily at 5 and 10 minutes and then decreased, indicating that AMPK can be reversibly activated by acute exposure to 1,1-DEE (Fig. 2B). In contrast, exposure to 1,2-DEE did not induce significant changes in AMPK activation over 30 minutes (Fig. 2C), whereas metformin treatment resulted in a gradual increase in AMPK phosphorylation after 60 minutes (Fig. 2D).

3. Elucidation of the Mechanism by Which 1,1-DEE-Induced AMPK Activation Regulates Fatty Acid Oxidation and Glycolysis

AMPK regulates fatty acid metabolism through the phosphorylation of ACC (acetyl-CoA carboxylase). When AMPK phosphorylates ACC, ACC activity is inhibited, suppressing fatty acid synthesis. Phosphorylation of ACC by AMPK reduces the production of malonyl-CoA, a substrate for fatty acid synthase (FAS). Since malonyl-CoA inhibits mitochondrial fatty acid β-oxidation, this mechanism allows AMPK to promote fatty acid oxidation.

AC16 cells were treated with various concentrations of 1,1-DEE for 30 minutes, and the phosphorylation levels of the AMPK downstream targets ACC and PFKFB2 were analyzed. AMPK activated by 1,1-DEE phosphorylated the Ser79 residue of ACC, inhibiting ACC activity and reducing fatty acid synthesis, thereby promoting mitochondrial fatty acid β-oxidation (Fig. 3A). Additionally, 1,1-DEE treatment decreased the expression of sterol regulatory element-binding

protein-1c (SREBP-1c) and FASN genes, further contributing to ACC inhibition (Fig. 3B).

AMPK is also known to regulate glycolysis by increasing PFKFB2 activity. Following 1,1-DEE exposure, phosphorylation at Ser466 and Ser483 of PFKFB2 increased, indicating enhanced PFKFB2 activation and glycolytic flux (Fig. 3A).

To confirm that ACC and PFKFB2 phosphorylation is AMPK-dependent, wild-type (WT) and AMPK double knockout (DKO) MEF cells were treated with 1,1-DEE for 30 minutes. Similar to the results in AC16 cells, 1,1-DEE treatment in WT MEF cells increased phosphorylation of AMPK and ACC, whereas AMPK DKO MEF cells showed no increase in phosphorylation (Fig. 3C). Moreover, pretreatment with the AMPK inhibitor compound C reduced phosphorylation at Ser483 of PFKFB2, demonstrating that PFKFB2 activation is dependent on 1,1-DEE-induced AMPK activation (Fig. 3D).

4. Determining Whether 1,1-DEE-Induced AMPK Activation Is Mediated by ROS

AMPK activation is primarily triggered by changes in the ADP/ATP and AMP/ATP ratios. However, multiple studies have reported that AMPK can also be activated by reactive oxygen species (ROS), particularly $H_2O_2$. To investigate whether 1,1-DEE-induced AMPK activation is mediated by ROS, the following experiments were conducted.

After 1,1-DEE treatment, intracellular ROS levels were measured using DCFH-DA and live-cell fluorescence staining. Compared with the control group, treatment with 1,1-DEE for 10 minutes increased intracellular ROS levels (Fig. 4A). Pretreatment with the ROS scavenger N-acetylcysteine (NAC) reduced 1,1-DEE-induced ROS production, confirming the involvement of ROS in the process (Fig. 4A).

Subsequently, cells were pretreated with NAC or ebselen, followed by 30 minutes of 1,1-DEE exposure. Western blot analysis revealed that AMPK phosphorylation was decreased in the presence of ROS scavengers, indicating that ROS removal inhibits 1,1-DEE-induced AMPK activation (Fig. 4B). These results suggest that ROS generation is a key mediator of AMPK activation in response to 1,1-DEE.

5. Whether Long-Term 1,1-DEE Treatment Increases PGC-1α Expression Through AMPK Activation

In this study, AMPK activation was assessed upon long-term exposure to 1,1-DEE. AC16 cells were treated with 15 mM 1,1-DEE for 8 hours, and AMPK phosphorylation was increased at 4 and 8 hours, confirming AMPK activation (Fig. 5A).

Among AMPK targets, PGC-1α is an important factor regulating most genes related to mitochondrial metabolism and is considered a key regulator of mitochondrial biogenesis. Treatment of AC16 cells with 1,1-DEE for 8 hours increased the mRNA expression of PPARGC1α, the gene encoding PGC-1α (Fig. 5B), and Western blot analysis confirmed that 1,1-DEE-induced AMPK activation increased PGC-1α expression (Fig. 5C) .

To determine whether the increase in PGC-1α expression is regulated by AMPK activation, wild-type (WT) and AMPK double knockout (DKO) MEF cells were treated with 15 mM 1,1-DEE for up to 12 hours. In WT MEF cells, AMPK phosphorylation induced by 1,1-DEE was associated with increased PGC-1α expression from 2 hours onward, whereas no difference in PGC-1α expression was observed in AMPK DKO MEF cells (Fig. 5D).

6. Mechanism by Which AMPK-Induced PGC-1α Regulates Mitochondrial Biogenesis

PGC1-α is a key transcriptional factor that regulates mitochondrial biogenesis, increasing mitochondrial mass through the transcriptional machinery. PGC-1α binds to Nrfl and Nrf2 to enhance the transcription levels of mitochondria-related genes associated with the electron transport chain (ETC) and simultaneously promotes the expression of Tfam, which is responsible for mitochondrial transcription and genome replication. Through this, the formation of new mitochondria becomes possible.

AC16 cells were treated with 15 mM of 1,1-DEE for up to 8 hours to evaluate whether PGC-1α induces mitochondrial biogenesis through the Nrf1/Nrf2-Tfam axis. Semiquantitative PCR and qRT-PCR results showed that mRNA expression of Nrfl and Nrf2 increased after 8 hours of treatment, whereas Tfam expression significantly increased from 4 hours (Fig. 6A). Western blot analysis also showed a significant increase in Nrf2 and Tfam protein expression, but not in Nrfl (Fig. 6B).

To confirm whether AMPK regulates the expression increase of the Nrf1/Nrf2-Tfam axis, wild-type (WT) and AMPK double knockout (DKO) MEF cells were treated with 15 mM of 1,1-DEE for up to 8 hours. As a result, in WT MEF cells, protein expression of Nrfl and Nrf2 increased, whereas no difference was observed in AMPK DKO cells (Fig. 6C).

When AC16 cells were pretreated with the AMPK inhibitor compound C, Tfam protein expression was suppressed during 1,1-DEE exposure, confirming that mitochondrial biogenesis through the Nrf1/Nrf2/Tfam axis is dependent on 1,1-DEE-induced AMPK activation (Fig. 6D).

Flow cytometry analysis using MitoTracker Red showed that exposing AC16 cells to 1,1-DEE increased the

fluorescent signal, indicating mitochondrial increase, whereas pretreatment with compound C reduced the signal (Fig. 6F). Confocal microscopy observation of mitochondrial morphology revealed fragmented mitochondrial structures (green arrows) upon 1,1-DEE treatment, which were reduced when pretreated with compound C. No such changes were observed with 1,2-DEE treatment (Fig. 6E).

## II. Insulin Resistance

### < Materials and Methods>

1. Materials

The four ethanol batches used in this study are shown in Table 2. 1,1-DEE (A902), N-ethylmaleimide (NEM, E3876), N-acetyl-L-cysteine (NAC, A9165), 2',7'-dichlorofluorescein diacetate (DCFH-DA; 35845), and H2O2 solution (88579) were purchased from Sigma-Aldrich (St. Louis, MO, USA). 1,2-DEE (D0456) was purchased from Tokyo Chemical Industry (Tokyo, Japan).

[Table 2]

| Batch | Name | Chemical Abstracts Service (CAS) # | Catalog # | Source |
|---|---|---|---|---|
| 1 | E1 | 64-17-5 | 1.00983.2511 | Merck |
| 2 | E2 | 64-17-5 | 1.00983.1011 | Merck |
| 3 | E3 | 64-17-5 | 1003304809 | Sigma-Aldrich |
| 4 | E4 | 64-17-5 | 1.00983.2511 | Merck |

2. Cell Culture, 1,1-DEE Treatment, and Transient Transfection of HA-Tagged pCGN Vector

C2C12, MEF, and Ea.hy926 cells were cultured in DMEM (Dulbecco-Modified Eagle's Medium, LM001-05) supplemented with 10% (v/v) fetal bovine serum (FBS, S001-01) and 1% (v/v) penicillin/streptomycin (P/S, LS202-02) purchased from Welgene (Gyeongsan, Republic of Korea). AC16 cells were cultured in DMEM/Nutrient Mixture F-12 (DMEM/F12, LM002-05) supplemented with 12.5% (v/v) FBS and 1% (v/v) P/S purchased from Welgene. When cells reached 70% confluence, they were seeded into 6-well plates and allowed to attach overnight. 1,1-DEE was diluted to a predetermined concentration in serum-free DMEM prior to cell treatment.

For transient transfection, cells were seeded in 6-well plates and allowed to attach overnight. Thereafter, cells were transfected with HA-tagged pCGN PTEN vectors containing WT, C71S, C124S, or C71S/C124S using Lipofectamine 2000 (#11608-027, Invitrogen, Waltham, MA, USA) according to the manufacturer's protocol. Briefly, DNA and Lipofectamine 2000 were separately mixed with Opti-MEM (Ref: 31985-070, Gibco Life Technologies, Grand Island, NY, USA) and incubated at room temperature (RT) for 5 minutes. After combining the mixtures, they were incubated at RT for an additional 20 minutes. The cells were then treated with the mixture and incubated at 37°C under 5% CO2 for 6 hours. Subsequently, the medium was replaced with serum-containing DMEM, and cells were further cultured. One day after transfection, cells were treated with predetermined concentrations of 1,1-DEE and used for various analyses.

3. Gas Chromatography-Mass Spectrometry (GC-MS)

*Gas Sampling*

In this study, the researchers used a continuous irrigation system for transurethral resection of the prostate (TURP) and vaporization. Gas generated during the cutting and cauterization processes accumulated at the top of the bladder. The mixture of the perfused solution and gas was drawn through tubing into a large container connected to a vacuum pump. A portion of the gas collected above the fluid in the container was directed through tubing connected to a Tenax adsorption tube (Tenax GR; Japan Analytical Industry, Tokyo, Japan) at the gas exhaust outlet. The flow rate of gas directed to the Tenax adsorption tube was maintained at 0.05 L/min using a gas flow pump (MP-S30; SIBATA, Tokyo, Japan). Measurements using a Tedlar Bag (Sigma-Aldrich) showed that approximately 45 L per hour of gas mixed with room air was generated. For quantification, 1 L of surgical gas was injected into the Tenax adsorption tube at a flow rate of 0.05 L/min.

*Gas Analysis*

The gas was subjected to purge and trap sampling using an Automated Purge & Trap Sampler JTD-505III (Japan Analytical Industry). Quantitative and qualitative analyses were performed using GC/MS QP 2010 Plus (Shimadzu, Kyoto, Japan) .

*Purge and Trap Conditions*

The purge and trap conditions were as follows: Desorption temperature: 280°C, desorption time: 30 min, desorption gas flow rate: 50 mL/min, cold trap temperature: -40°C, pyrolysis temperature: 280°C, transfer line temperature: 280°C, needle heater temperature: 280°C, cold trap heater temperature: 200°C, head press: 86 MPa, column flow rate: 1.0 mL/min, and split ratio: 1/100.

*GC/MS Conditions*

The analysis conditions were as follows: Column: DB-624 column (30 m × 0.251 mm × 1.40 μm; Agilent Technologies, Wilmington, DE, USA), scan range: 30-600 mass, oven temperature program: 40°C (hold 3 min), ramp at 10°C/min to 260°C, hold 5 min, ion source temperature: 200°C, transfer line temperature: 250°C, electron energy (EM voltage): 70 eV.

4. Freeze-Drying

Ethanol samples were stored in 15 mL Falcon tubes and frozen at -80°C for 5 hours. Subsequently, small holes were punctured in the caps of all Falcon tubes. The frozen samples were freeze-dried overnight to completely remove volatile and moisture-containing components. The next day, the contents of each Falcon tube were reconstituted in serum-free DMEM prior to cell treatment.

5. DCFH-DA Staining for ROS Detection

The total intracellular reactive oxygen species (ROS) levels were assessed as previously described [Kim, H. and X. Xue, Detection of Total Reactive Oxygen Species in Adherent Cells by 2',7'-Dichlorodihydrofluorescein Diacetate Staining. J Vis Exp, 2020(160)]. Briefly, cells were seeded in 6-well plates and cultured overnight at 37°C in 5% CO2. After treatment, the cells were washed once with serum-free DMEM and then incubated with 10 μM DCFH-DA at 37°C for 30 minutes. Subsequently, the cells were washed once with serum-free DMEM and twice with PBS. Finally, the cells were promptly imaged using a fluorescence microscope.

6. Flow Cytometry Analysis

Intracellular reactive oxygen species (ROS) and mitochondrial peroxide levels were determined using flow cytometry (FACS CANTO II, BD Biosciences, NJ, USA). After 1,1-DEE treatment, cells were incubated with predetermined concentrations of DCFH-DA at 37°C for 30 minutes. Subsequently, cells were collected using Trypsin-EDTA (#25300-062, Gibco) and washed with cold PBS. Intracellular ROS levels were immediately quantified by excitation at 485 nm and emission at 530 nm, while mitochondrial peroxide levels were quantified by excitation at 510 nm and emission at 580 nm.

7. Western Blot Analysis and Antibodies

The redox status of PTEN was analyzed as previously described. Briefly, cells were lysed in a lysis buffer containing 20 mM Tris-HCl (pH 7.5), 150 mM NaCl, 5% glycerol, 0.1% NP-40, phosphatase inhibitors, protease inhibitors, and 10 mM NEM. The lysates were sonicated and centrifuged at 13,000 rpm for 10 minutes. The supernatant was collected, and protein concentration was determined using the Pierce™ BCA Protein Assay Kit (Thermo Fisher Scientific, Waltham, MA, USA). Lysates were mixed with either reducing sample buffer containing 60 mM Tris (pH 6.8), 25% glycerol, 2% SDS, 5% 2-mercaptoethanol, and 0.5% bromophenol blue, or non-reducing sample buffer without 2-mercaptoethanol. The samples were subjected to SDS-PAGE, and immunoblotting was performed using PTEN-specific antibodies.

To examine other protein expressions, lysates were mixed with the described reducing sample buffer, subjected to SDS-PAGE, and immunoblotted with various specific antibodies. The antibodies used in this study included: Akt (#9272S, 1:1000), phospho-Akt473 (#9271S, 1:1000), phospho-Akt308 (#9275S, 1:1000), phospho-PFKFB2 (Ser483) (#13064, 1:1000), PFKFB2 (#13029, 1:1000), and β-Actin (#4970, 1:1000), purchased from Cell Signaling Technology

(Danvers, MA, USA). GAPDH antibody (LF-PA0212, 1:1000) and anti-rabbit IgG horseradish peroxidase-conjugated antibody (LF-SA8002, 1:5000) were purchased from Ab Frontier (Daejeon, Republic of Korea).

8. Statistical Analysis

Western blot protein bands were quantified using densitometry with ImageJ 1.50i (National Institutes of Health, Bethesda, MD, USA). All values are presented as the mean ± standard error of the mean (SEM) from three independent experiments. Statistical significance was analyzed using two-way ANOVA for multiple group comparisons in GraphPad Prism, version 6 (GraphPad, San Diego, CA, USA). A p-value of $<0.05$ was considered statistically significant.

**<Results>**

1. Evaluation of the Effect of 1,1-DEE Ethanol Batches on PTEN Oxidation

In this study, the effects of various ethanol batches provided by commercial sources on the redox status of PTEN were evaluated. HepG2 cells were used for the experiments, and the influence of different ethanol batches at various concentrations on PTEN oxidation rates was examined. The results showed that, even at the same concentration, PTEN oxidation rates varied significantly depending on the ethanol batch. In particular, batch E1 induced PTEN oxidation more strongly than batches E2 and E3. Additionally, when E1 was mixed with E2 or E3, the level of PTEN oxidation tended to decrease compared to treatment with E1 alone (Fig. 7A).

Furthermore, the odor characteristics of batch E1 were clearly distinct from those of other ethanol batches. E1 exhibited a strong alcoholic and fruity aroma, whereas this odor was relatively less pronounced in batches E2 and E3. This suggests that specific compounds present in batch E1 may influence the redox status of PTEN.

To identify the compound characteristics of batches E1, E2, and E4, the volatile compounds in each batch were analyzed. Batch E4 displayed odor characteristics similar to those of E1 (Fig. 7B). To confirm this, 100 mM ethanol was freeze-dried to volatilize the volatile compounds, and the dried samples were reconstituted in PBS or 100 mM E2. HepG2 cells were then treated for 10 minutes with ethanol from each batch (100 mM, E1, E2, E4) or the corresponding reconstituted dried samples. The results showed that E1 and E4 batches strongly induced PTEN oxidation compared to E2 and the control. However, the dried samples of E1, E2, and E4 did not show similar effects under reconstitution conditions (PBS or E2) (Fig. 7C), indicating that volatile compounds were lost during the freeze-drying process.

Subsequently, GC-MS analysis was performed to identify additional compounds in batches E1, E2, E3, and E4. The analysis revealed that the fragrance compound 1,1-diethoxyethane (1,1-DEE) was present at high concentrations in E1 and E4 but was not detected in E2 and E3. The GC-MS-based fragmentation analysis and confirmed chemical structure of 1,1-DEE in batches E1 and E4 are shown in Fig. 7D. These results demonstrate that 1,1-DEE can play a novel role in mediating PTEN redox regulation in cellular functions.

2. Evaluation of Whether 1,1-DEE Induces PTEN Oxidation in a Concentration-Dependent Manner

To determine whether 1,1-DEE can exhibit a novel molecular function by regulating the redox status of PTEN, cells were treated with various concentrations of 1,1-DEE, and PTEN oxidation levels were assessed. 1,1-DEE-mediated PTEN oxidation was observed within 10 minutes post-treatment in various cell lines, including C2C12, MEF, AC16, and Ea.hy926. In C2C12 and MEF cells, PTEN oxidation was induced by 1 mM 1,1-DEE, whereas similar levels of oxidation were achieved in AC16 and Ea.hy926 cells at 5 mM 1,1-DEE (Fig. 8A). 1,1-DEE-mediated oxidation occurred in a concentration-dependent manner, with higher concentrations leading to increased oxidation levels. In contrast, the 1,2-DEE isomer of 1,1-DEE failed to induce PTEN oxidation in C2C12 cells at various concentrations, suggesting a specific structural requirement for 1,1-DEE in regulating PTEN redox status (Fig. 8B) .

To investigate the underlying mechanism of 1,1-DEE-mediated PTEN oxidation, C2C12 cells were transiently transfected with HA-tagged pCGN vectors encoding either wild-type (WT) PTEN or PTEN mutants (C71S, C124S, or C71/124S) prior to 1,1-DEE treatment. PTEN oxidation was detected in cells transfected with WT PTEN, but not in cells transfected with PTEN C71S, PTEN C124S, or PTEN C71/124S (Fig. 8C). These results indicate that 1,1-DEE mediates oxidative inactivation of PTEN by promoting disulfide bond formation between Cys124 and Cys71 residues.

3. Evaluation of Whether 1,1-DEE Induces PTEN Oxidation in a Time-Dependent Manner

This study investigated the time-dependent regulatory pattern of 1,1-DEE on the redox status of PTEN. Various cell types, including C2C12, MEF, AC16, and Ea.hy926, were treated with 1,1-DEE for up to 120 minutes. Interestingly, PTEN oxidation levels began to increase just 5 minutes after the start of 1,1-DEE treatment, reached a peak at 10 minutes, and gradually returned to basal levels by 120 minutes (Fig. 9A). In contrast, 1,2-DEE failed to induce PTEN oxidation in

C2C12 cells even after 120 minutes of treatment (Fig. 9B). These results demonstrate that the specific form of 1,1-DEE can induce reversible PTEN oxidation in a time-dependent manner.

4. Evaluation of Whether 1,1-DEE-Induced PTEN Oxidation Regulates Subsequent Akt Activation

This study examined Akt activation mediated by 1,1-DEE-induced PTEN oxidation. C2C12 cells were treated with various concentrations of 1,1-DEE for 10 minutes or with 10 mM 1,1-DEE for different durations up to 120 minutes. Phosphorylation of Akt at Ser473 and Thr308 was enhanced after 10-minute treatment with 1 mM 1,1-DEE, indicating increased Akt activity. Akt phosphorylation increased in a concentration-dependent manner in response to 1,1-DEE, reflecting the negative regulation of the Akt signaling pathway by PTEN (Fig. 10A). Similar to the reversible oxidation of PTEN mediated by 1,1-DEE, phosphorylation at Ser473 and Thr308 was also reversible. Levels of phosphorylated Akt473 and Akt308 increased 5 minutes after 1,1-DEE treatment, peaked at 10 minutes, and gradually returned to basal levels by 120 minutes (Fig. 10B). These results suggest that 1,1-DEE-induced oxidative inhibition of PTEN can lead to reversible activation of Akt.

5. Evaluation of Whether 1,1-DEE-Induced PTEN Oxidation Is Mediated by ROS Generation

To investigate the underlying mechanism of 1,1-DEE-induced PTEN oxidation, ROS levels were assessed in cells after 1,1-DEE treatment. DCFH-DA staining showed that cytosolic ROS levels increased 10 minutes after 1,1-DEE treatment compared with the control (Fig. 11A). Flow cytometry analysis revealed that ROS levels began to rise 5 minutes after treatment, peaked at 10 minutes, and returned to basal levels by 30 minutes, indicating that 1,1-DEE can reversibly increase ROS generation (Fig. 11B).

Next, to examine the relationship between ROS generation and 1,1-DEE-induced PTEN oxidation, cells were pretreated with the ROS scavenger NAC for 120 minutes prior to 1,1-DEE treatment. NAC pretreatment reduced 1,1-DEE-induced PTEN oxidation, indicating that ROS generated by 1,1-DEE mediates the oxidative inactivation of PTEN. Moreover, NAC pretreatment decreased phosphorylation levels of Akt at Ser473 and Thr308 (Fig. 11C). These results suggest a regulatory mechanism of 1,1-DEE-induced ROS generation in the PTEN/Akt signaling pathway.

6. Evaluation of Whether 1,1-DEE Promotes Glycolysis via Akt Activation

Akt activation is known to regulate the rate of glycolysis through phosphorylation of PFKFB2 (6-phosphofructo-2-kinase/fructose-2,6-biphosphatase 2) at the Ser483 residue. PFKFB2 is a bifunctional enzyme involved in both the synthesis and degradation of Fru-2,6-P2 (fructose-2,6-bisphosphate), which controls glycolysis in eukaryotes. Phosphorylation of PFKFB2 enhances its activity, leading to increased Fru-2,6-P2 production and further upregulation of glycolysis. To examine the effect of Akt activation on glycolysis, phosphorylation of PFKFB2 at Ser483 was assessed in AC16 cells. The results showed that 1,1-DEE treatment increased PTEN oxidation and Akt activation via phosphorylation at Ser473, and exposure to 1,1-DEE for 5 minutes induced phosphorylation of PFKFB2 at Ser483 (Fig. 12A).

To confirm whether Ser483 phosphorylation of PFKFB2 is regulated by Akt activation, AC16 cells were pretreated with the Akt inhibitor MK-2206 prior to 1,1-DEE treatment. MK-2206 pretreatment reduced both Akt activation and PFKFB2 phosphorylation, indicating that glycolysis can be regulated by 1,1-DEE-induced Akt activation (Fig. 12A).

Next, to examine the relationship between 1,1-DEE-induced ROS generation and glycolysis, cells were pretreated with Ebselen. Interestingly, Ebselen pretreatment attenuated the 1,1-DEE-induced increase in PFKFB2 phosphorylation (Fig. 12B). These results suggest that 1,1-DEE-induced ROS generation mediates PTEN oxidative inactivation and Akt activation, thereby further enhancing glycolysis via phosphorylation of PFKFB2.

7. Evaluation of Whether 1,1-DEE Enhances Insulin Sensitivity and Ameliorates Palmitate-Induced Insulin Resistance

In this study, the effect of 1,1-DEE on insulin signaling and insulin sensitivity was investigated in C2C12 cells. Treatment with 1,1-DEE induced PTEN oxidation and Akt activation, as assessed by levels of phosphorylated Akt at Ser473 and Thr308. Co-treatment of cells with 1,1-DEE and insulin resulted in higher phosphorylation of Akt at Ser473 and Thr308 compared to treatment with either 1,1-DEE or insulin alone (Fig. 13A), indicating that 1,1-DEE can enhance insulin signaling and insulin sensitivity.

To evaluate the effect of 1,1-DEE on insulin resistance, an in vitro cell model of insulin resistance was established using palmitate. These cells were treated with 1,1-DEE or insulin for 10 minutes. In insulin-resistant cells, Akt phosphorylation at Ser473 and Thr308 residues was markedly reduced compared to control cells (Fig. 13B), confirming successful establishment of the palmitate-induced insulin resistance model. Importantly, co-treatment with 1,1-DEE and insulin restored Akt phosphorylation in insulin-resistant cells (Fig. 13B), suggesting that 1,1-DEE can alleviate palmitate-induced insulin resistance via Akt activation.

**III. Obesity**

< **Materials and Methods>**

1. Animal Model and Oral Administration of 1,1-DEE

Eight-week-old male C57BL/6J mice were purchased from Damool Science (Daejeon, Korea). The mice were divided into four groups (n = 5) and fed different diets, including a normal diet (ND) group, an ND group with 1,1-DEE administration, a high-fat diet (HFD) group, and an HFD group with 1,1-DEE administration (Fig. 1). The ND consisted of 10 kcal% fat, 70 kcal% carbohydrate, and 20 kcal% protein, whereas the HFD consisted of 60 kcal% fat, 20 kcal% carbohydrate, and 20 kcal% protein. Mice were orally administered 1,1-DEE at 100 mg/kg every other day, while the control group received the same volume of water.

After 8 weeks of treatment, mice were subjected to glucose tolerance and insulin tolerance tests. After 10 weeks of treatment, mice were sacrificed, and blood samples were collected and stored at room temperature (RT) for 30 minutes. Liver, white adipose tissue (WAT), colon, and heart tissues were collected, rapidly frozen in liquid nitrogen, and stored at -80°C for further analysis.

2. Serum Biochemical Analysis

After allowing blood to clot at room temperature (RT), the blood samples were centrifuged at 3,000 rpm for 20 minutes at 4°C, and the serum was transferred to Eppendorf tubes. Serum biochemical analysis was performed using an automated blood chemistry analyzer (Duyeol Biotech Company, Seoul, Republic of Korea), which included markers such as AST, ALT, ALP, lactate dehydrogenase (LDH), total bilirubin (T-Bili), total cholesterol (T-chol), triglycerides (TG), albumin (Alb), LDL-cholesterol (LDL-C), and HDL-cholesterol (HDL-C). Plasma insulin levels were measured using a mouse insulin ELISA kit (#292-89401, FUJIFILM Wako Pure Chemical Corporation, Japan).

3. Intraperitoneal Glucose Tolerance Test (IGTT) and Insulin Tolerance Test (IITT)

For the glucose tolerance test, mice were transferred to a new cage without food and fasted overnight for 12 hours. Fasting blood glucose levels were measured using a glucose meter. Subsequently, mice were administered D-glucose intraperitoneally at a dose of 2 g/kg. Blood glucose levels were measured at 15, 30, 60, 90, and 120 minutes after glucose injection.

For the insulin tolerance test, mice were fasted for 4 hours. Insulin (Humulin R) was administered intraperitoneally at a dose of 0.6 U/kg. Blood glucose levels were recorded at 15, 30, 60, 90, and 120 minutes after insulin injection using a glucose meter.

4. Histological Analysis

Liver, adipose tissue, colon, and heart tissues were collected from each mouse and fixed in 4% paraformaldehyde. The tissues were sectioned into 5mm-thick slices and embedded in paraffin. The paraffin sections were stained with hematoxylin and eosin (H&E).

5. Western Blot Analysis

Tissues were lysed using NP-40 lysis buffer (RIPA, 1% NP-40, 10mM NEM). The tissue extracts were kept on ice for 1hour and then centrifuged at 13,000rpm for 30minutes. The supernatant was collected, and protein concentration was determined using a BCA kit. Protein samples were prepared by adding reducing and non-reducing sample buffers. Western blotting was performed to examine the protein expression of phospho-AMPK, AMPK, phospho-ACC, ACC, and β-actin.

6. Statistical Analysis

Statistical analysis was performed using GraphPad Prism. Data are expressed as the mean ± standard error of the mean (SEM). Statistical significance was determined using two-way analysis of variance (ANOVA), and a p-value ≤ 0.05 was considered statistically significant.

**<Results>**

1. Evaluation of the Body Weight-Reducing Effect of 1,1-DEE

(1) Assessment of 1,1-DEE on Body Weight Gain in High-Fat Diet (HFD) Mice

In this study, the effect of 1,1-DEE on body weight gain in a high-fat diet (HFD)-induced obesity model was investigated (Fig. 14). Eight-week-old mice were orally administered HFD supplemented with 100 mg/kg of 1,1-DEE every other day for 8 weeks. Representative images of mice from each group are shown in Fig. 15A.

The results demonstrated that the HFD group showed a significant increase in body weight compared with the normal diet (ND) group, whereas administration of 1,1-DEE significantly suppressed HFD-induced weight gain (Fig. 15B). No significant difference in body weight changes was observed between the ND group and the ND+1,1-DEE group (Fig. 15B). Although 1,1-DEE increased food intake in ND-fed mice, food intake in the HFD and HFD+1,1-DEE groups remained comparable (Fig. 15C), suggesting that 1,1-DEE may stimulate appetite in mice.

Additionally, post-sacrifice tissue analysis revealed that 1,1-DEE reduced the weights of epididymal (eWAT) and inguinal (iWAT) white adipose tissue induced by HFD (Figs. 15D-E). No significant differences were observed in the weight or length of the heart, liver, and colon (Figs. 15F-H).

(2) Evaluation of Body Weight Regulation by Comparison Between 1,1-DEE and Metformin

An additional experiment was conducted to compare the anti-obesity effects of 1,1-DEE and metformin. In the high-fat diet (HFD) mouse model, 1,1-DEE rapidly suppressed body weight gain from the early stages of administration, and this weight-reducing effect was maintained continuously over 4 weeks compared with the HFD group. In contrast, the metformin-treated group showed a slight increase in body weight during the first week, followed by a gradual slowing of weight gain. In normal diet (ND) mice, 1,1-DEE treatment did not significantly affect body weight changes.

Notably, in the HFD+D100 group, mice treated with 1,1-DEE exhibited increased food intake compared with the HFD group, suggesting that 1,1-DEE may exert its weight-controlling effect through an exercise-mimetic action rather than appetite suppression. These results indicate that 1,1-DEE can achieve more rapid suppression of body weight gain compared with metformin (Fig. 16). Therefore, 1,1-DEE may serve as a useful candidate for the prevention and treatment of obesity and diabetes-related metabolic disorders.

2. Evaluation of Glucose Regulation and Improvement of Insulin Resistance by 1,1-DEE

To assess the effect of 1,1-DEE on glucose and insulin metabolism, random blood glucose levels were measured every 2 weeks. The results showed that mice treated with 1,1-DEE had significantly lower blood glucose levels compared with the HFD group (Fig. 17A). After 8 weeks of treatment, fasting blood glucose levels measured following a 12-hour fast were higher in the HFD group than in the ND group, whereas 1,1-DEE administration reduced fasting blood glucose levels (Fig. 17B).

Additionally, the HFD group supplemented with 1,1-DEE exhibited a lower glucose tolerance test (GTT) area under the curve (AUC) compared with the HFD group, indicating improved glucose tolerance (Fig. 17C). These findings suggest that 1,1-DEE positively affects glucose metabolism. After 8 weeks, serum insulin levels were elevated in the HFD group compared with the ND group, whereas 1,1-DEE treatment reduced insulin levels (Fig. 17D).

Insulin tolerance tests (ITT) further demonstrated that the 1,1-DEE-treated group had a lower AUC, indicating enhanced insulin sensitivity ($p=0.2427$, Fig. 17E). Therefore, 1,1-DEE improves glucose metabolism and insulin sensitivity under conditions of hyperglycemia and insulin resistance.

3. Evaluation of Hepatic Injury and Lipid Accumulation Improvement by 1,1-DEE

To assess whether 1,1-DEE ameliorates hepatic injury and lipid accumulation in HFD-fed mice, serum biomarkers were analyzed. Levels of liver injury-related markers, including ALT, AST, and LDH, were elevated in the HFD group but were reduced in mice treated with 1,1-DEE (Fig. 18A-C). No significant differences were observed in serum ALP, total bilirubin (T-Bil), or albumin (Alb) levels (Fig. 18D-F).

Furthermore, serum lipid biomarkers, including triglycerides (TG), total cholesterol (T-chol), HDL-C, and LDL-C, were evaluated. 1,1-DEE treatment decreased TG levels compared with the HFD group (Fig. 18G), and both HDL-C and LDL-C levels induced by HFD were reduced following 1,1-DEE administration (Fig. 18H-K). Notably, 1,1-DEE improved HFD-induced LDL-C levels, suggesting a potential reduction in atherosclerosis risk (Fig. 18K).

These results indicate that 1,1-DEE can ameliorate hepatic injury and lipid accumulation in HFD-induced obese mice.

This invention was supported by a national research and development project:

Project Number: 2018R1D1A1B06051438
Ministry: Ministry of Science and ICT
Project Management (Specialized) Organization: National Research Foundation of Korea
Project Title: Alcohol-Induced Regulation of the Tumor Suppressor Protein PTEN
Project Implementing Organization: Chonnam National University
Research Period: March 1, 2023 - February 29, 2024
Project Number: 2022M3A9E4017151
Ministry: Ministry of Science and ICT
Project Management Organization: National Research Foundation of Korea
Project Title: Development of Heart Failure Control and Treatment Technology Based on MyHeart Platform
Host Organization: Chonnam National University
Participating Company: LuxAnima Co., Ltd.
Research Period: January 2024 - December 2024
Ministry / Funding Organization: Chonnam Technopark
Research Program: Hwasun Vaccine Industrial Complex Intellectual Property Capacity Building Support Project
Project Title: IP-R&D
Host Organization: LuxAnima Co., Ltd.

As described above, specific aspects of the present invention have been described in detail. It will be apparent to those skilled in the art that such specific descriptions are merely preferred embodiments, and the scope of the present invention is not limited thereby. Accordingly, the actual scope of the present invention is defined by the appended claims and their equivalents.

## Claims

1. An AMPK (AMP-activated protein kinase) activator comprising 1,1-diethoxyethane (1,1-DEE) as an active ingredient.

2. The AMPK activator according to Claim 1, wherein the 1,1-DEE induces phosphorylation of the Ser172 residue of AMPK to promote fatty acid oxidation or inhibit fatty acid synthesis, and thereby regulates glucose catabolism or suppresses gluconeogenesis.

3. The AMPK activator according to Claim 1, wherein the 1,1-DEE temporarily inhibits oxidative phosphorylation (OXPHOS) and activates AMPK via reactive oxygen species (ROS) generation.

4. The AMPK activator according to Claim 1, wherein the 1,1-DEE promotes bioenergy ATP biosynthesis.

5. The AMPK activator according to Claim 1, wherein the 1,1-DEE increases PGC-1$\alpha$ (peroxisome proliferator-activated receptor gamma coactivator-1 alpha) expression to promote mitochondrial biogenesis.

6. A pharmaceutical composition for preventing or treating diseases requiring AMPK activation, comprising 1,1-DEE as an active ingredient,
the diseases requiring AMPK activation includes at least one selected from metabolic disorders including Nonalcoholic Fatty Liver Disease (NAFLD), Nonalcoholic Steatohepatitis (NASH), Liver Fibrosis, Dyslipidemia, Atherosclerosis, Hypertension, Ischemic Heart Disease, Inflammatory Bowel Disease and Rheumatoid Arthritis.

7. A cosmetic composition for anti-aging, comprising 1,1-DEE as an active ingredient.

8. A food composition for preventing or ameliorating diseases requiring AMPK activation, anti-aging, lifespan extension, or enhancement of physical vitality, comprising 1,1-DEE as an active ingredient.

9. A feed composition for preventing or ameliorating diseases requiring AMPK activation, anti-aging, lifespan extension, or enhancement of physical vitality, comprising 1,1-DEE as an active ingredient.

10. A composition for increasing insulin sensitivity or improving insulin resistance, comprising 1,1-DEE as an active ingredient.

11. The composition according to Claim 10, wherein the 1,1-DEE induces oxidative inactivation of PTEN via disulfide

bond formation between Cys124 and Cys71 residues.

**12.** The composition according to Claim 10, wherein the 1,1-DEE increases phosphorylation of Akt at Ser473 and Thr308, thereby activating Akt.

**13.** The composition according to Claim 10, wherein the 1,1-DEE increases phosphorylation of PFKFB2 (6-phosphofructo-2-kinase/fructose-2,6-biphosphatase 2) at Ser483, thereby promoting glycolysis.

**14.** A pharmaceutical composition for preventing or treating insulin resistance-related diseases, comprising 1,1-DEE as an active ingredient,
wherein the diseases are selected from Type 1 Diabetes, Type 2 Diabetes, impaired glucose tolerance (IGT), impaired fasting glucose (IFG), hyperglycemia, postprandial hyperglycemia, polycystic ovary syndrome (PCOS), hyperlipidemia, fasting plasma glucose (FPG), postprandial plasma glucose (PPG), HbAlc reduction, progression from IGT, IFG, insulin resistance or metabolic syndrome to Type 2 Diabetes, diabetic complications (including cataracts, microvascular and macrovascular diseases, nephropathy, retinopathy, neuropathy, tissue ischemia, diabetic foot, acute coronary syndrome, peripheral artery occlusive disease, cardiomyopathy, vascular restenosis), weight management disorders, beta-cell degeneration or dysfunction, and insulin-related disorders (including maintenance or improvement of insulin sensitivity, prevention or treatment of hyperinsulinemia or insulin resistance).

**15.** A cosmetic composition for preventing or ameliorating insulin resistance-related diseases, comprising 1,1-DEE as an active ingredient.

**16.** A food composition for preventing or ameliorating insulin resistance-related diseases, comprising 1,1-DEE as an active ingredient.

**17.** A feed composition for preventing or ameliorating insulin resistance-related diseases, comprising 1,1-DEE as an active ingredient.

**18.** A pharmaceutical composition for preventing or treating obesity, comprising 1,1-DEE as an active ingredient.

**19.** A cosmetic composition for preventing or ameliorating obesity, comprising 1,1-DEE as an active ingredient.

**20.** A food composition for preventing or ameliorating obesity, comprising 1,1-DEE as an active ingredient.

**21.** A feed composition for preventing or ameliorating obesity, comprising 1,1-DEE as an active ingredient.

Figure 1A

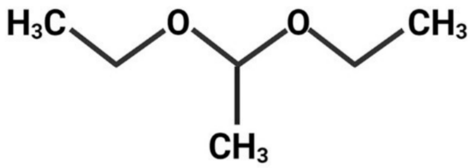

1,1-diethoxyethane

Figure 1B

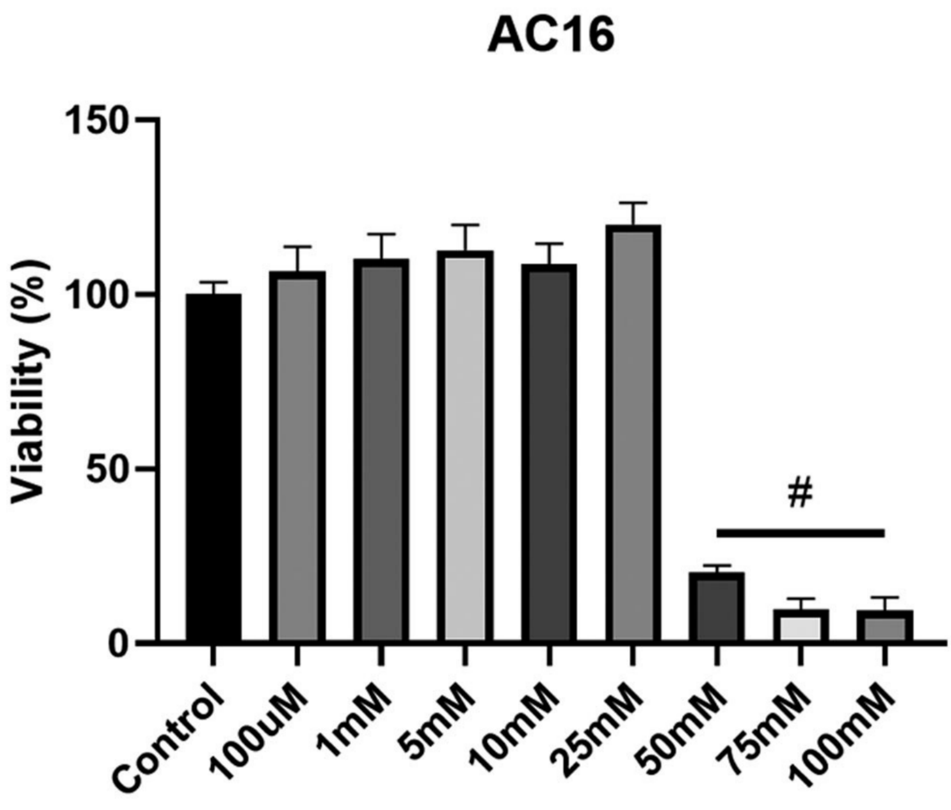

**Figure 1C**

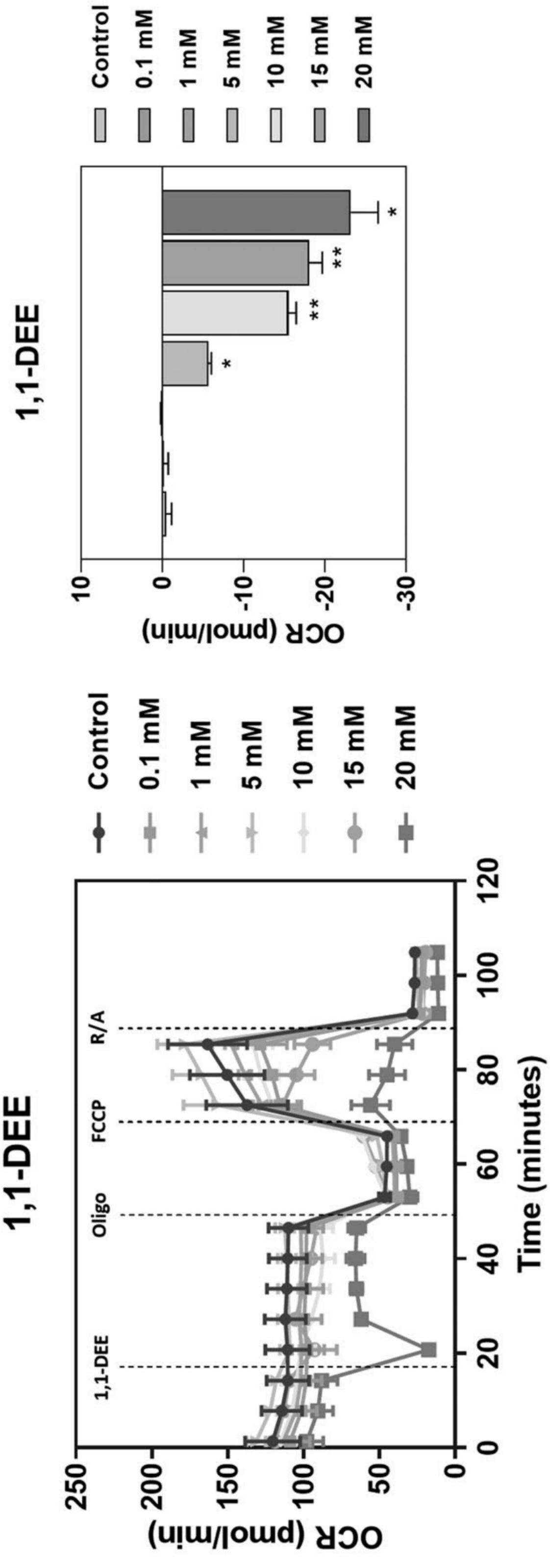
1,1-DEE
OCR (pmol/min)
10
0
-10
-20
-30
Control
0.1 mM
1 mM
5 mM
10 mM
15 mM
20 mM
*
**
**
*
1,1-DEE
OCR (pmol/min)
250
200
150
100
50
0
Time (minutes)
0
20
40
60
80
100
120
1,1-DEE
Oligo
FCCP
R/A
Control
0.1 mM
1 mM
5 mM
10 mM
15 mM
20 mM

**Figure 1C continued**

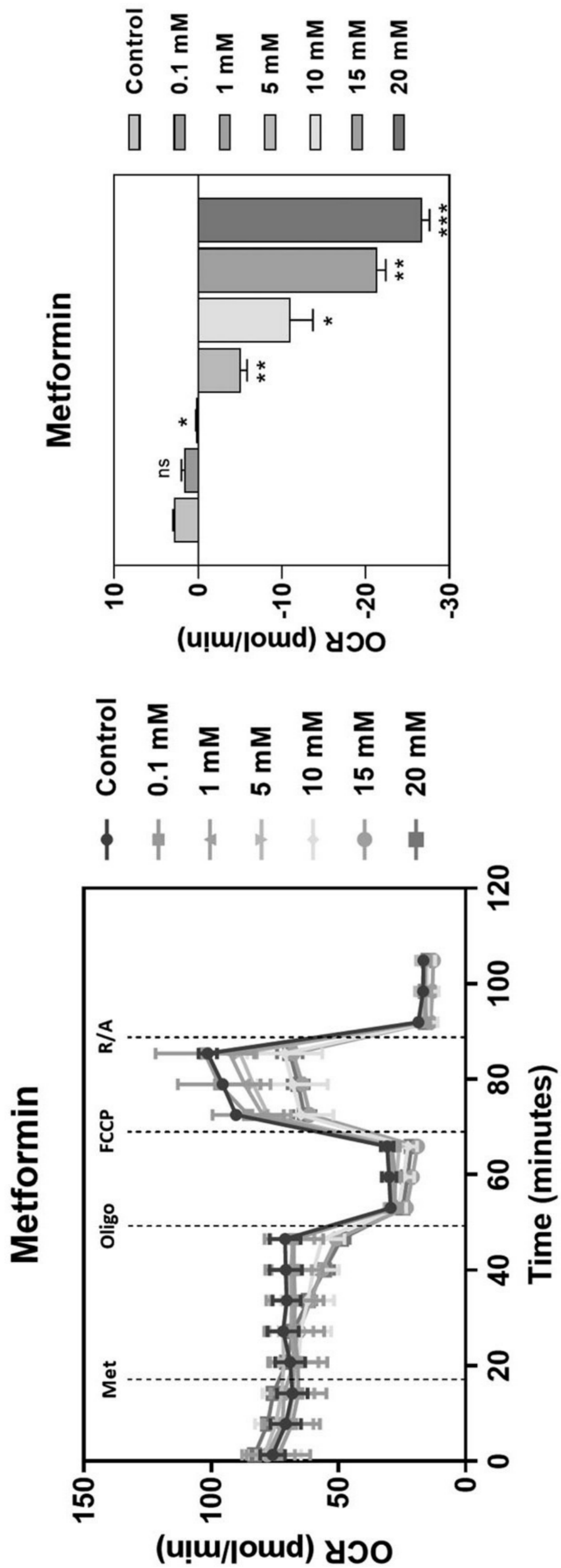

**Figure 1C continued**

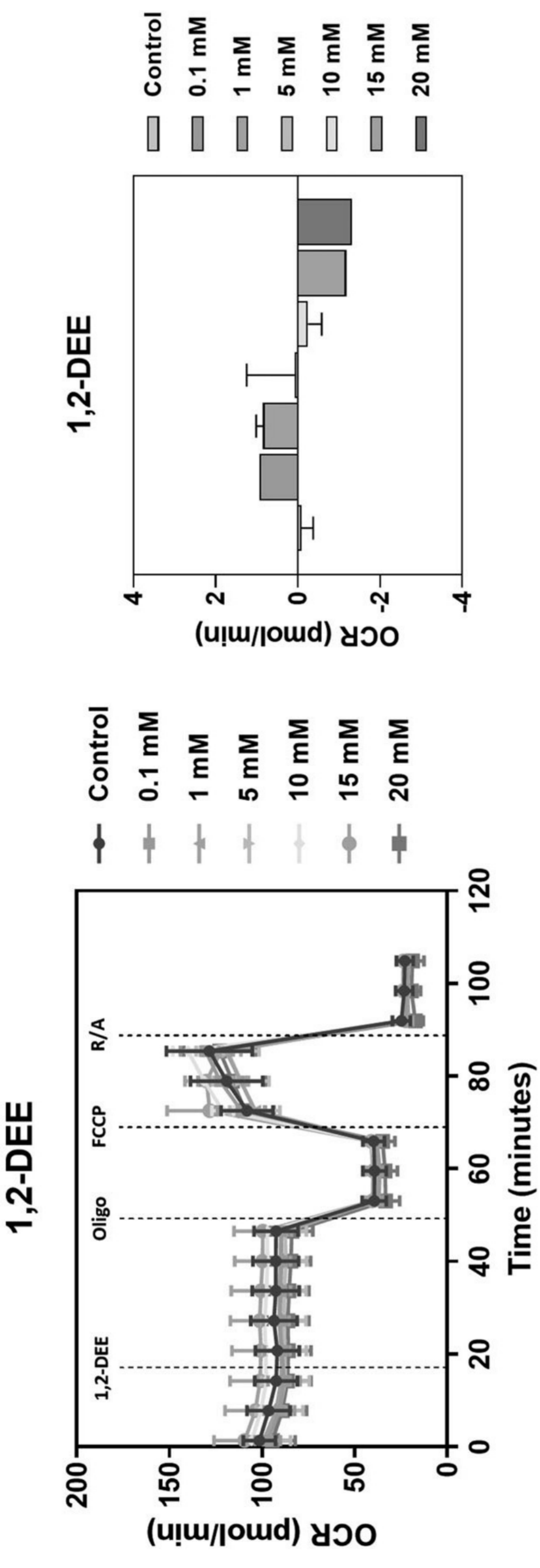

**Figure 1D**

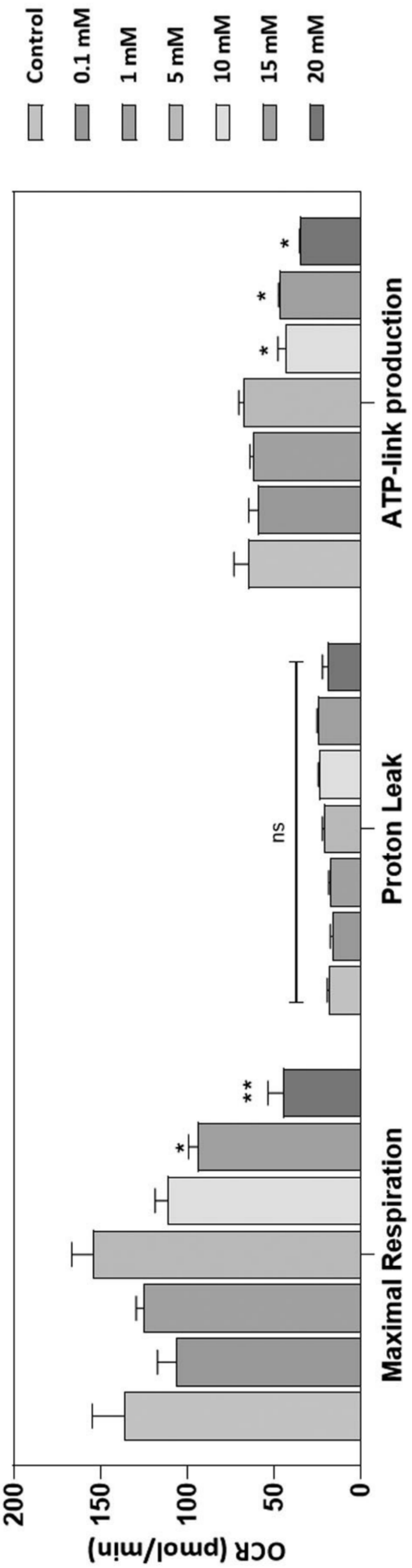
Control
0.1 mM
1 mM
5 mM
10 mM
15 mM
20 mM
OCR (pmol/min)
200
150
100
50
0
Maximal Respiration
Proton Leak
ATP-link production
ns

Figure 1E

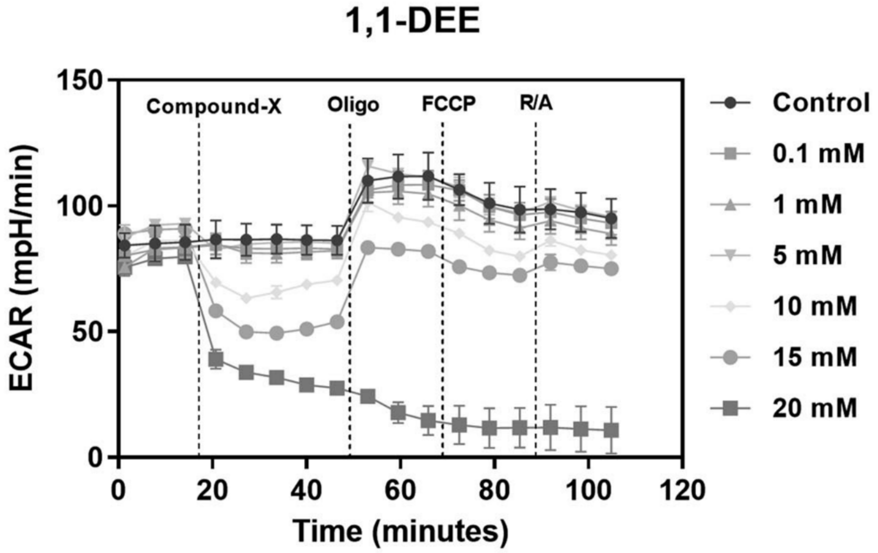
1,1-DEE
ECAR (mpH/min)
Time (minutes)
Compound-X
Oligo
FCCP
R/A
150
100
50
0
0
20
40
60
80
100
120
Control
0.1 mM
1 mM
5 mM
10 mM
15 mM
20 mM

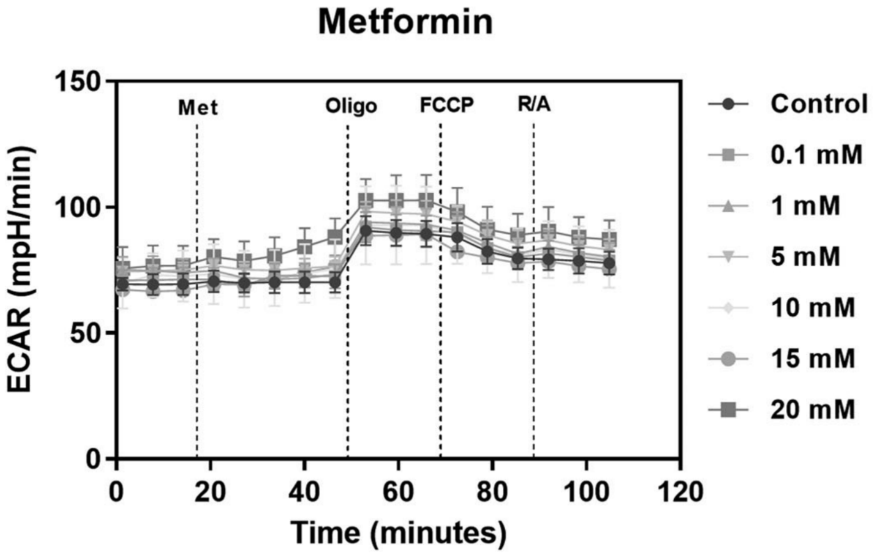
Metformin
ECAR (mpH/min)
Time (minutes)
Met
Oligo
FCCP
R/A
150
100
50
0
0
20
40
60
80
100
120
Control
0.1 mM
1 mM
5 mM
10 mM
15 mM
20 mM

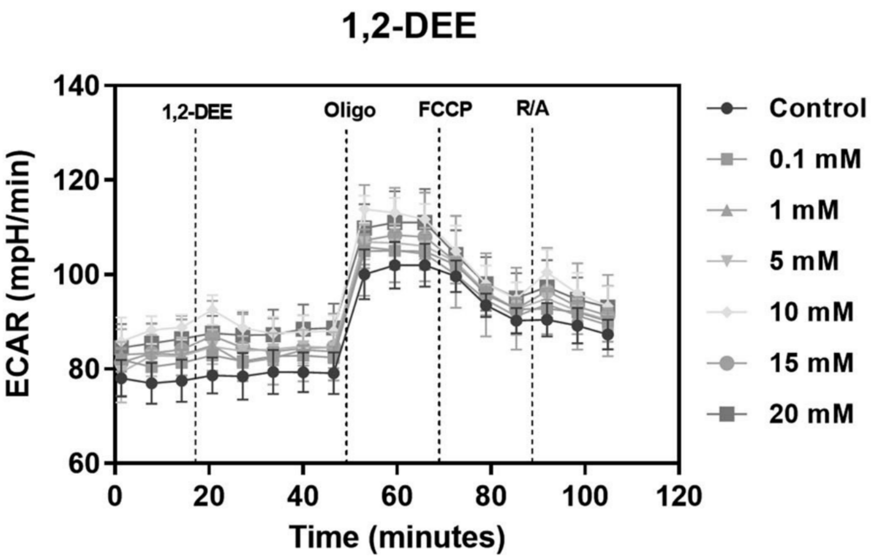
1,2-DEE
ECAR (mpH/min)
Time (minutes)
1,2-DEE
Oligo
FCCP
R/A
140
120
100
80
60
0
20
40
60
80
100
120
Control
0.1 mM
1 mM
5 mM
10 mM
15 mM
20 mM

Figure 2A

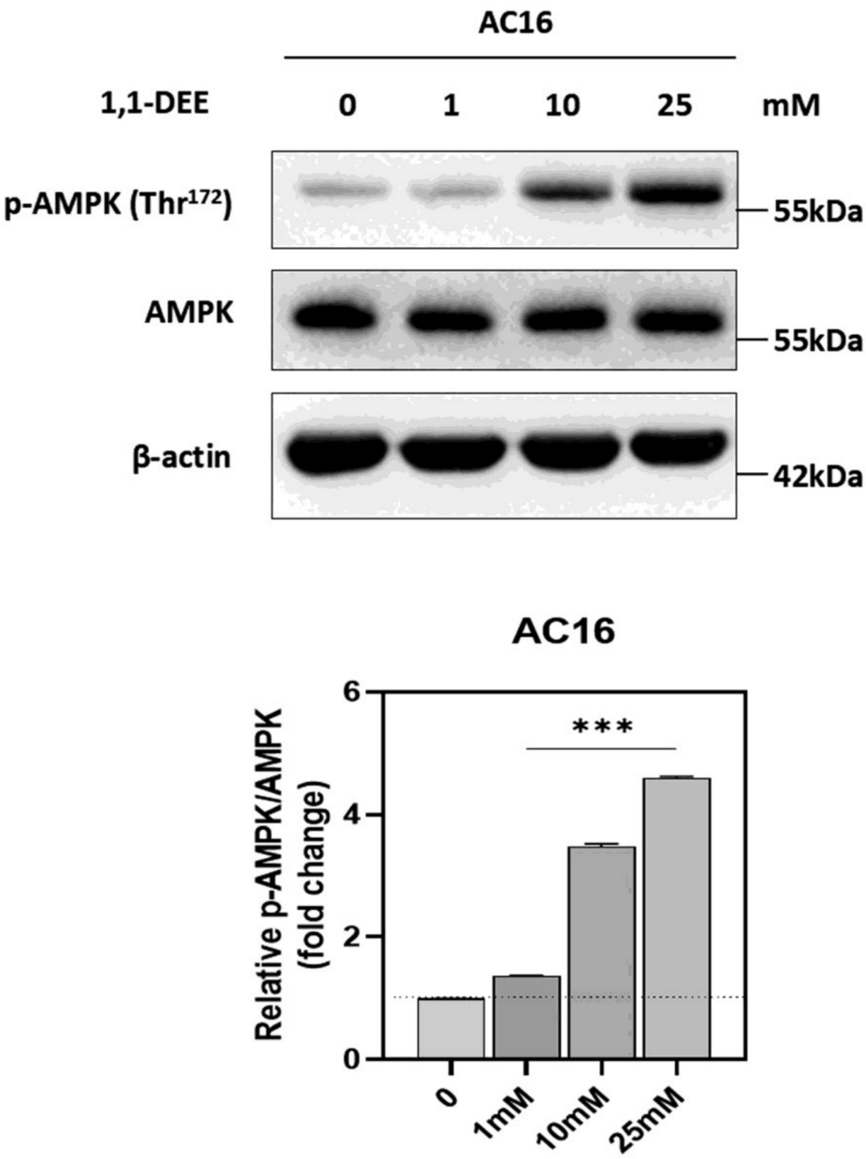
AC16
1,1-DEE
0
1
10
25
mM
p-AMPK (Thr172)
55kDa
AMPK
55kDa
β-actin
42kDa
AC16
Relative p-AMPK/AMPK (fold change)
6
4
2
0
***
0
1mM
10mM
25mM

Figure 2B

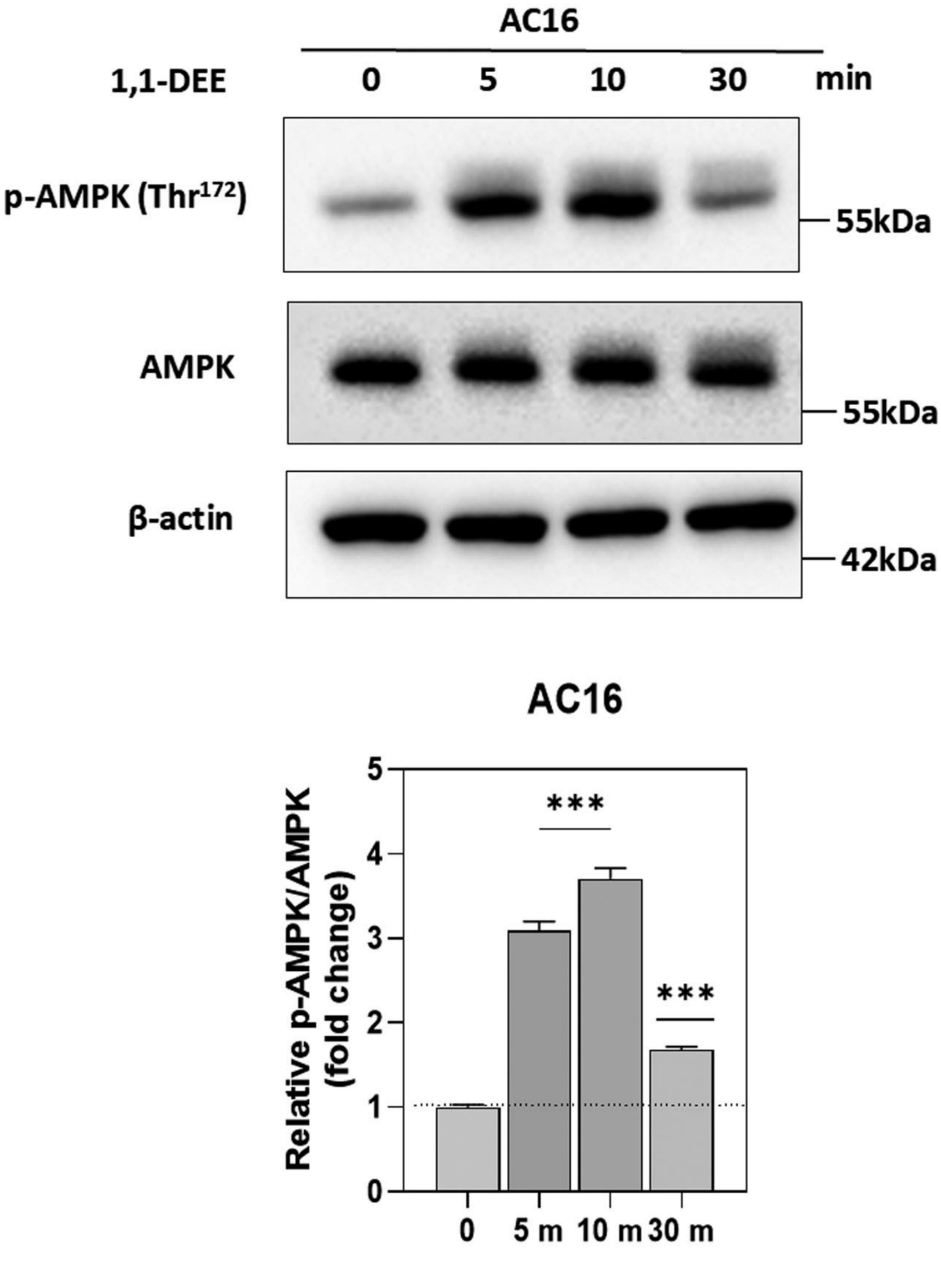
AC16
1,1-DEE
0
5
10
30
min
p-AMPK (Thr172)
55kDa
AMPK
55kDa
β-actin
42kDa
AC16
Relative p-AMPK/AMPK (fold change)
0
1
2
3
4
5
***
***
0
5 m
10 m
30 m

Figure 2C

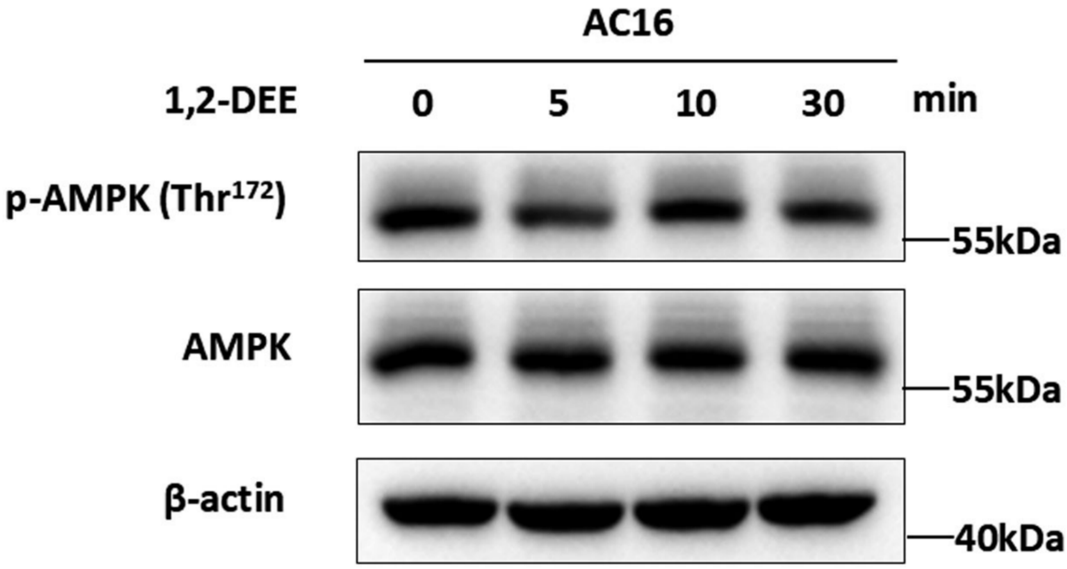


Figure 2D

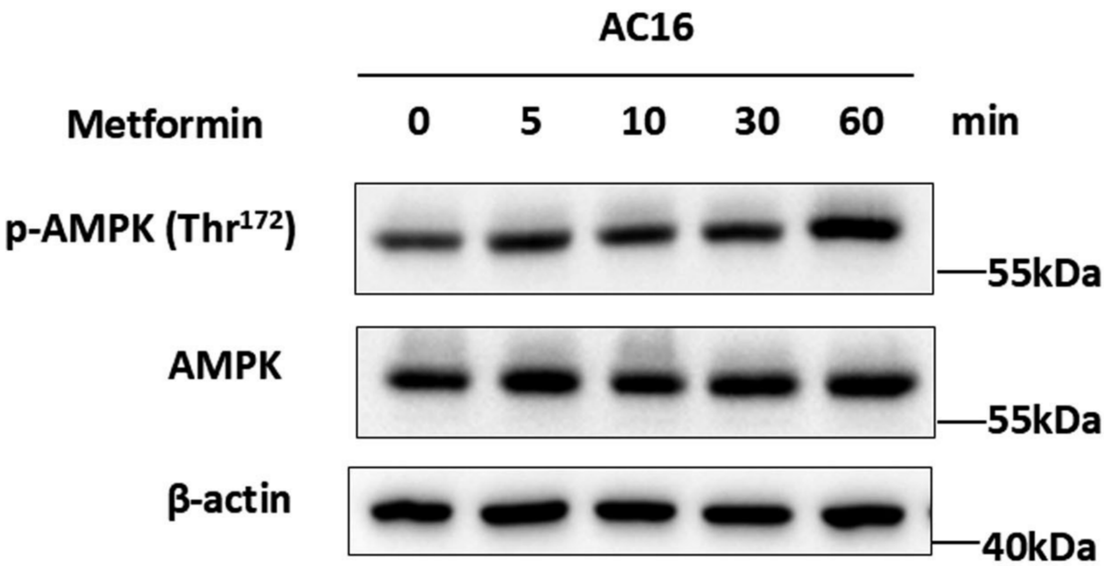

Figure 3A

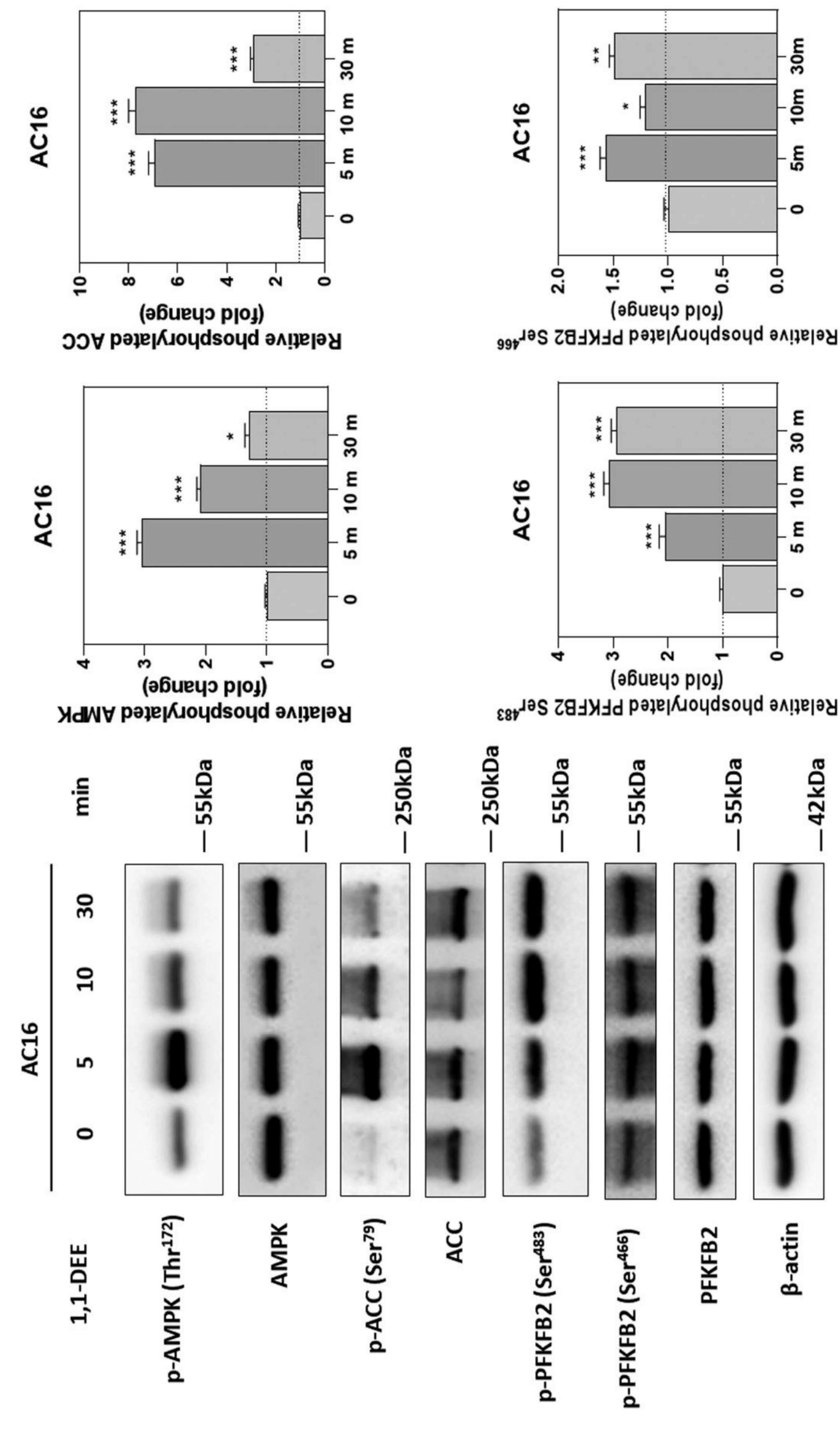
AC16
1,1-DEE
0
5
10
30
min
p-AMPK (Thr172)
AMPK
p-ACC (Ser79)
ACC
p-PFKFB2 (Ser483)
p-PFKFB2 (Ser466)
PFKFB2
β-actin
55kDa
250kDa
42kDa
Relative phosphorylated AMPK (fold change)
Relative phosphorylated ACC (fold change)
Relative phosphorylated PFKFB2 Ser483 (fold change)
Relative phosphorylated PFKFB2 Ser466 (fold change)
5 m
10 m
30 m

Figure 3B

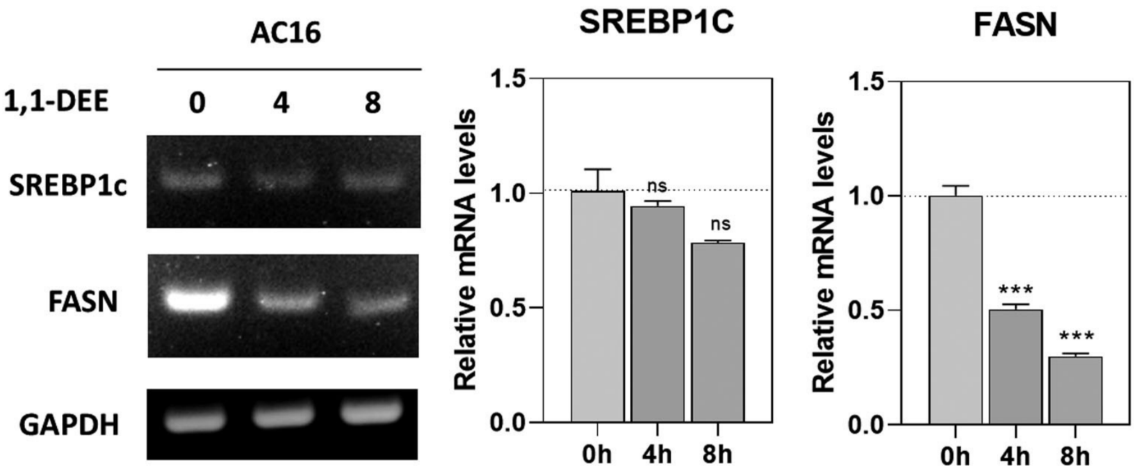


Figure 3C

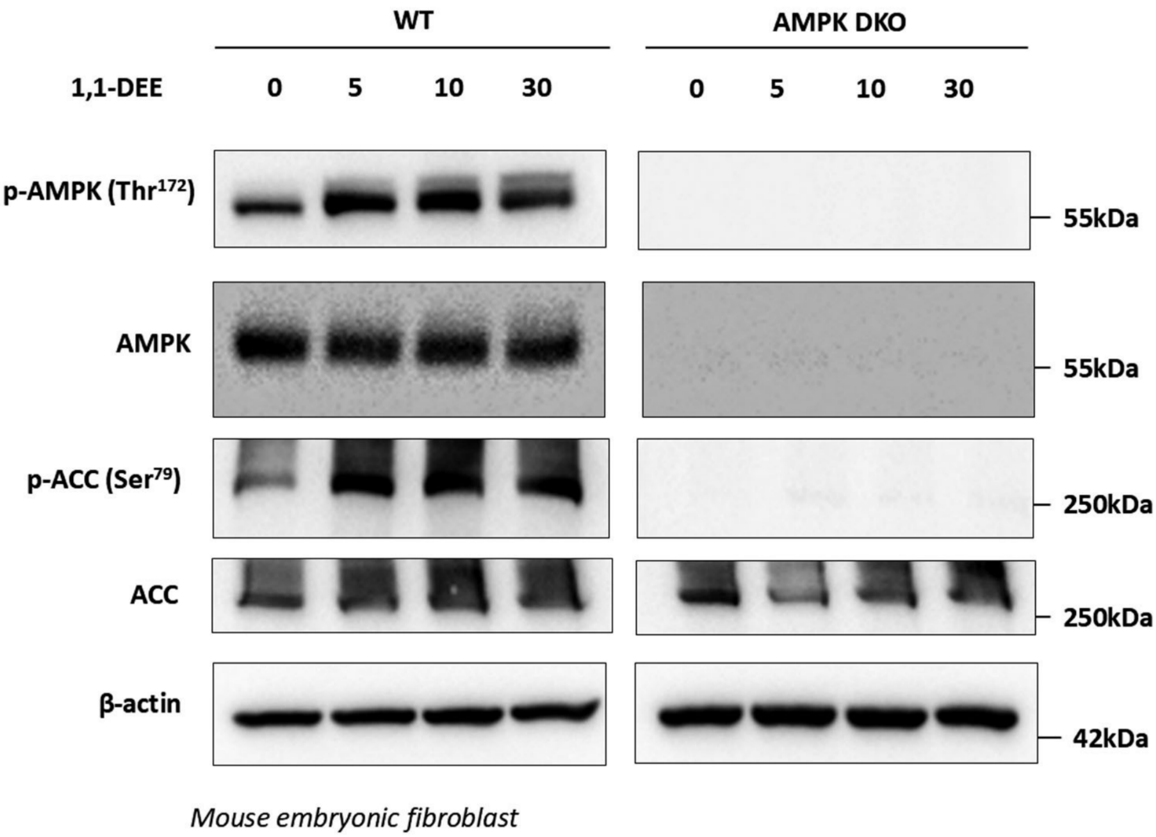

Figure 3D

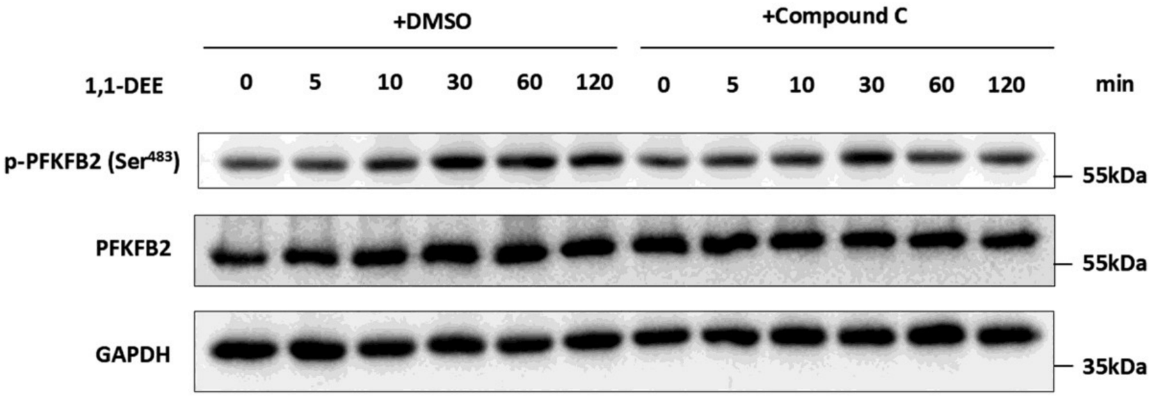


Figure 4A

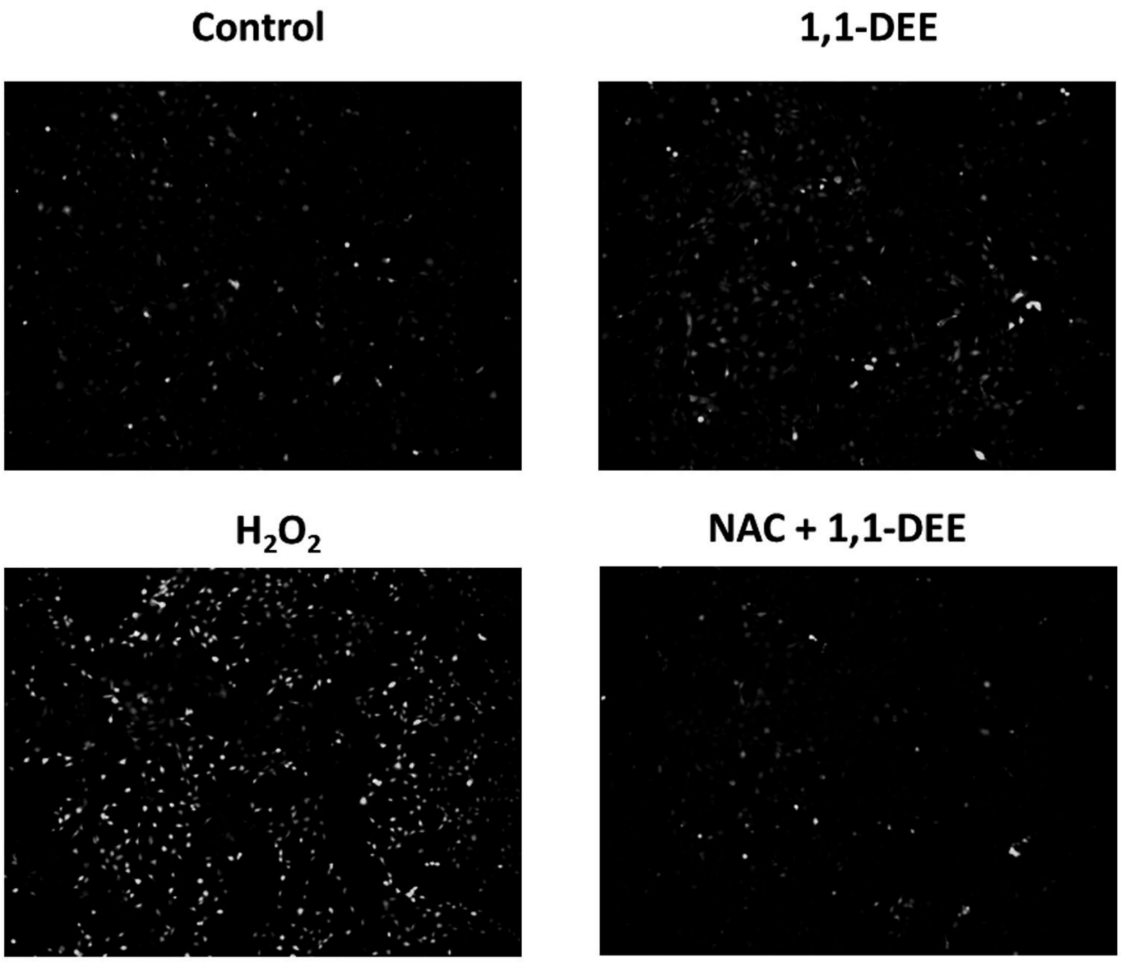

Figure 4B

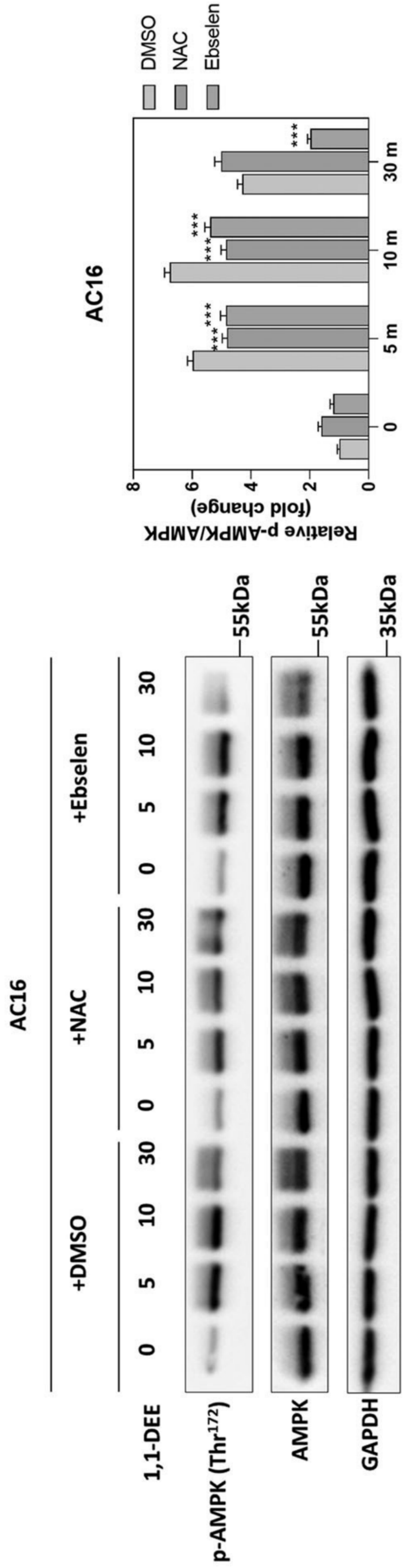
AC16
DMSO
NAC
Ebselen
Relative p-AMPK/AMPK (fold change)
0
2
4
6
8
0
5 m
10 m
30 m
***
AC16
1,1-DEE
+DMSO
+NAC
+Ebselen
0 5 10 30
0 5 10 30
0 5 10 30
p-AMPK (Thr172)
AMPK
GAPDH
55kDa
55kDa
35kDa

Figure 5A

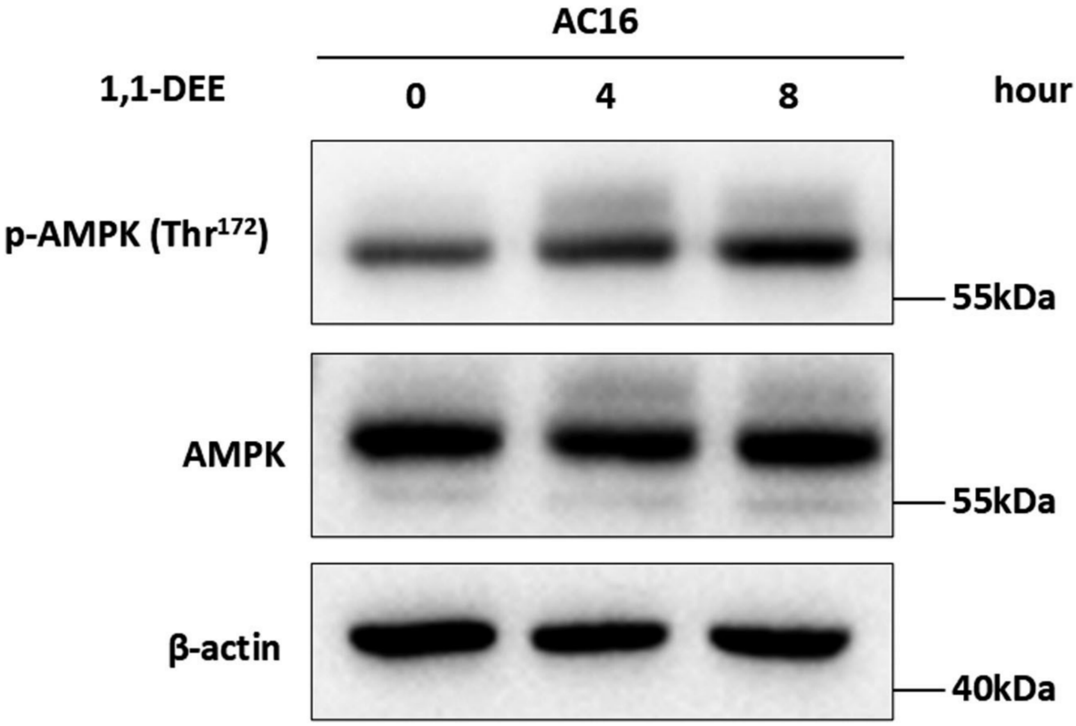


Figure 5B

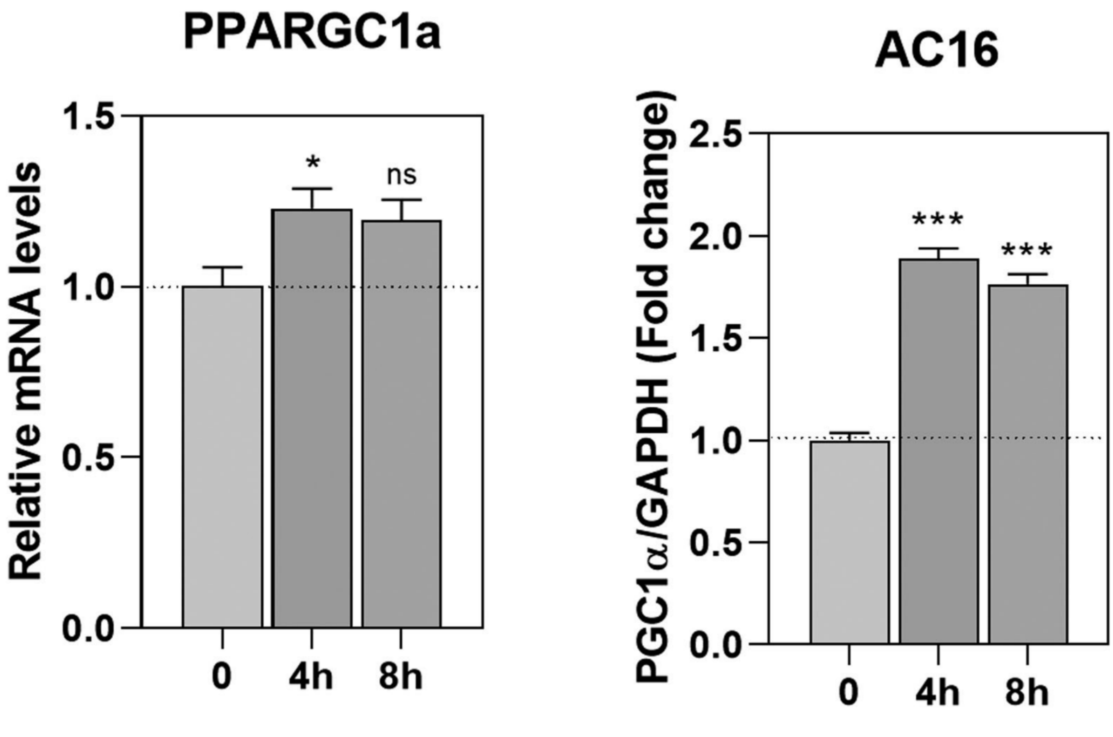

Figure 5C

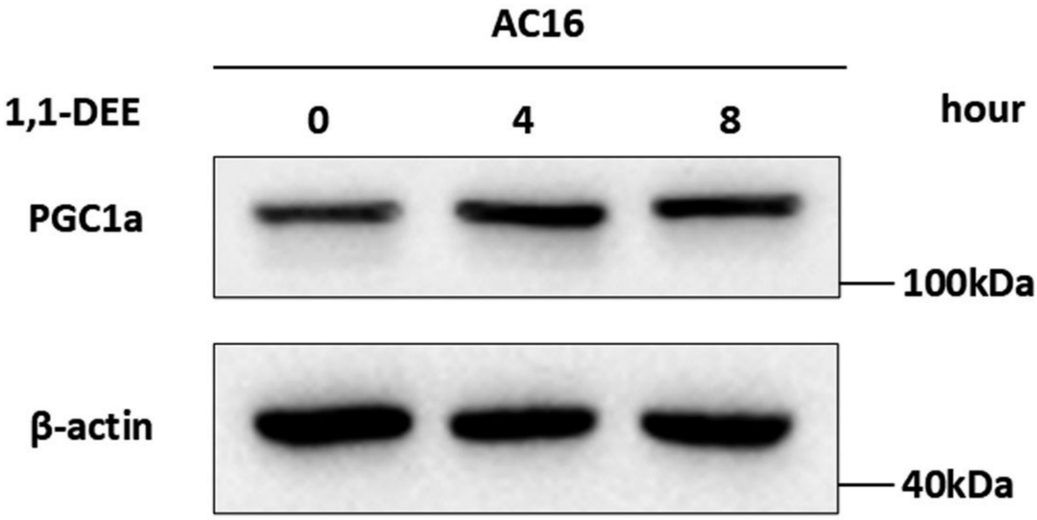


Figure 5D

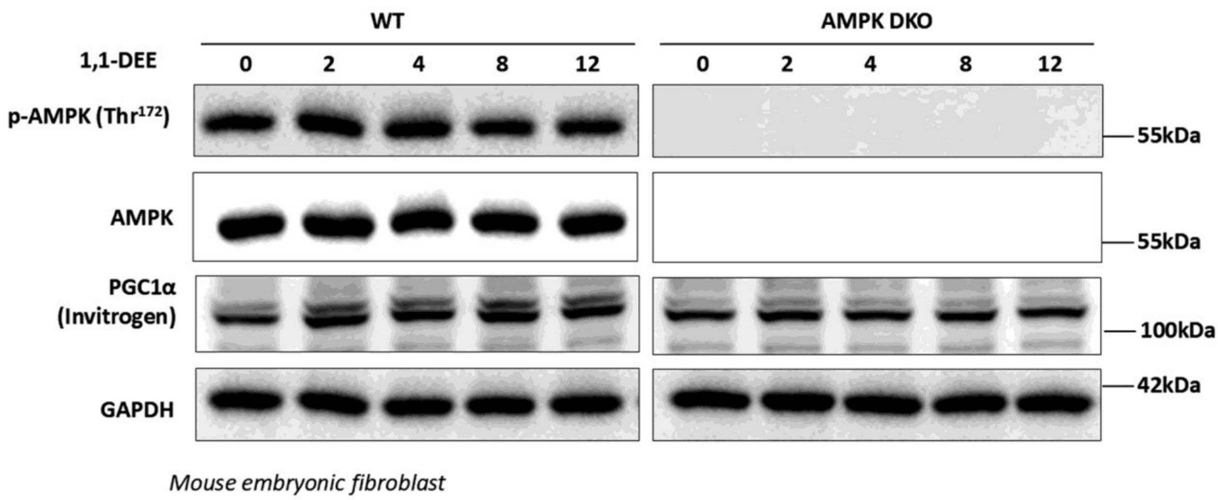

Figure 6A

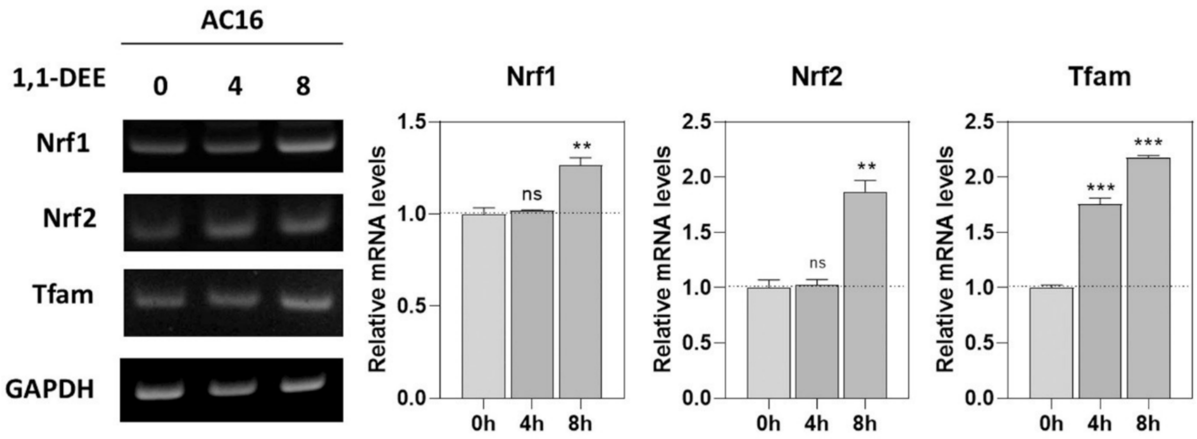


Figure 6B

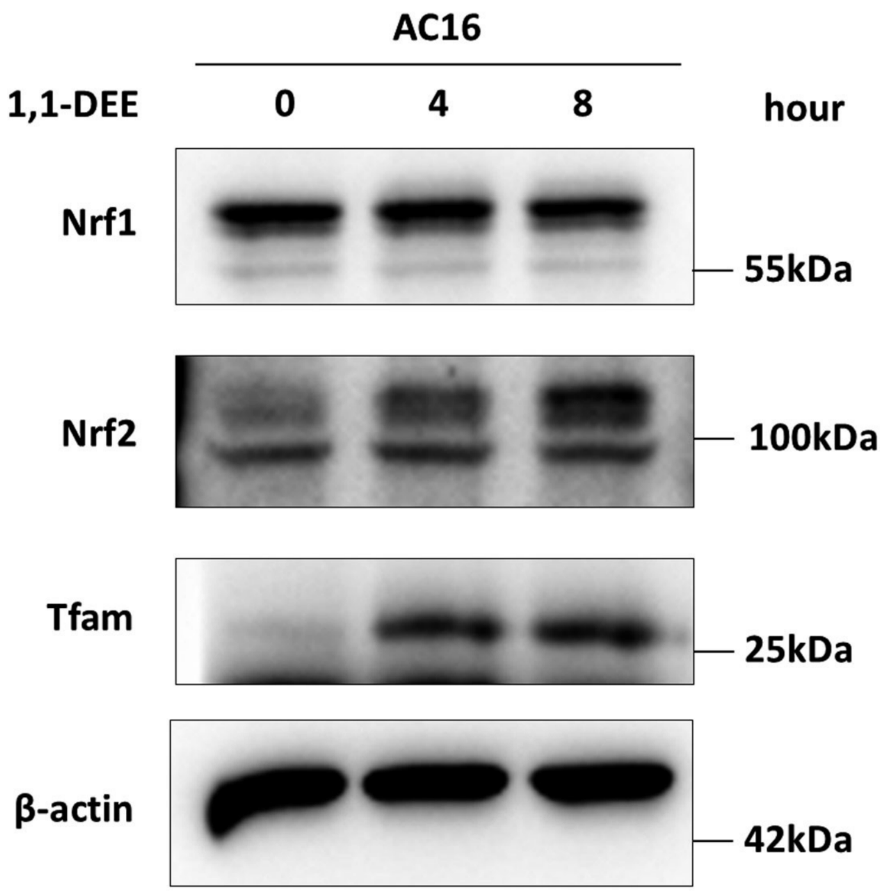

Figure 6C

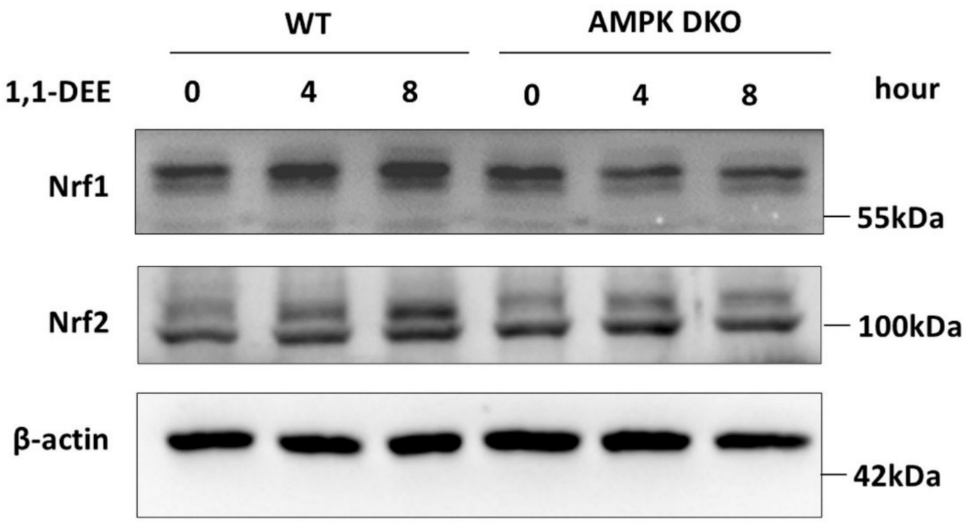


*Mouse embryonic fibroblast*

Figure 6D

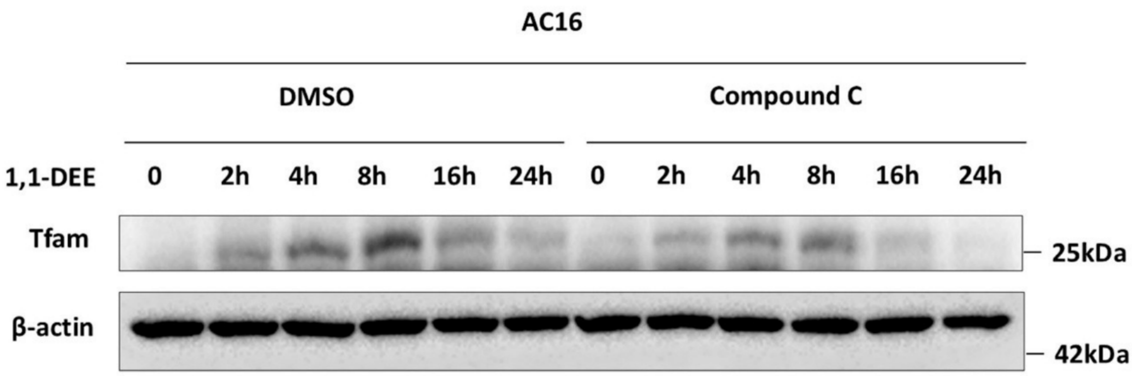

Figure 6E

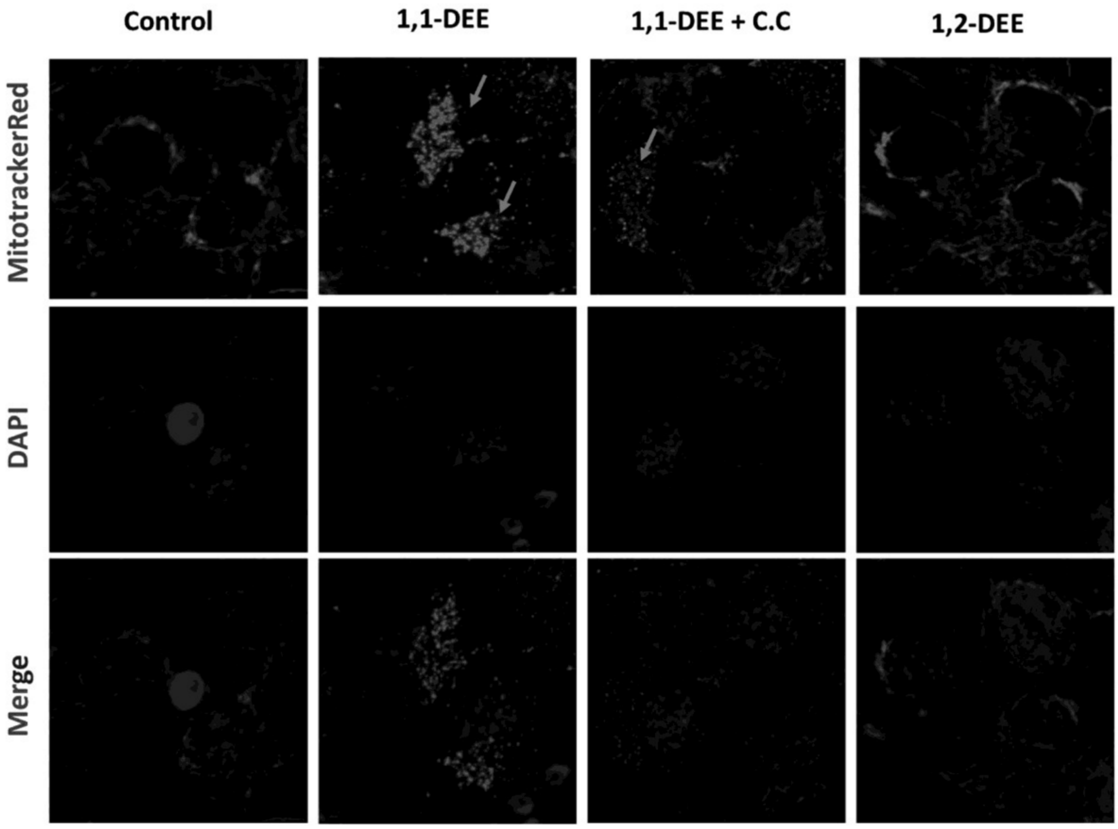


Figure 6F

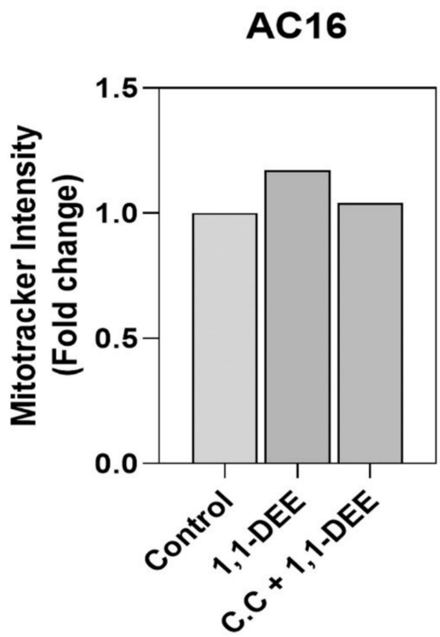

Figure 7A

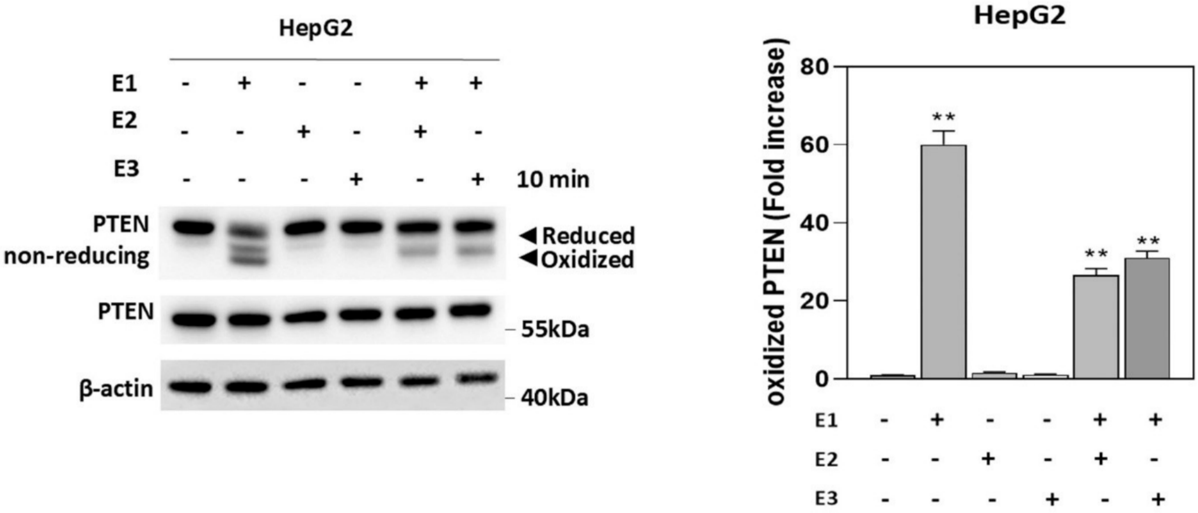


Figure 7B

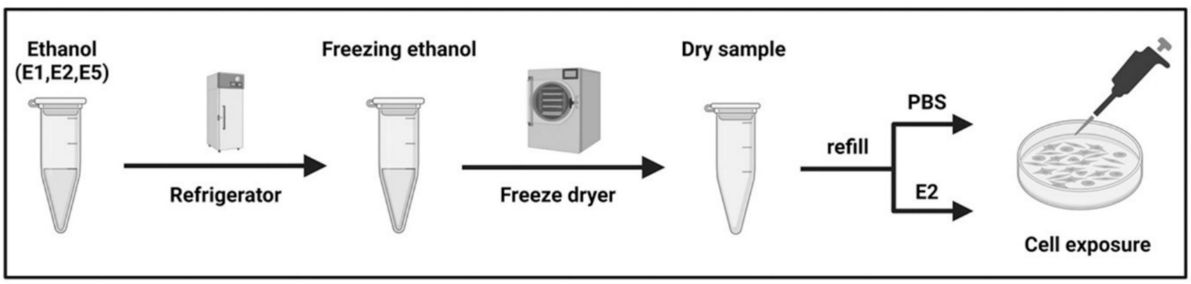

Figure 7C

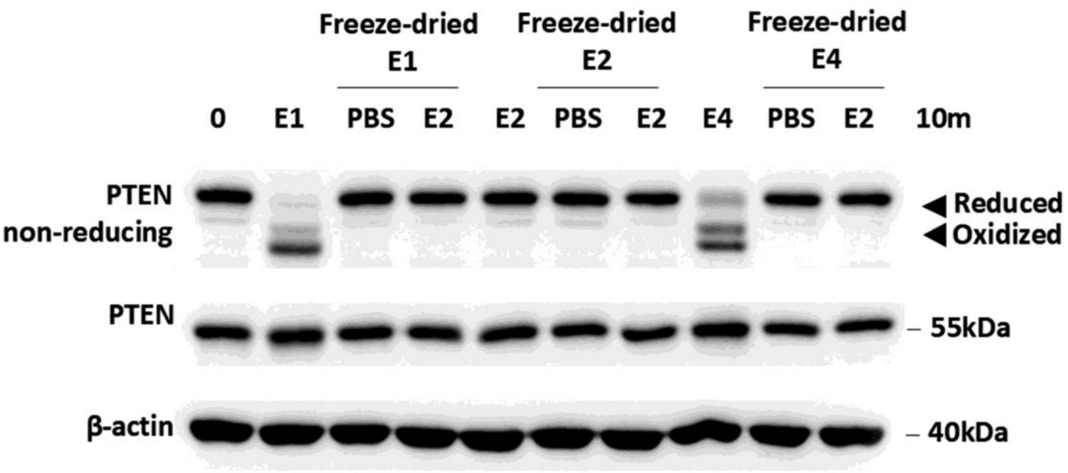


Figure 7D

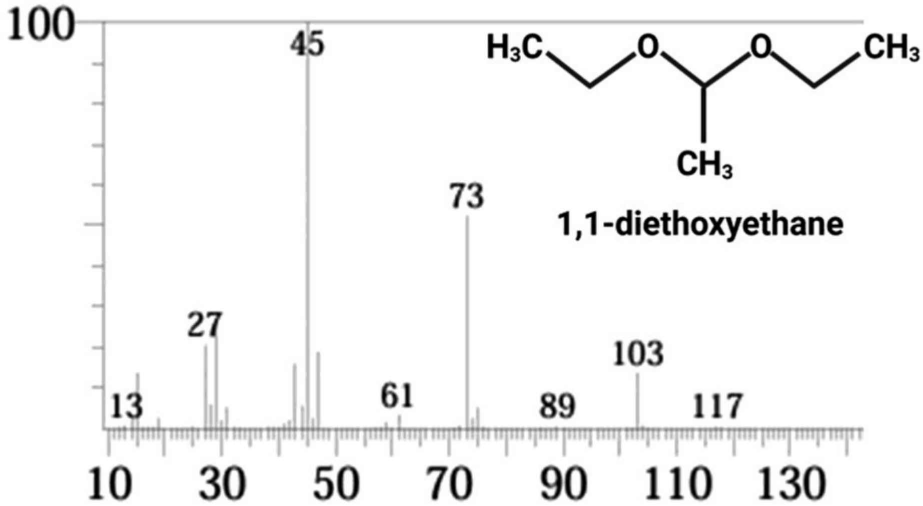

**Figure 8A**

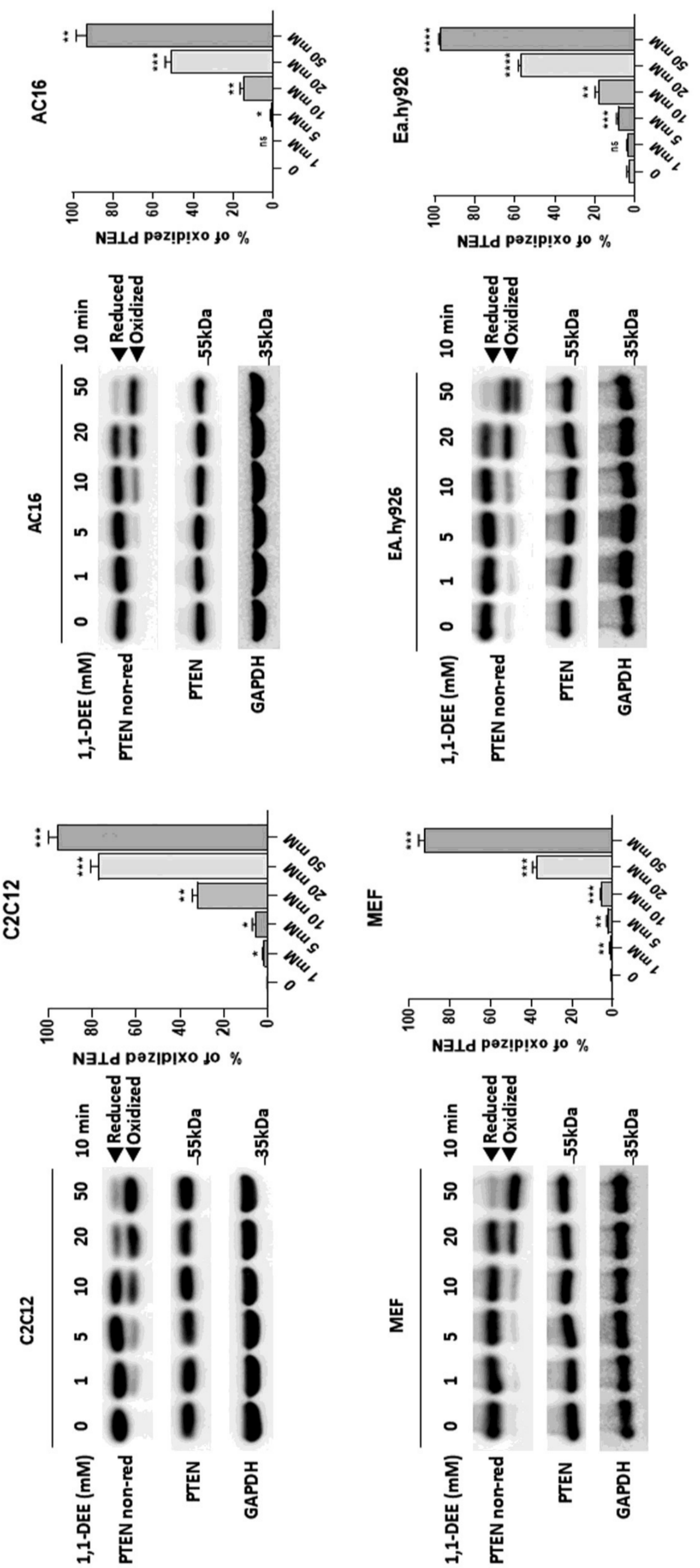
C2C12
AC16
MEF
EA.hy926
Ea.hy926
1,1-DEE (mM)
0
1
5
10
20
50
10 min
PTEN non-red
PTEN
GAPDH
Reduced
Oxidized
55kDa
35kDa
% of oxidized PTEN
0
20
40
60
80
100
0
1 mM
5 mM
10 mM
20 mM
50 mM
ns
*
**
***
****

Figure 8B

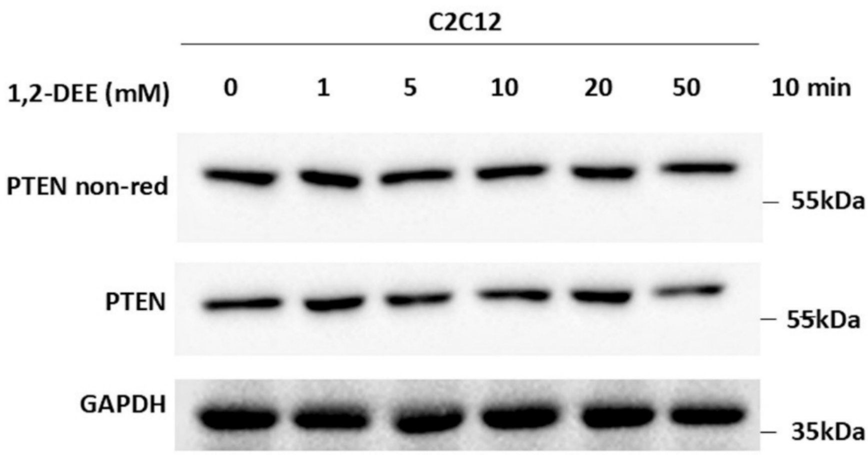


Figure 8C

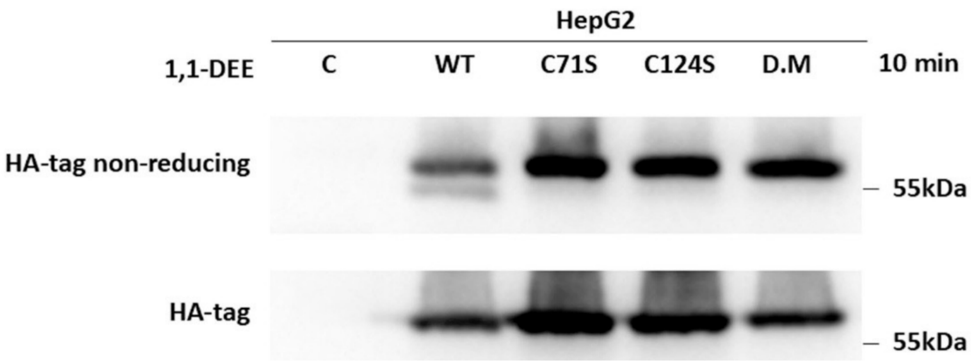

**Figure 9A**

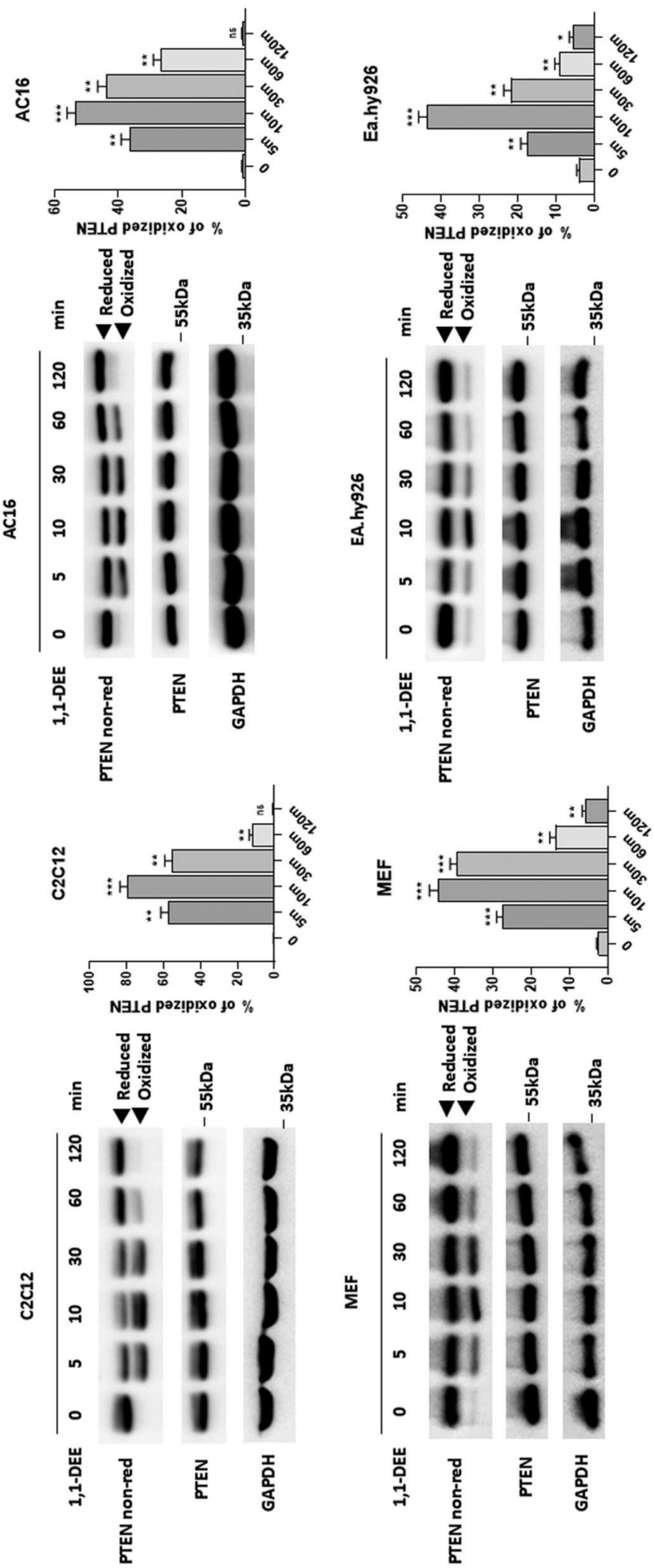

C2C12
1,1-DEE
0
5
10
30
60
120
min
PTEN non-red
Reduced
Oxidized
PTEN
55kDa
GAPDH
35kDa
% of oxidized PTEN
0m
5m
10m
30m
60m
120m
ns
MEF
AC16
EA.hy926
Ea.hy926

Figure 9B

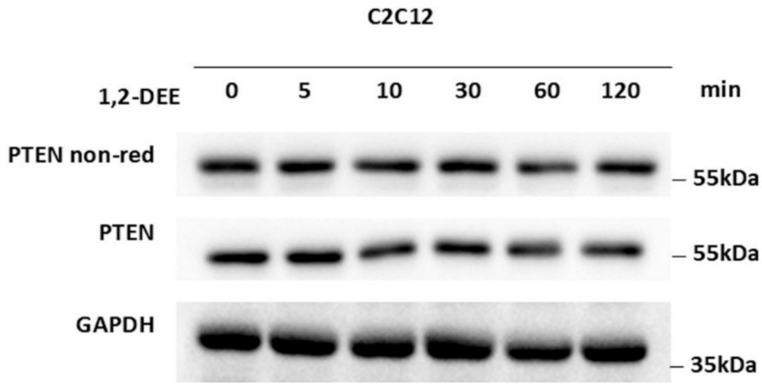
C2C12
1,2-DEE
0
5
10
30
60
120
min
PTEN non-red
PTEN
GAPDH
55kDa
55kDa
35kDa

**Figure 10A**

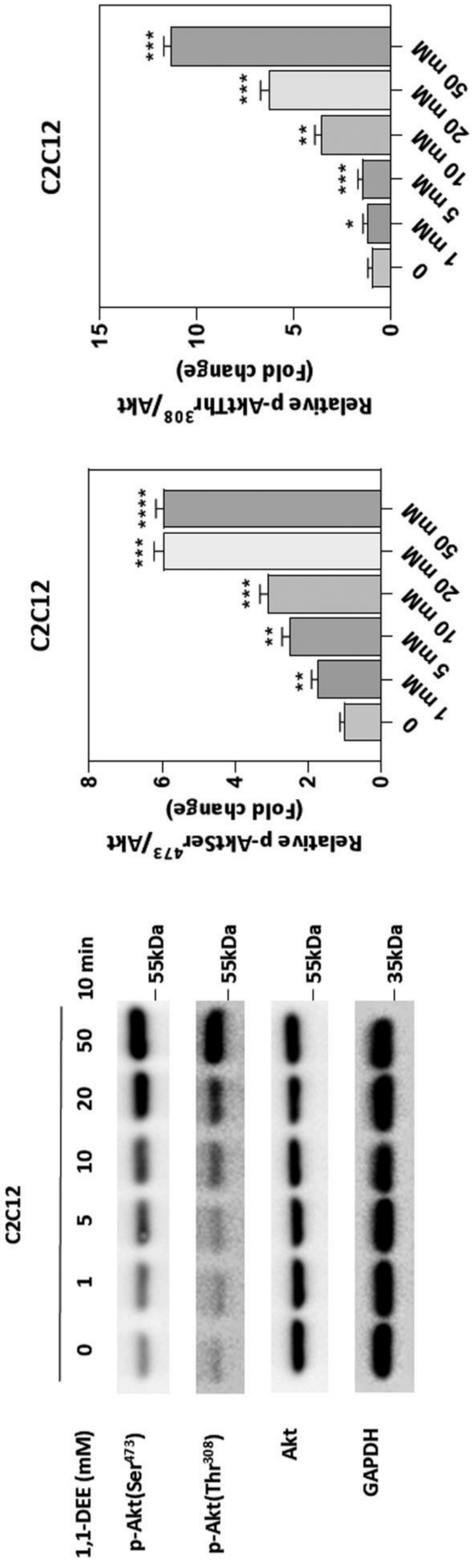
C2C12
Relative p-AktThr308/Akt
(Fold change)
0
5
10
15
0
1 mM
5 mM
10 mM
20 mM
50 mM
C2C12
Relative p-AktSer473/Akt
(Fold change)
0
2
4
6
8
0
1 mM
5 mM
10 mM
20 mM
50 mM
C2C12
1,1-DEE (mM)
0
1
5
10
20
50
10 min
p-Akt(Ser473)
p-Akt(Thr308)
Akt
GAPDH
55kDa
55kDa
55kDa
35kDa

**Figure 10B**

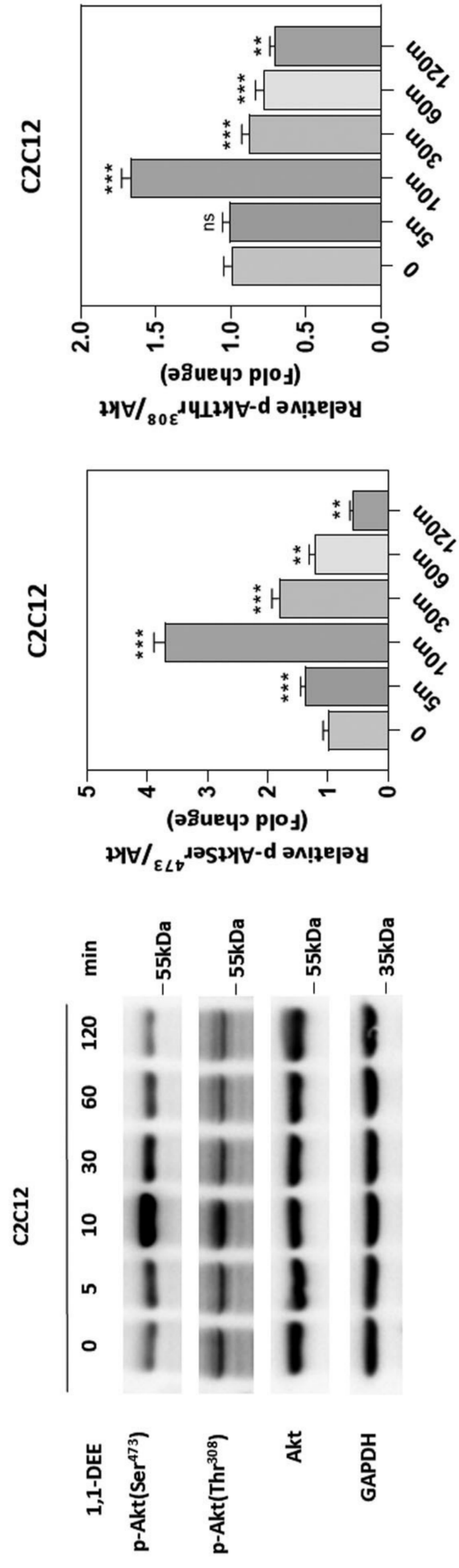
C2C12
1,1-DEE
0
5
10
30
60
120
min
p-Akt(Ser473)
p-Akt(Thr308)
Akt
GAPDH
55kDa
55kDa
55kDa
35kDa
C2C12
Relative p-AktSer473/Akt
(Fold change)
0
1
2
3
4
5
0
5m
10m
30m
60m
120m
***
***
***
**
**
C2C12
Relative p-AktThr308/Akt
(Fold change)
0.0
0.5
1.0
1.5
2.0
0
5m
10m
30m
60m
120m
ns
***
***
***
**

Figure 11A

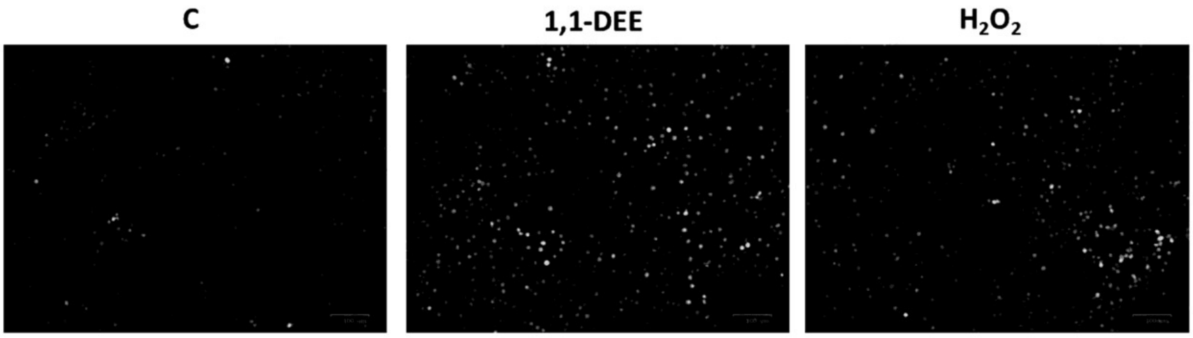


Figure 11B

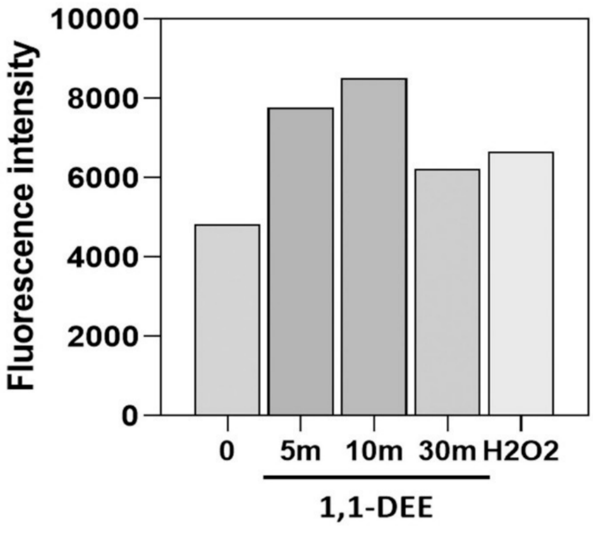

Figure 11C

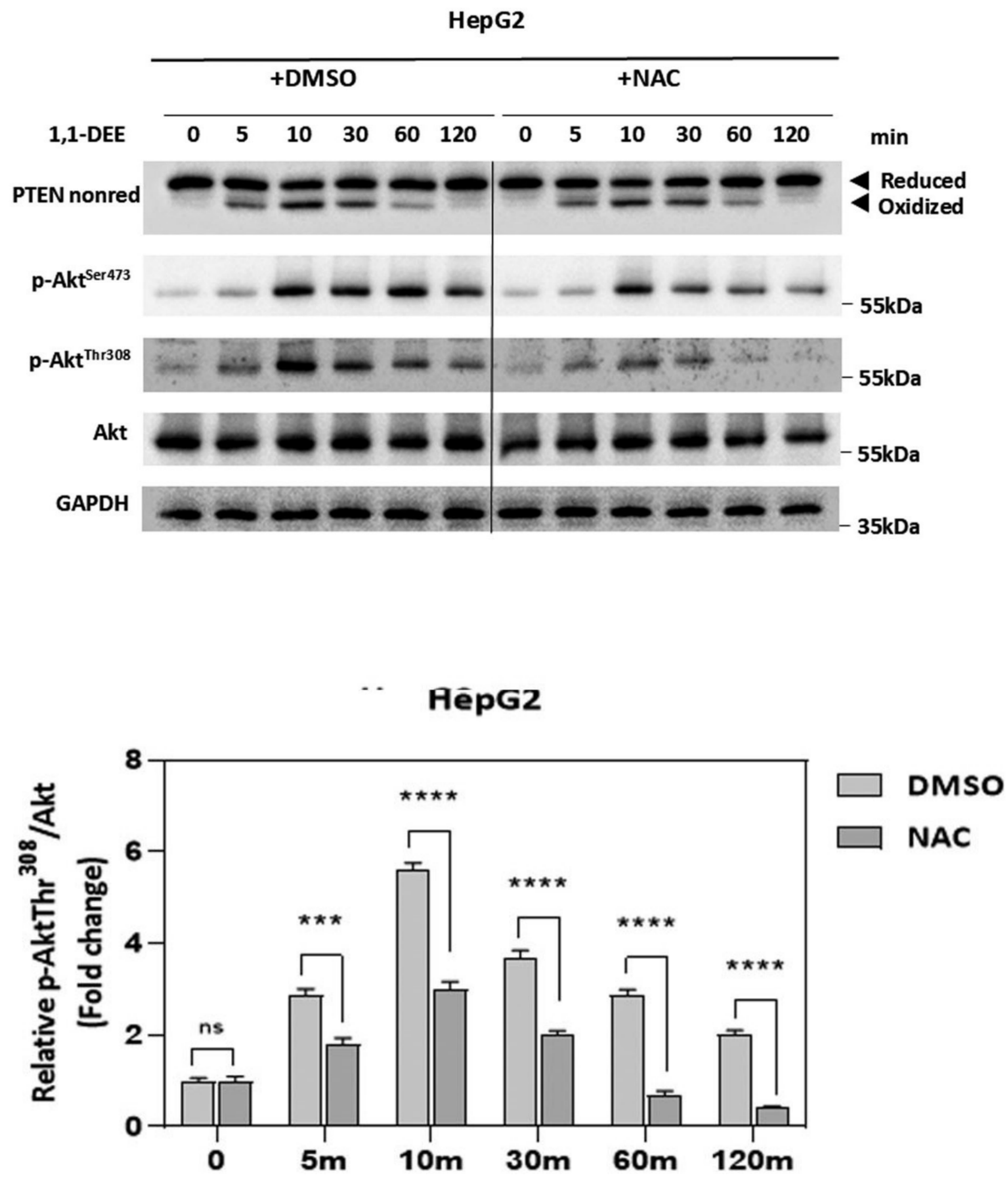
HepG2
+DMSO
+NAC
1,1-DEE
0 5 10 30 60 120 0 5 10 30 60 120
min
PTEN nonred
Reduced
Oxidized
p-AktSer473
55kDa
p-AktThr308
55kDa
Akt
55kDa
GAPDH
35kDa
HepG2
Relative p-AktThr308/Akt (Fold change)
DMSO
NAC
ns
***
****
****
****
****
0
2
4
6
8
0
5m
10m
30m
60m
120m

Figure 11C continued

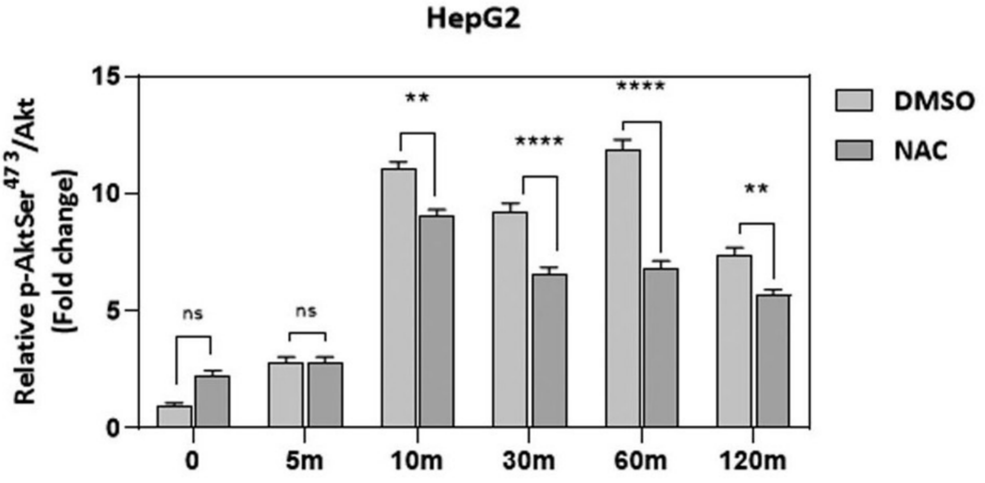


Figure 12A

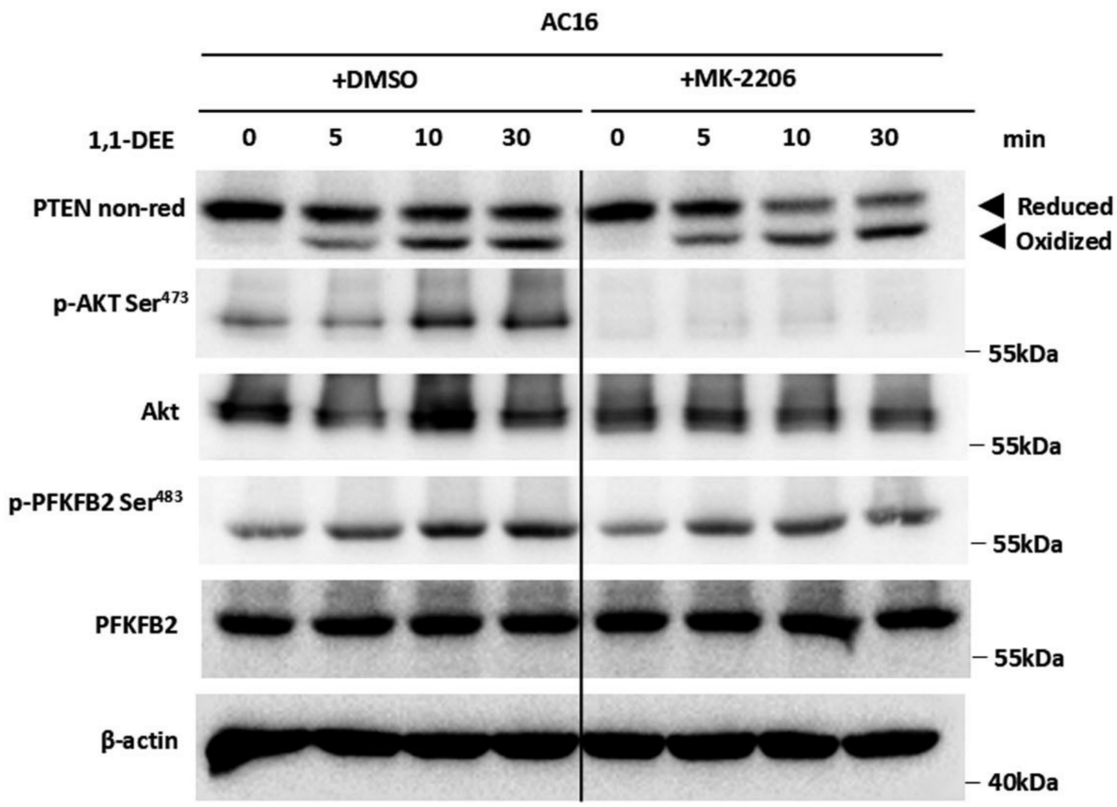

**Figure 12A continued**

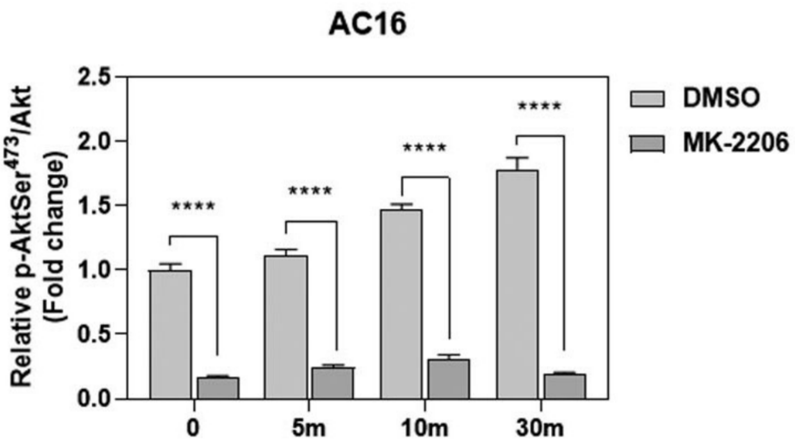


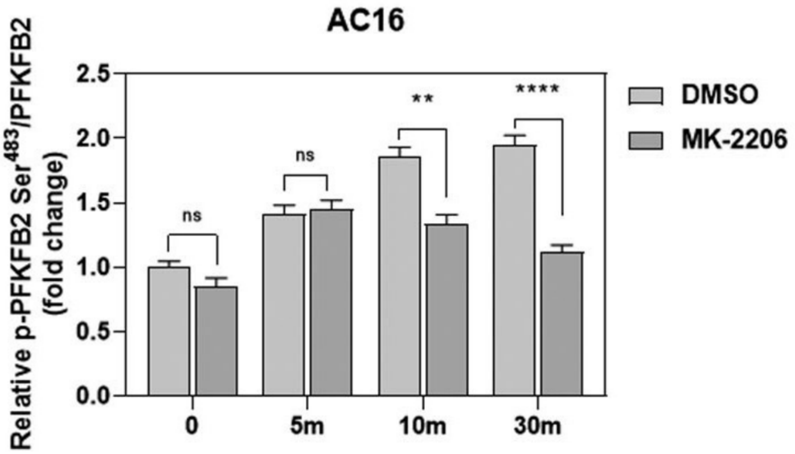

**Figure 12B**

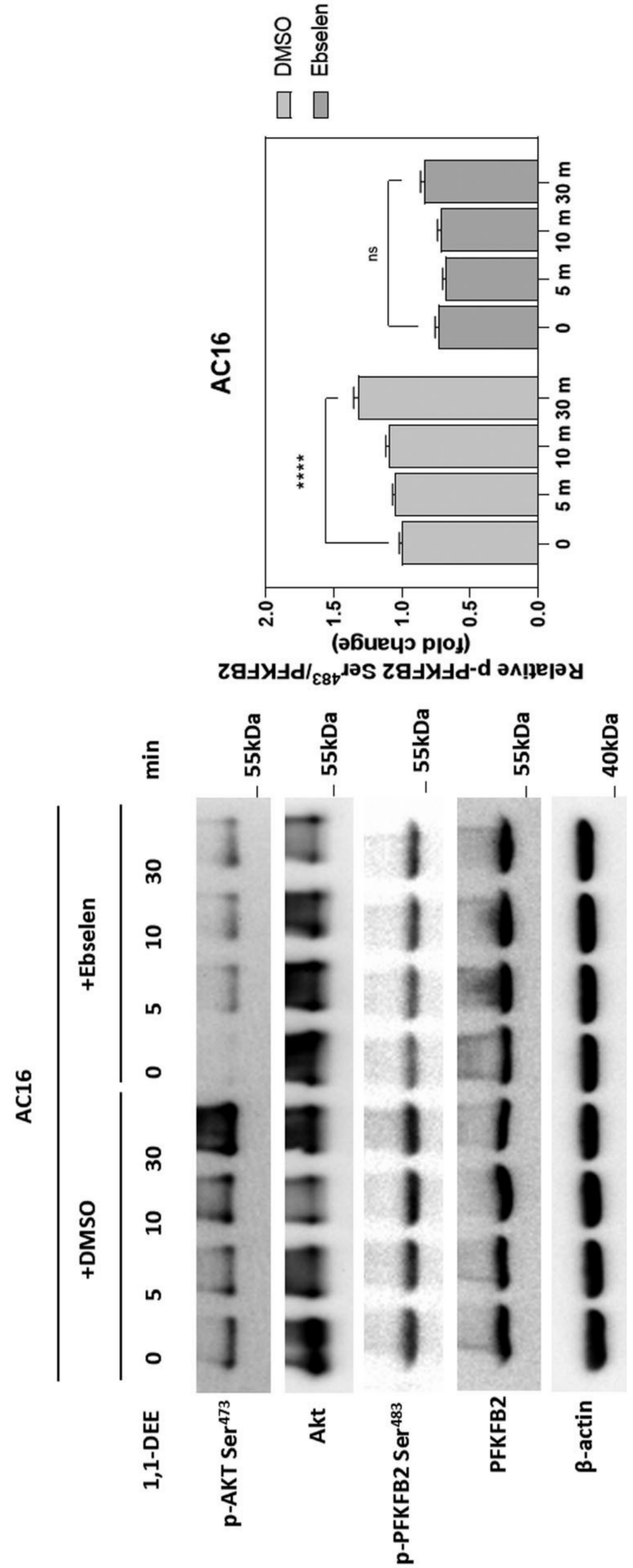
AC16
Relative p-PFKFB2 Ser483/PFKFB2 (fold change)
2.0
1.5
1.0
0.5
0.0
****
ns
0
5 m
10 m
30 m
0
5 m
10 m
30 m
DMSO
Ebselen
AC16
+DMSO
+Ebselen
1,1-DEE
0
5
10
30
0
5
10
30
min
p-AKT Ser473
Akt
p-PFKFB2 Ser483
PFKFB2
β-actin
55kDa
55kDa
55kDa
55kDa
40kDa

**Figure 13A**

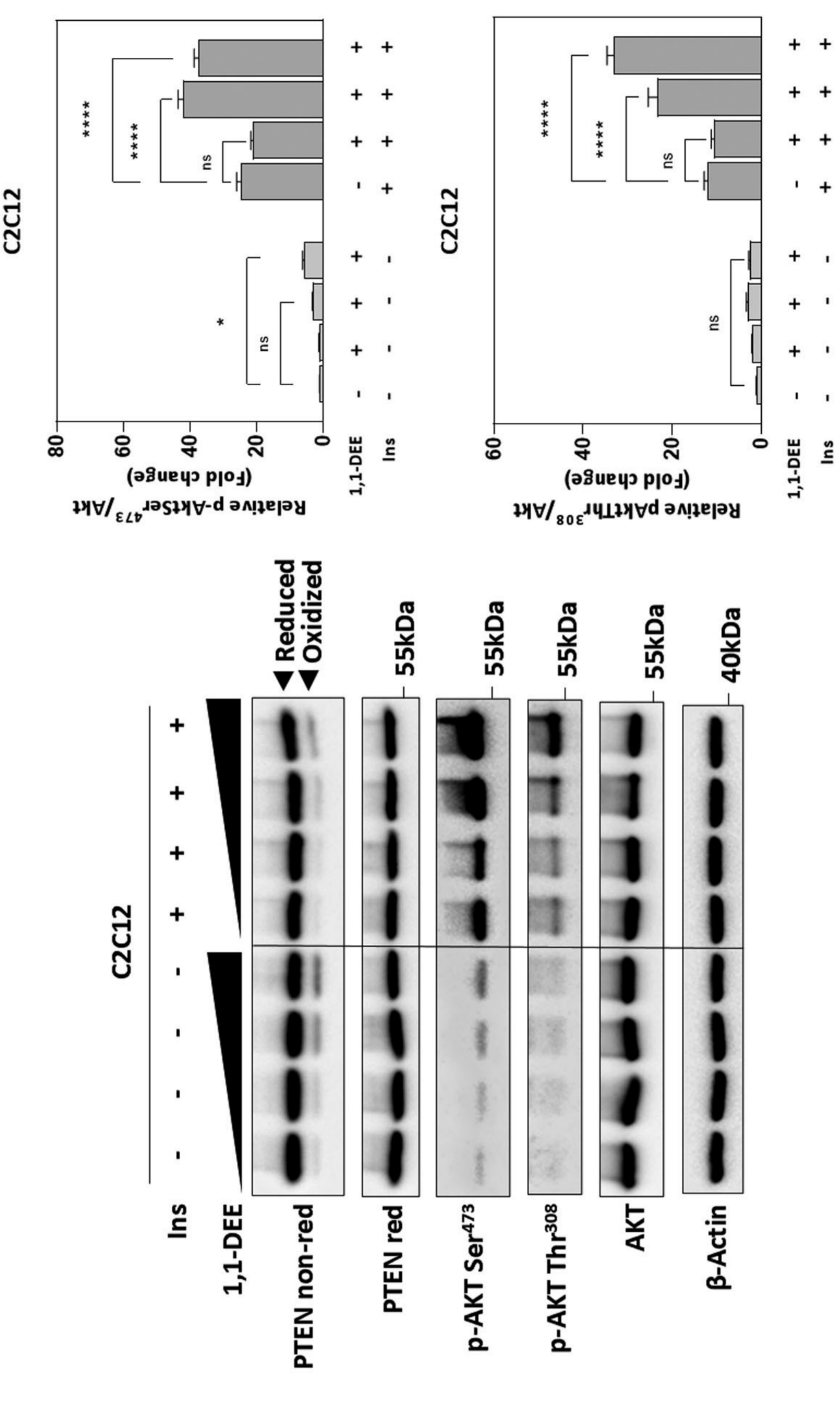
C2C12
Relative p-AktSer473/Akt (Fold change)
1,1-DEE
Ins
ns
*
****
C2C12
Relative pAktThr308/Akt (Fold change)
1,1-DEE
Ins
ns
****
C2C12
Ins
1,1-DEE
PTEN non-red
PTEN red
p-AKT Ser473
p-AKT Thr308
AKT
β-Actin
Reduced
Oxidized
55kDa
55kDa
55kDa
55kDa
40kDa

**Figure 13B**

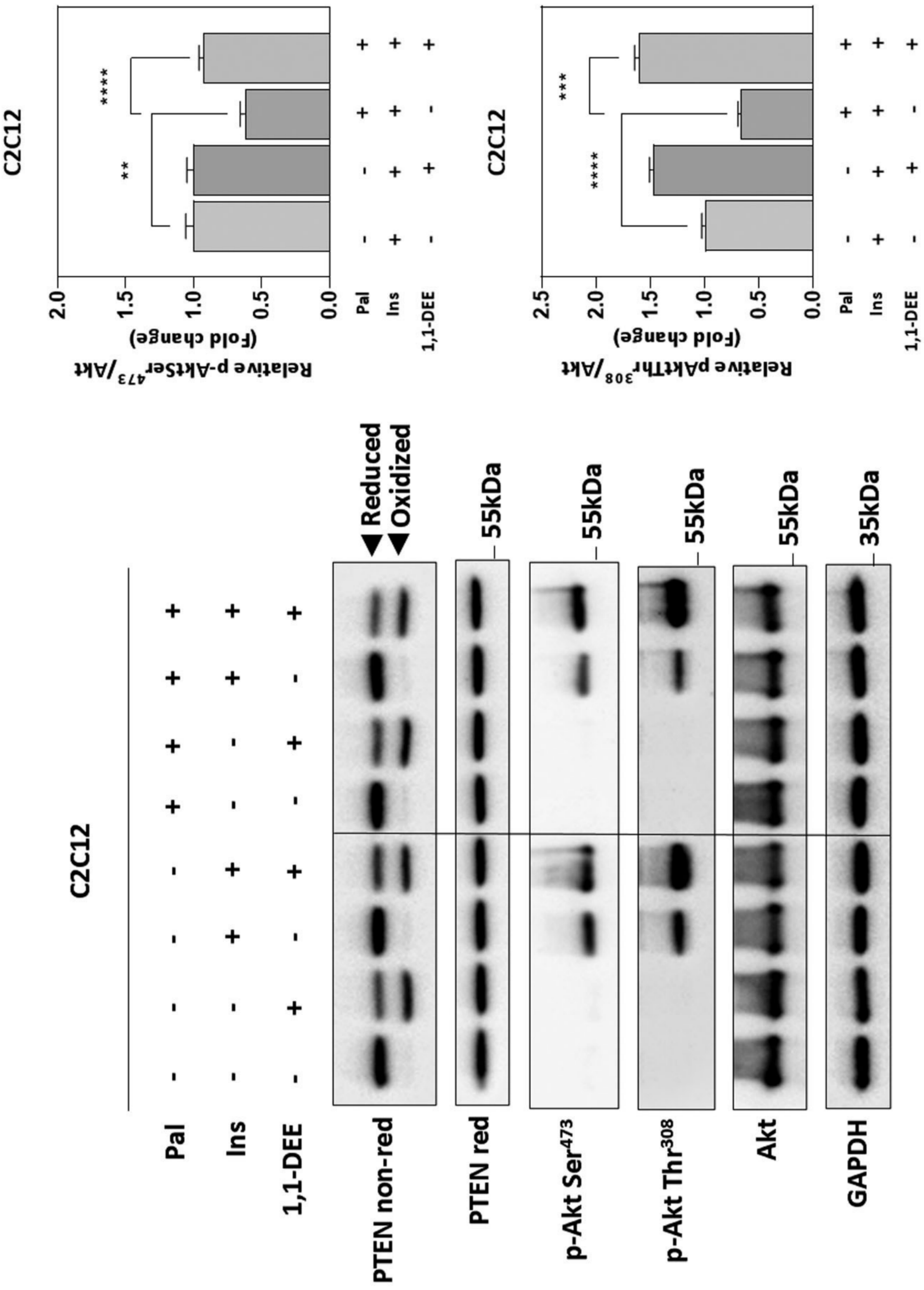
C2C12
Pal
Ins
1,1-DEE
PTEN non-red
PTEN red
p-Akt Ser473
p-Akt Thr308
Akt
GAPDH
Reduced
Oxidized
55kDa
35kDa
Relative p-AktSer473/Akt (Fold change)
Relative pAktThr308/Akt (Fold change)
**
****
***

**Figure 14**

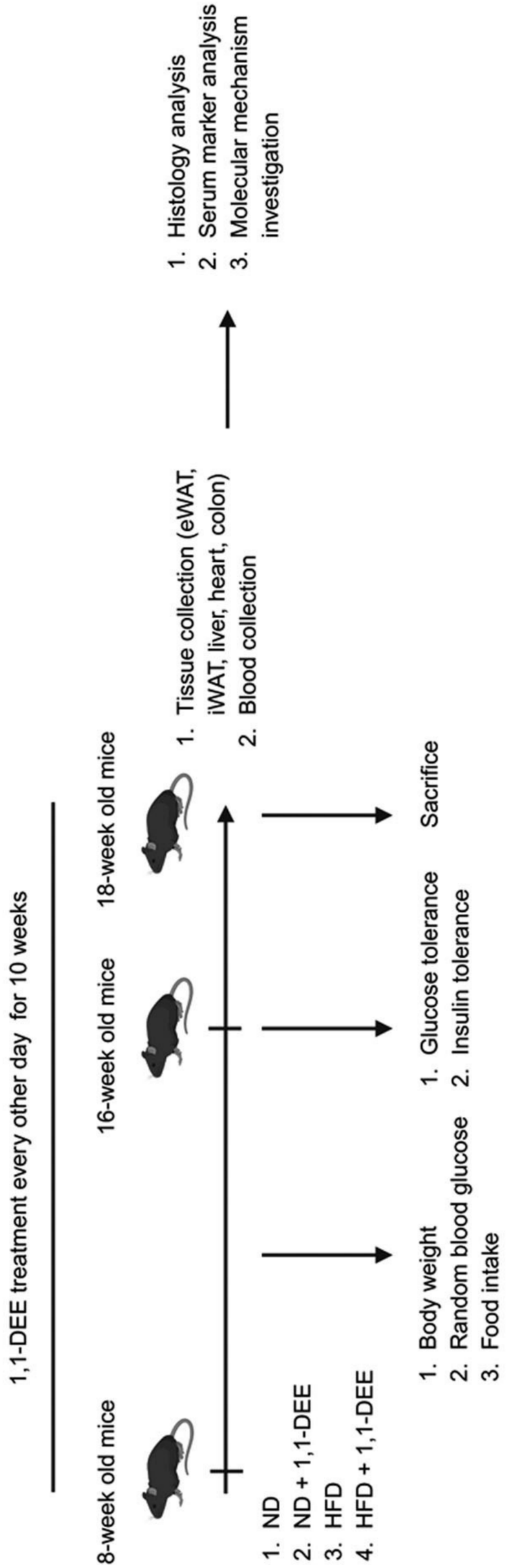
1,1-DEE treatment every other day for 10 weeks
8-week old mice
16-week old mice
18-week old mice
1. ND
2. ND + 1,1-DEE
3. HFD
4. HFD + 1,1-DEE
1. Body weight
2. Random blood glucose
3. Food intake
1. Glucose tolerance
2. Insulin tolerance
Sacrifice
1. Tissue collection (eWAT, iWAT, liver, heart, colon)
2. Blood collection
1. Histology analysis
2. Serum marker analysis
3. Molecular mechanism investigation

Figure 15A

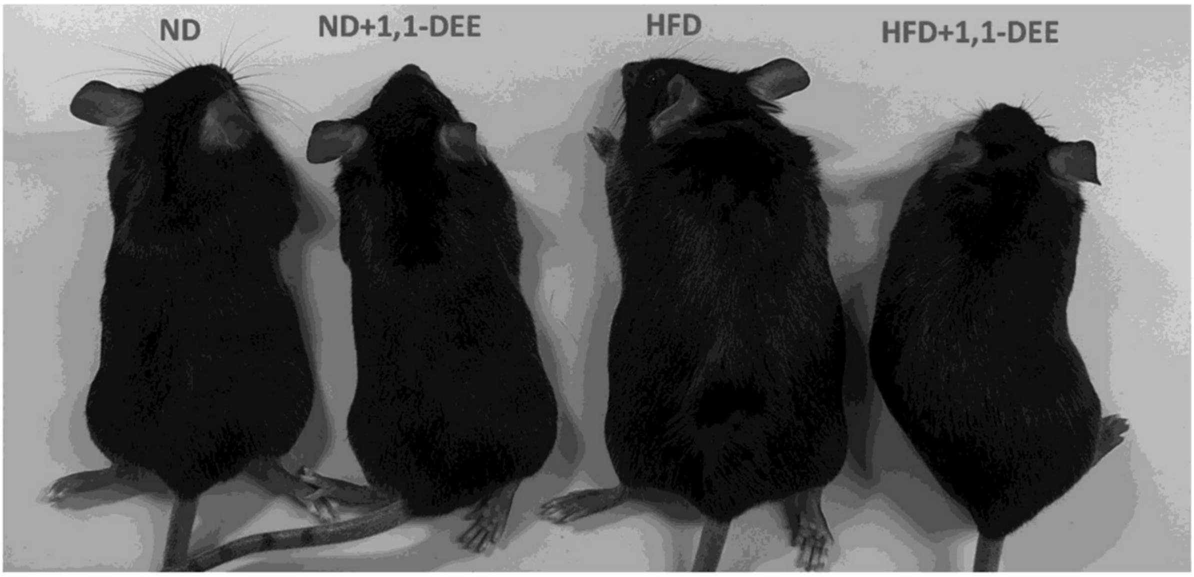


Figure 15B

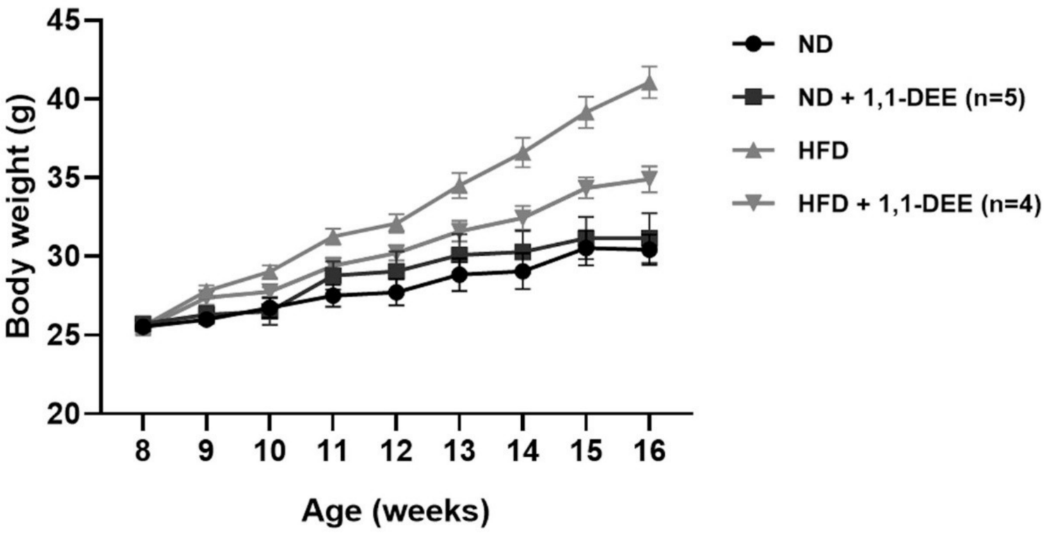

Figure 15C

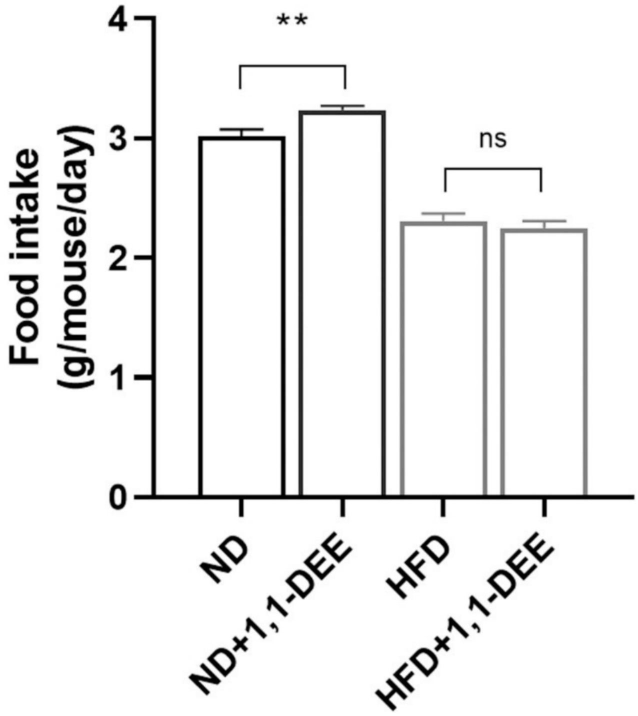


Figure 15D

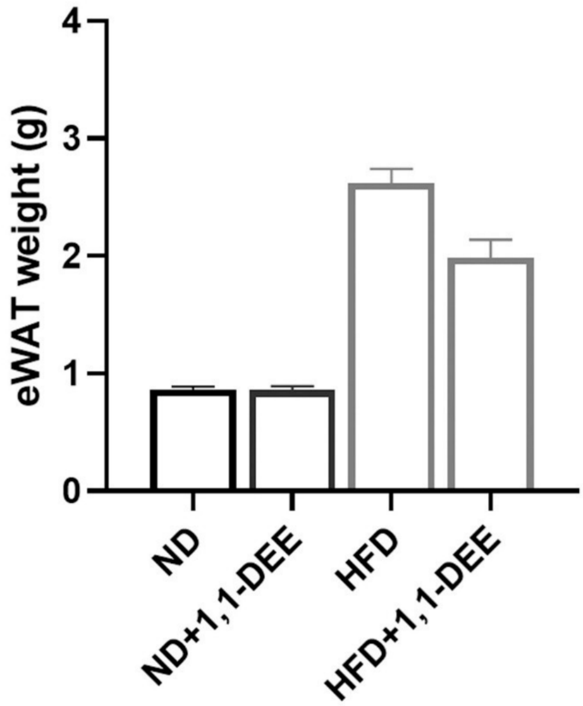

Figure 15E

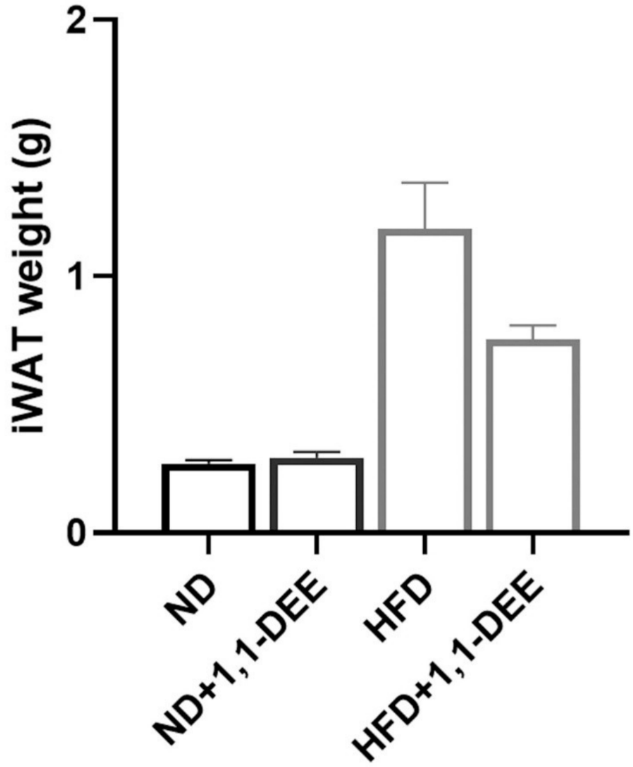


Figure 15F

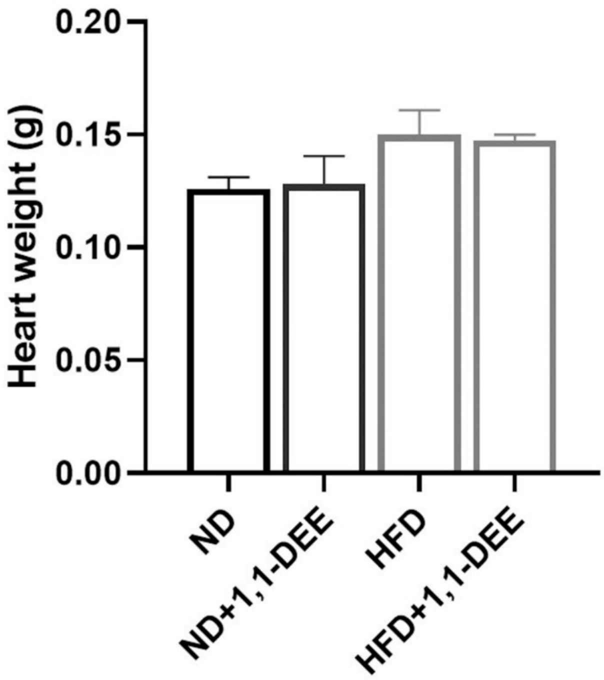

Figure 15G

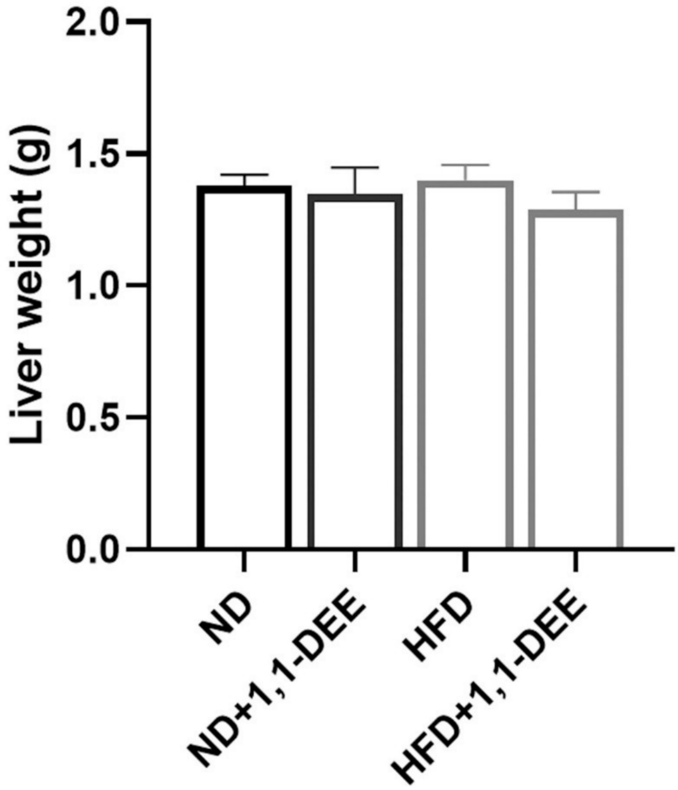


Figure 15H

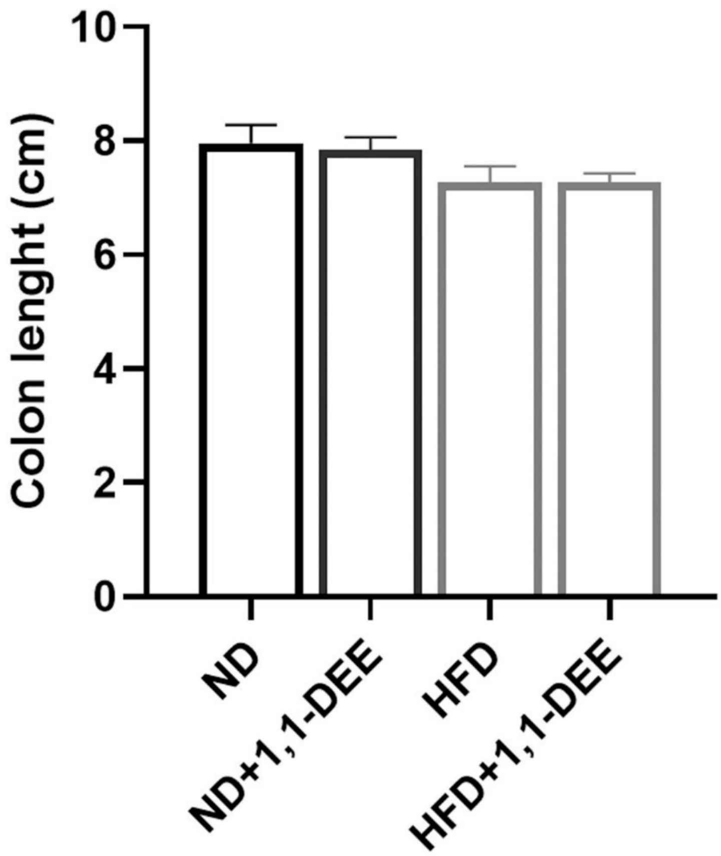

**Figure 16A**

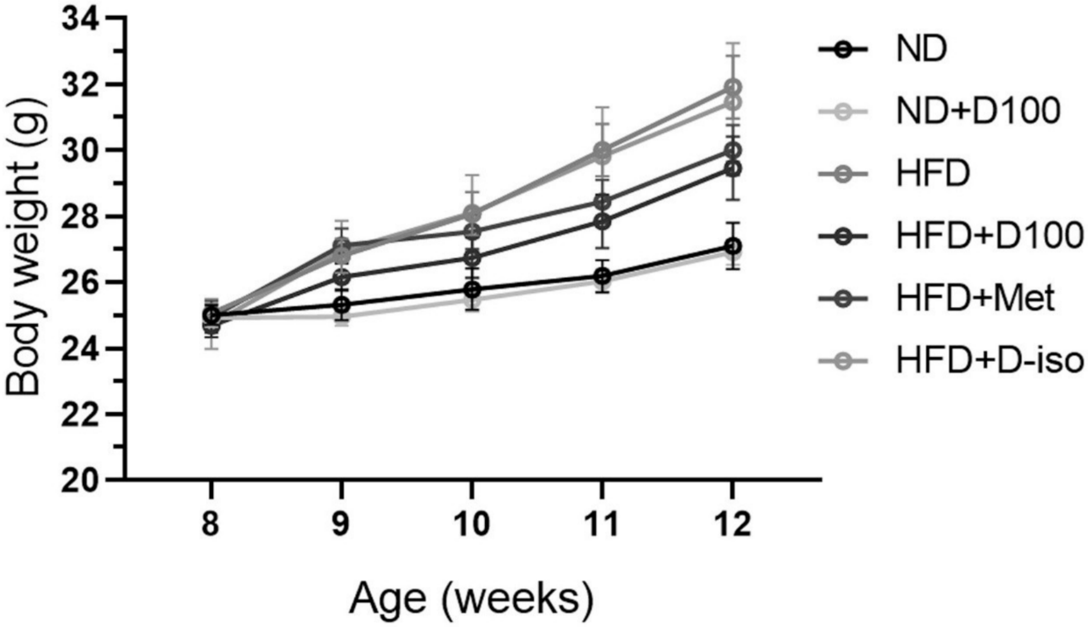


**Figure 16B**

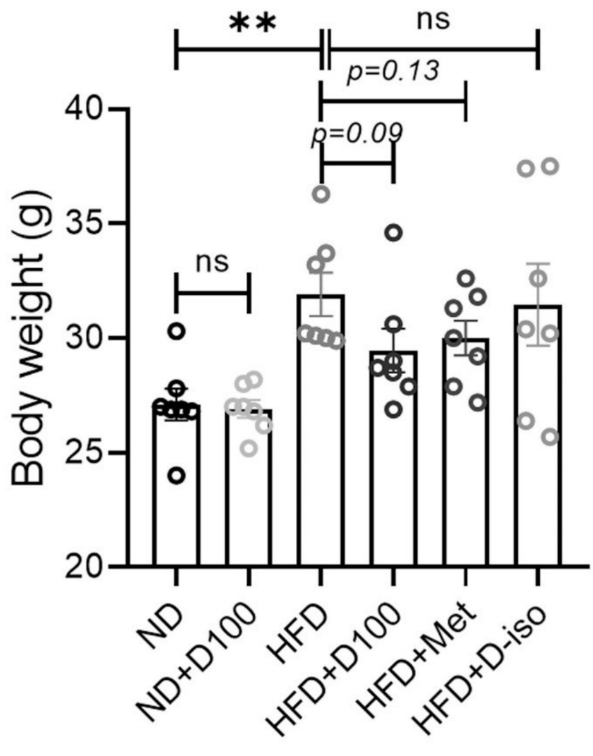

**Figure 16C**

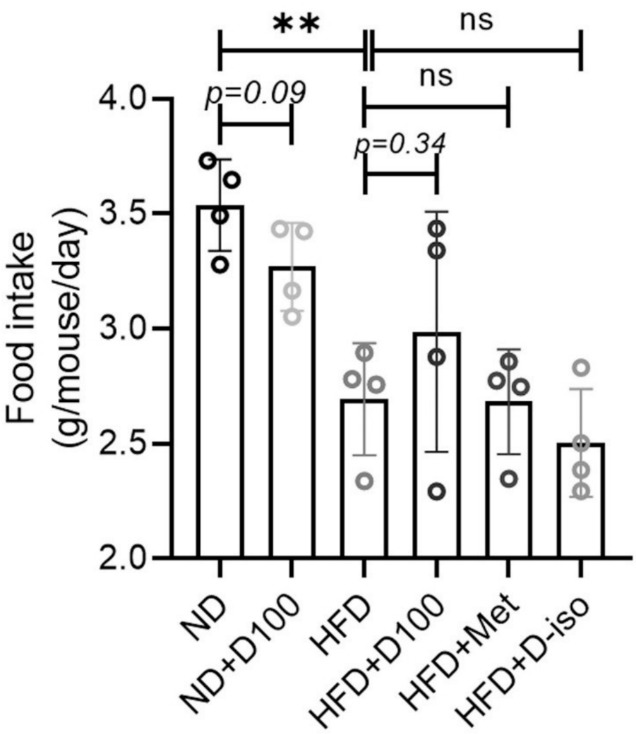


**Figure 17A**

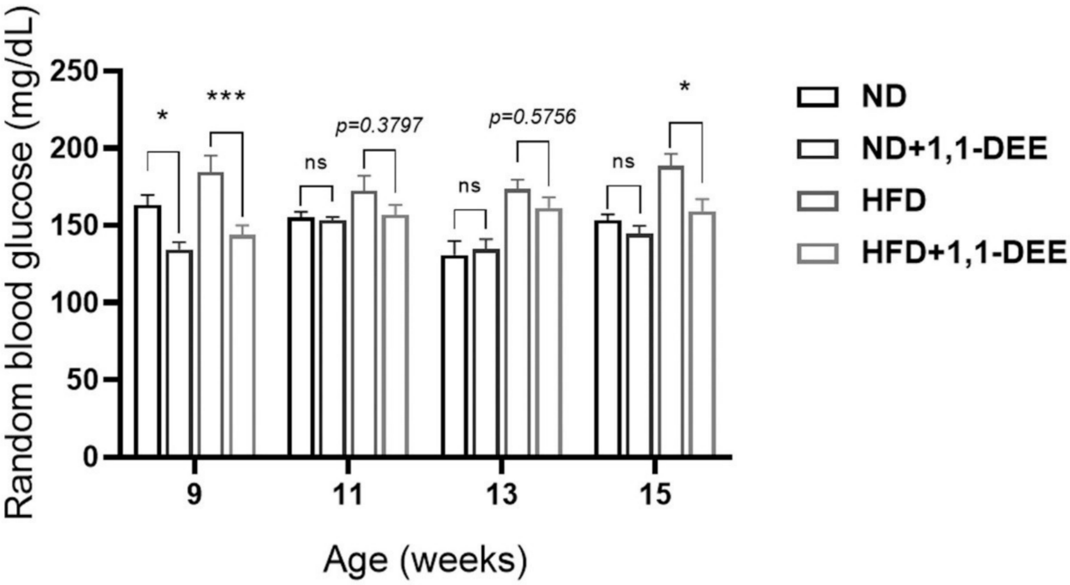

Figure 17B

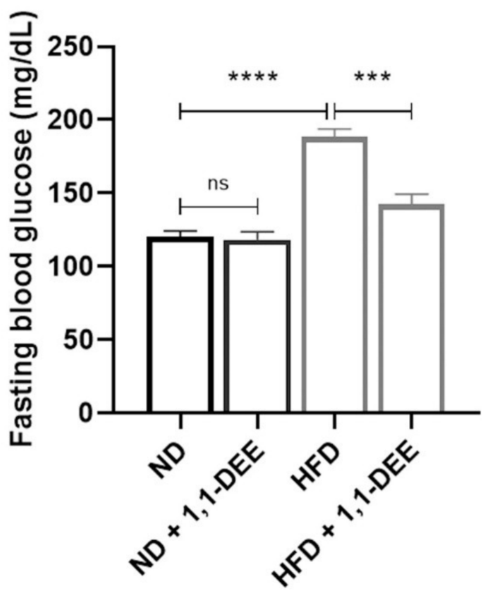
Fasting blood glucose (mg/dL)
250
200
150
100
50
0
****
***
ns
ND
ND + 1,1-DEE
HFD
HFD + 1,1-DEE

Figure 17C

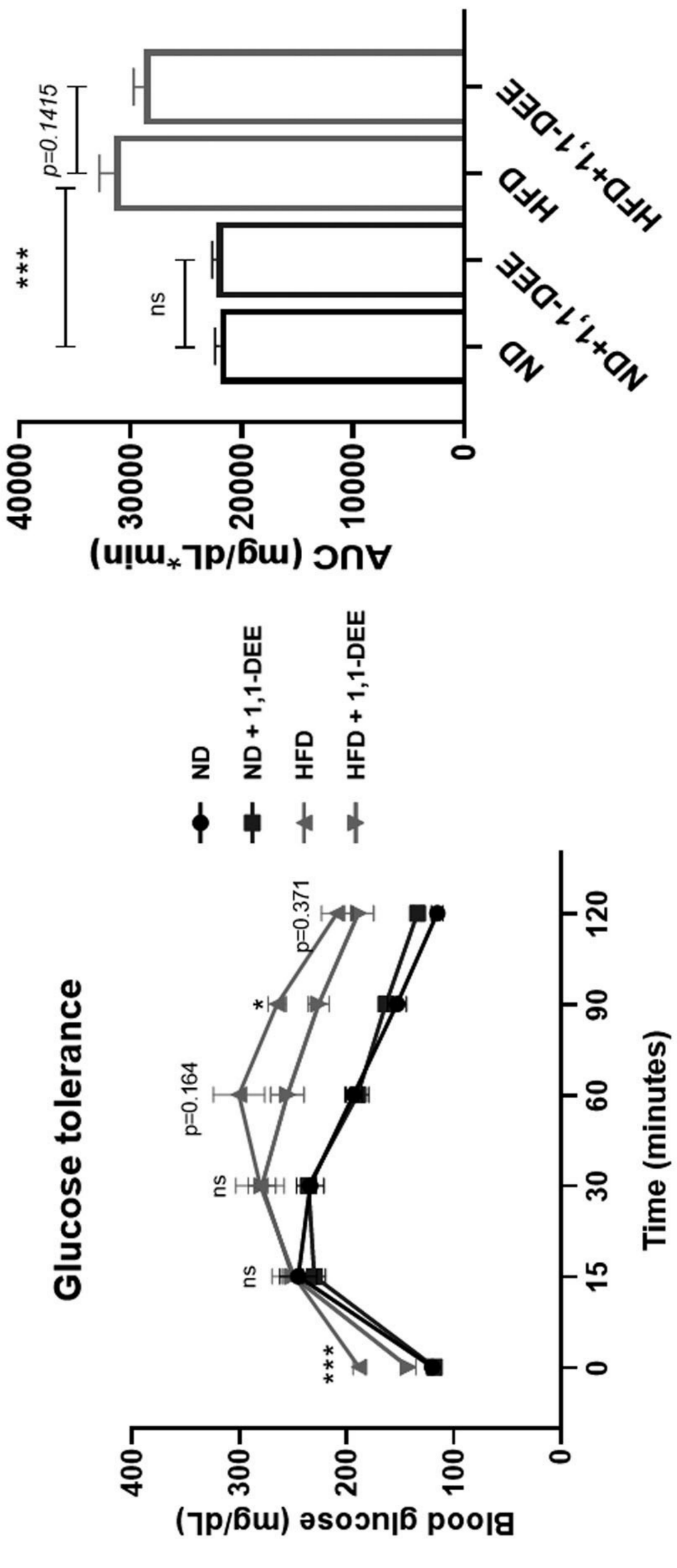
Glucose tolerance
Blood glucose (mg/dL)
Time (minutes)
0
15
30
60
90
120
0
100
200
300
400
***
ns
ns
p=0.164
*
p=0.371
ND
ND + 1,1-DEE
HFD
HFD + 1,1-DEE
AUC (mg/dL*min)
0
10000
20000
30000
40000
ns
***
p=0.1415
ND
ND+1,1-DEE
HFD
HFD+1,1-DEE

**Figure 17D**

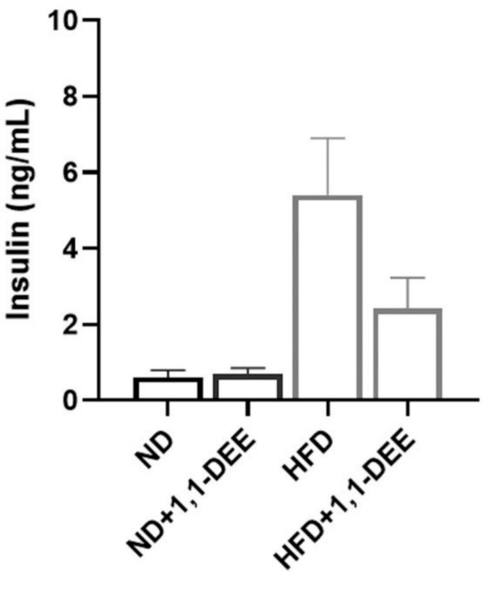

**Figure 17E**

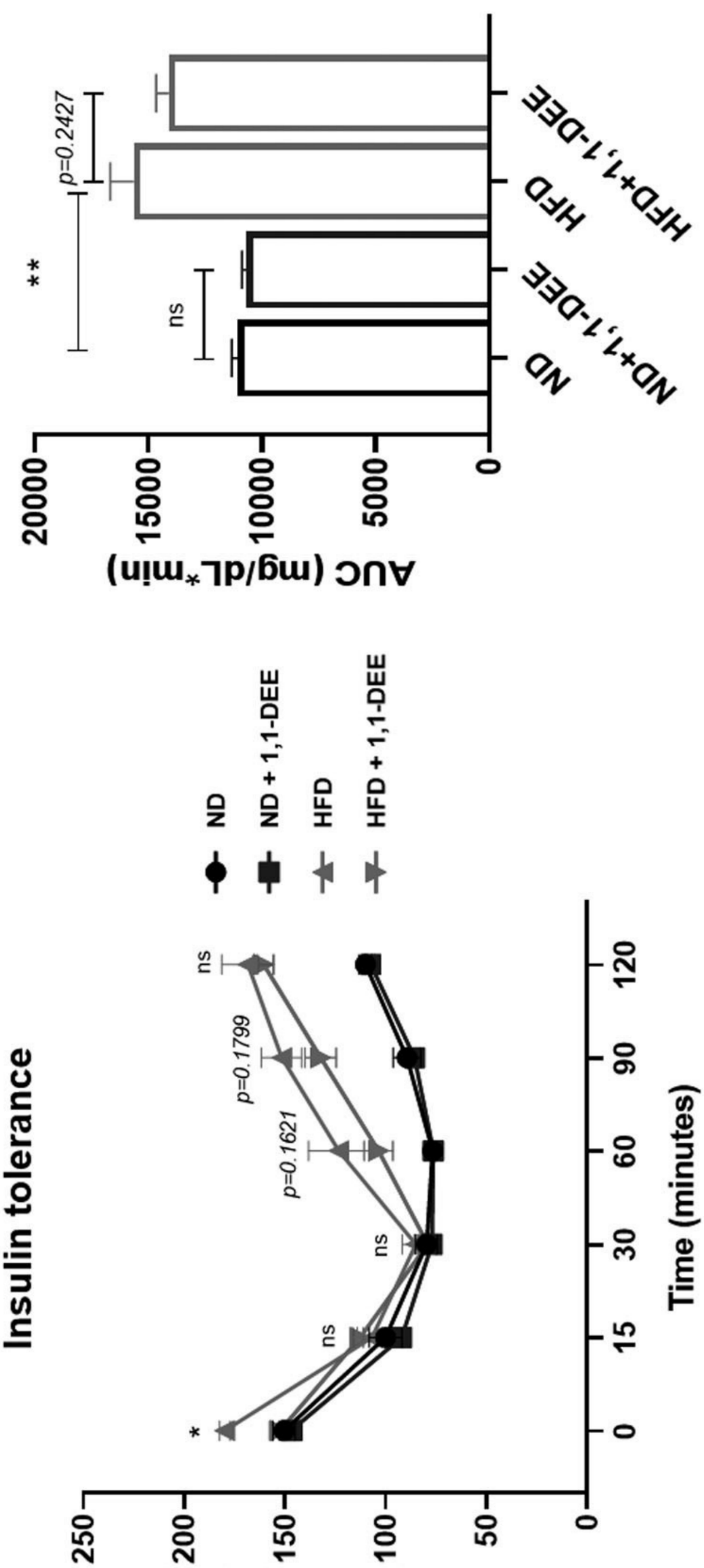
AUC (mg/dL*min)
20000
15000
10000
5000
0
ND
ND+1,1-DEE
HFD
HFD+1,1-DEE
ns
**
p=0.2427
Insulin tolerance
Blood glucose (mg/dL)
250
200
150
100
50
0
0
15
30
60
90
120
Time (minutes)
*
ns
ns
p=0.1621
p=0.1799
ns
ND
ND + 1,1-DEE
HFD
HFD + 1,1-DEE

Figure 18A

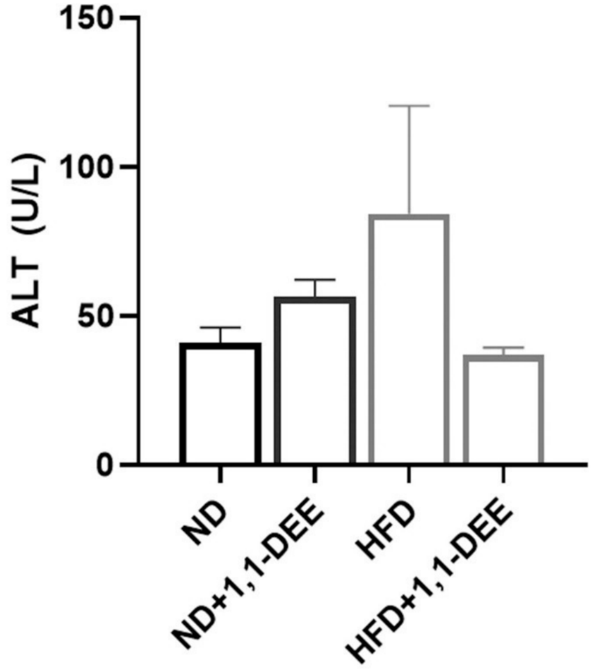


Figure 18B

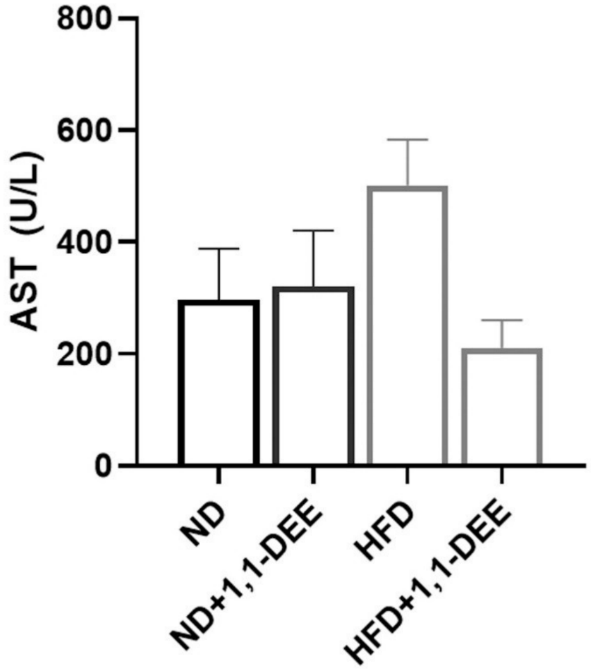

**Figure 18C**

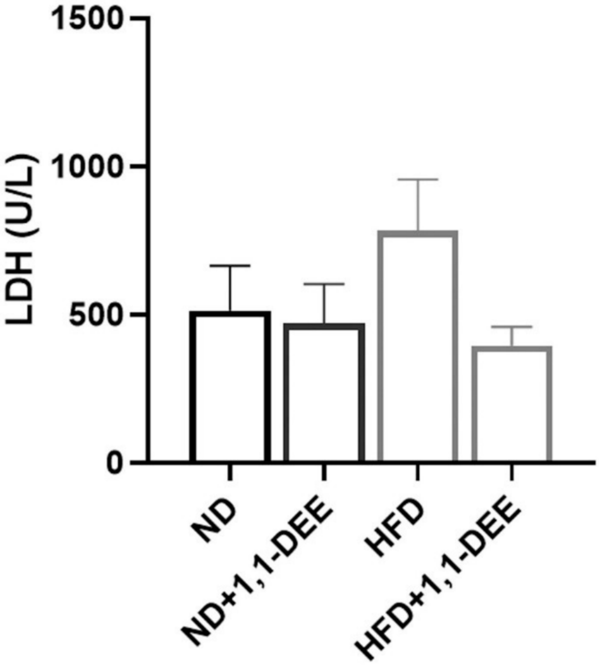


**Figure 18D**

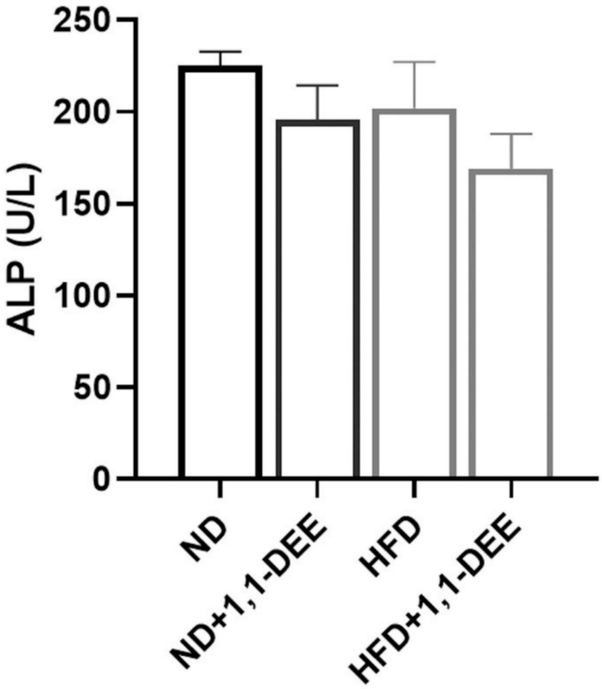

**Figure 18E**

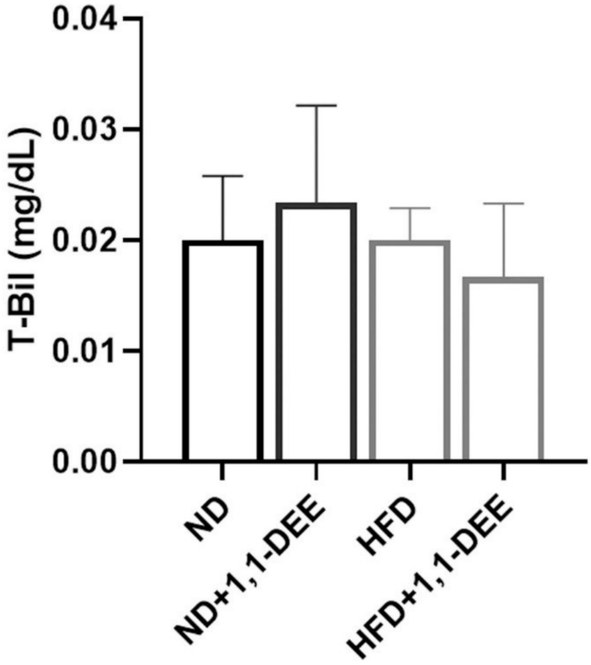


**Figure 18F**

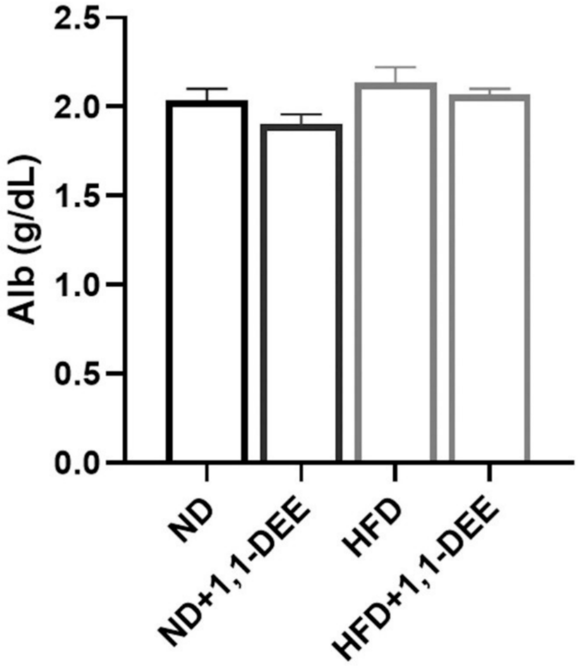

Figure 18G

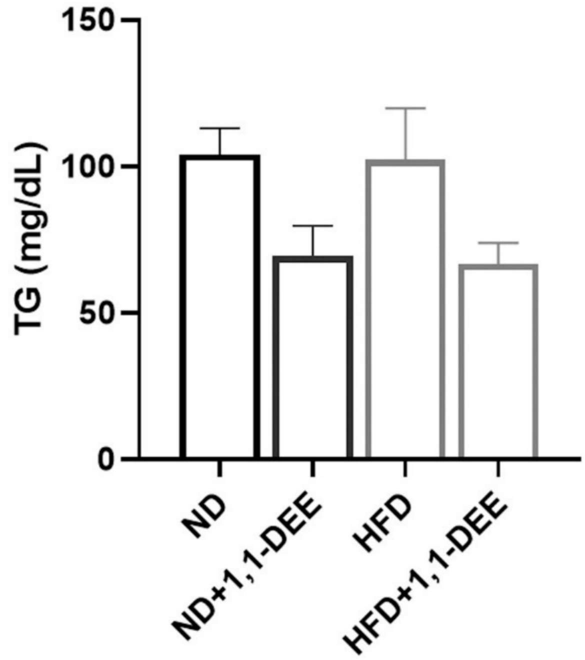


Figure 18H

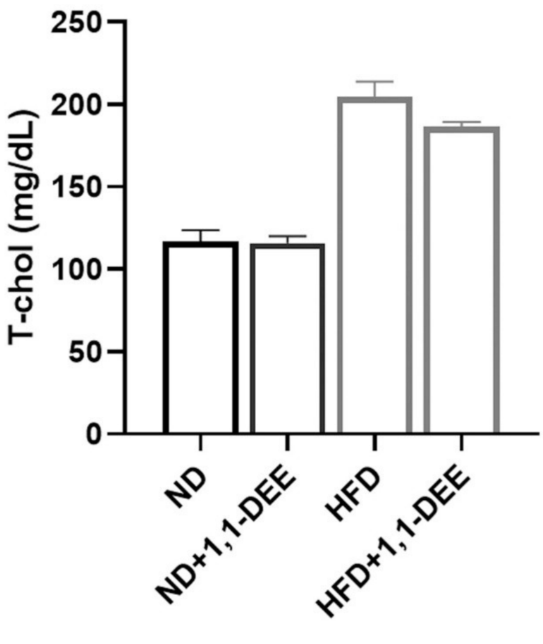

Figure 18I

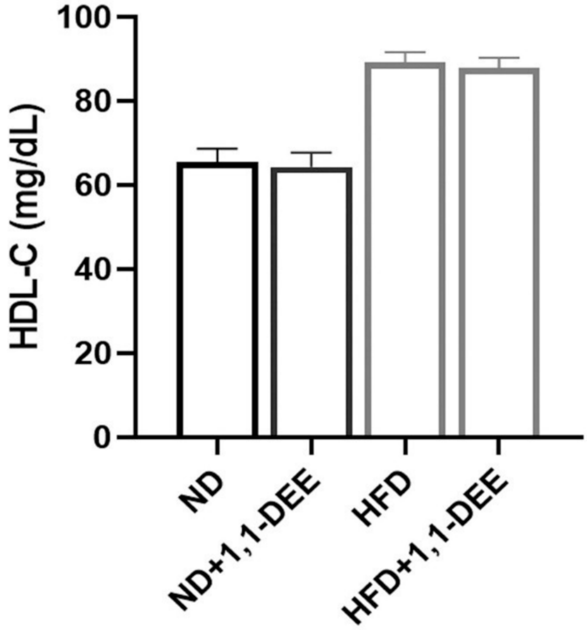


Figure 18K

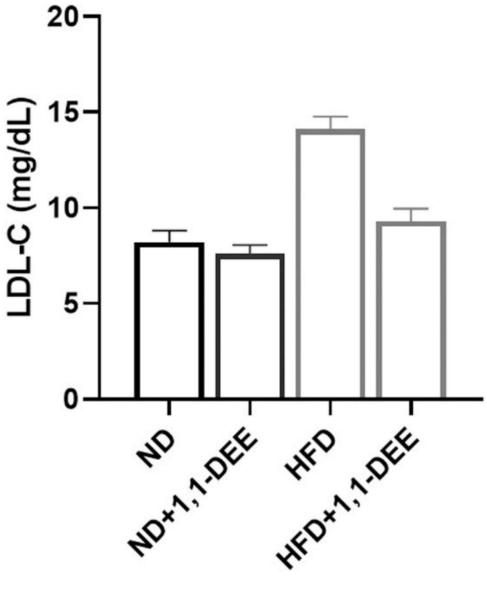

Europäisches Patentamt
European Patent Office
Office européen des brevets

# EUROPEAN SEARCH REPORT

Application Number
EP 25 22 1998

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PRAPAPORN CHANIAD ET AL: "Antimalarial efficacy and toxicological assessment of medicinal plant ingredients of Prabchompoothaweep remedy as a candidate for antimalarial drug development", BMC COMPLEMENTARY MEDICINE AND THERAPIES BIOMED CENTRAL LTD, LONDON, UK, vol. 23, no. 1, 18 January 2023 (2023-01-18), pages 1-16, XP021313490, DOI: 10.1186/S12906-023-03835-X | 1-5,7, 10-13, 15,19 | INV. A61K31/08 A61P1/16 A61P3/04 A61P3/06 A61P3/08 A61P5/50 A61P9/12 A61P19/02 |
| A | * table 5 * * page 13/16, right-hand column, paragraph 3 * | 6,8,9, 14, 16-18, 20,21 | |
| T | TRINH VU HOANG ET AL: "Biosacetalin (1,1-Diethoxyethane) Improves Healthy Lifespan in C. elegans and Rats", ANTIOXIDANTS, vol. 15, no. 2, 24 January 2026 (2026-01-24), page 160, XP093383061, Switzerland ISSN: 2076-3921, DOI: 10.3390/antiox15020160 * the whole document * | | TECHNICAL FIELDS SEARCHED (IPC) A61K A61P |

The present search report has been drawn up for all claims

1

| Place of search | Date of completion of the search | Examiner |
|---|---|---|
| Munich | 30 March 2026 | Collura, Alessandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **STEINBERG, G.R.** ; **D.G. HARDIE**. New insights into activation and function of the AMPK.. *Nat Rev Mol Cell Biol*, 2023, vol. 24 (4), 255-272
- **HERZIG, S.** ; **R.J. SHAW**. AMPK: guardian of metabolism and mitochondrial homeostasis.. *Nat Rev Mol Cell Biol*, 2018, vol. 19 (2), 121-135
- **DAVIES, S.P. et al.** Purification of the AMP-activated protein kinase on ATP-gamma-sepharose and analysis of its subunit structure.. *Eur J Biochem*, 1994, vol. 223 (2), 351-7
- **HARDIE, D.G.** ; **F.A. ROSS** ; **S.A. HAWLEY**. AMPK: a nutrient and energy sensor that maintains energy homeostasis.. *Nat Rev Mol Cell Biol*, 2012, vol. 13 (4), 251-62
- **WOODS, A. et al.** LKB1 is the upstream kinase in the AMP-activated protein kinase cascade.. *Curr Biol*, 2003, vol. 13 (22), 2004-8
- **WOODS, A. et al.** Ca2+/calmodulin-dependent protein kinase kinase-beta acts upstream of AMP-activated protein kinase in mammalian cells.. *Cell Metab*, 2005, vol. 2 (1), 21-33
- **XIE, M. et al.** A pivotal role for endogenous TGF-beta-activated kinase-1 in the LKB1/AMP-activated protein kinase energy-sensor pathway.. *Proc Natl Acad Sci U S A*, 2006, vol. 103 (46), 17378-83
- **SHAW, R.J. et al.** The kinase LKB1 mediates glucose homeostasis in liver and therapeutic effects of metformin.. *Science*, 2005, vol. 310 (5754), 1642-6
- **CHAN, A.Y. et al.** Resveratrol inhibits cardiac hypertrophy via AMP-activated protein kinase and Akt.. *J Biol Chem*, 2008, vol. 283 (35), 24194-201
- **CORTON, J.M. et al.** 5-aminoimidazole-4-carboxamide ribonucleoside. A specific method for activating AMP-activated protein kinase in intact cells?. *Eur J Biochem*, 1995, vol. 229 (2), 558-65
- **JEON, S.M.** Regulation and function of AMPK in physiology and diseases.. *Exp Mol Med*, 2016, vol. 48 (7), e245
- **LI, Y. et al.** AMPK phosphorylates and inhibits SREBP activity to attenuate hepatic steatosis and atherosclerosis in diet-induced insulin-resistant mice.. *Cell Metab*, 2011, vol. 13 (4), 376-388
- **PULIPAKA, S. et al.** Therapeutic efficacies of mitochondria-targeted esculetin and metformin in the improvement of age-associated atherosclerosis via regulating AMPK activation.. *Geroscience*, 2024, vol. 46 (2), 2391-2408
- **WANG, Z. et al.** Dexmedetomidine attenuates myocardial ischemia/reperfusion-induced ferroptosis via AMPK/GSK-3β/Nrf2 axis.. *Biomed Pharmacother*, 2022, vol. 154, 113572
- **ZHANG, Y. et al.** Melatonin attenuates myocardial ischemia-reperfusion injury via improving mitochondrial fusion/mitophagy and activating the AMPK-OPA1 signaling pathways.. *J Pineal Res*, 2019, vol. 66 (2), e12542
- **GÉLINAS, R. et al.** AMPK activation counteracts cardiac hypertrophy by reducing O-GlcNAcylation.. *Nat Commun*, 2018, vol. 9 (1), 374
- **GUO, R.** ; **J. REN**. Deficiency in AMPK attenuates ethanol-induced cardiac contractile dysfunction through inhibition of autophagosome formation.. *Cardiovasc Res*, 2012, vol. 94 (3), 480-91
- **TONG, L.** Acetyl-coenzyme A carboxylase: crucial metabolic enzyme and attractive target for drug discovery.. *Cell Mol Life Sci*, 2005, vol. 62 (16), 1784-803
- **LEPROPRE, S. et al.** AMPK-ACC signaling modulates platelet phospholipids and potentiates thrombus formation.. *Blood*, 2018, vol. 132 (11), 1180-1192
- **TURDI, S. et al.** Deficiency in AMP-activated protein kinase exaggerates high fat diet-induced cardiac hypertrophy and contractile dysfunction.. *J Mol Cell Cardiol*, 2011, vol. 50 (4), 712-22
- **HOPKINS, T.A.** ; **J.R. DYCK** ; **G.D. LOPASCHUK**. AMP-activated protein kinase regulation of fatty acid oxidation in the ischaemic heart.. *Biochem Soc Trans*, 2003, vol. 31 (1), 207-12
- **HAROLD, K.M. et al.** Loss of Cardiac PFKFB2 Drives Metabolic, Functional, and Electrophysiological Remodeling in the Heart.. *J Am Heart Assoc*, 2024, vol. 13 (7), e033676
- **FU, C. et al.** PFKFB2 Inhibits Ferroptosis in Myocardial Ischemia/Reperfusion Injury Through Adenosine Monophosphate-Activated Protein Kinase Activation.. *J Cardiovasc Pharmacol*, 2023, vol. 82 (2), 128-137
- **GAO, J. et al.** HIF-1/AKT Signaling-Activated PFKFB2 Alleviates Cardiac Dysfunction and Cardiomyocyte Apoptosis in Response to Hypoxia.. *Int Heart J*, 2021, vol. 62 (2), 350-358
- **BERGERON, R. et al.** Chronic activation of AMP kinase results in NRF-1 activation and mitochondrial biogenesis.. *Am J Physiol Endocrinol Metab*, 2001, vol. 281 (6), E1340-6

- **LANTIER, L. et al.** AMPK controls exercise endurance, mitochondrial oxidative capacity, and skeletal muscle integrity.. *Faseb j*, 2014, vol. 28 (7), 3211-24
- **SETTEMBRE, C. et al.** TFEB controls cellular lipid metabolism through a starvation-induced autoregulatory loop.. *Nat Cell Biol*, 2013, vol. 15 (6), 647-58
- **JÄGER, S. et al.** AMP-activated protein kinase(AMPK) action in skeletal muscle via direct phosphorylation of PGC-1alpha.. *Proc Natl Acad Sci U S A*, 2007, vol. 104 (29), 12017-22
- **CHEN, L. et al.** PGC-1α-Mediated Mitochondrial Quality Control: Molecular Mechanisms and Implications for Heart Failure.. *Front Cell Dev Biol*, 2022, vol. 10, 871357
- **TIAN, L. et al.** Pretreatment with Tilianin improves mitochondrial energy metabolism and oxidative stress in rats with myocardial ischemia/reperfusion injury via AMPK/SIRT1/PGC-1 alpha signaling pathway.. *J Pharmacol Sci*, 2019, vol. 139 (4), 352-360
- **KIM, H.** ; **X. XUE**. Detection of Total Reactive Oxygen Species in Adherent Cells by 2',7'-Dichlorodihydrofluorescein Diacetate Staining.. *J Vis Exp*, 2020, vol. 160